# FASCICULE DE BREVET EUROPEEN

(11) **EP 2 167 972 B1**
(45) Date de publication et mention de la délivrance du brevet: **17.02.2016**
(21) Numéro de dépôt: 08827168.9
(22) Date de dépôt: 10.07.2008
(51) Int. Cl.: G01N 33/574

(54) **PROCEDE DE DOSAGE DE L'APOLIPOPROTEINE AII POUR LE DIAGNOSTIC IN VITRO DU CANCER COLORECTAL**
APOLIPOPROTEIN-AII-TESTVERFAHREN ZUR IN-VITRO-DIAGNOSE VON KOLOREKTALKARZINOM
APOLIPOPROTEIN AII ASSAY METHOD FOR THE IN VITRO DIAGNOSIS OF COLORECTAL CANCER

(30) Priorité: 19.07.2007 FR 0705210
(43) Date de publication de la demande: 31.03.2010
(73) Titulaire: bioMérieux, 69280 Marcy-L'Etoile (FR)
(72) Inventeur: ATAMAN-ÖNAL, Yasemin, F-01600 Reyrieux (FR); CHARRIER, Jean-Philippe, F-69160 Tassin La Demi-lune (FR); CHOQUET-KASTYLEVSKY, Geneviève, F-69340 Francheville (FR); POIRIER, Florence, F-93200 Saint-denis (FR)
(86) Numéro de dépôt international: PCT/FR2008/051295
(87) Numéro de publication internationale: WO 2009/019370

(56) Documents cités:
- WO-A-2004/073730
- WO-A-2005/015219
- WO-A-2006/074360
- WO-A-2007/002535
- HACHEM H. ET AL.: "Serum apolipoproteins A-I, A-II and B in hepatic metastases comparison with other liver diseases: hepatomas and cirrhosis." J. CLIN. CHEM. CLIN. BIOCHEM., vol. 24, no. 3, 1986, pages 161-166, XP009092070
- DUFFY M.J. ET AL.: "Tumour markers in colorectal cancer: European Group on Tumour Markers (EGTM) guidelines for clinical use." EUR. J. CANCER, vol. 43, no. 9, 7 juin 2007 (2007-06-07), pages 1348-1360, XP022107659

## Description

La présente invention concerne le domaine de la cancérologie. Plus particulièrement, la présente invention a pour objet un procédé de diagnostic *in vitro* du cancer colorectal chez un patient humain par détermination de la présence de l'Apolipoprotéine AII dans un échantillon biologique issu de ce patient, ledit procédé pouvant être utilisé tant dans le diagnostic précoce, le dépistage, le suivi thérapeutique, le pronostic, que dans le diagnostic des rechutes dans le cadre du cancer colorectal.

Le cancer colorectal (CCR) est un problème majeur de santé publique. Son incidence mondiale a été estimée à 875000 nouveaux cas en 1996¹. Tous sexes confondus, c'est le cancer qui survient le plus fréquemment dans les pays occidentaux où il est généralement classé parmi les 3 premières causes de décès par cancer. Le taux de survie à 5 ans tout stade confondu est voisin de 60%.

Seul un diagnostic précoce offre l'espoir d'un traitement curatif. Or, à l'heure actuelle, il n'existe aucun test sérologique de dépistage, ni de diagnostic spécifique qui soit précoce.

Le dépistage du cancer colorectal est réalisé actuellement en Europe avec deux approches distinctes : premièrement à l'aide d'un test paraclinique qui consiste à rechercher la présence de sang dans les selles (Faecal Occult Blood test, FOBT, commercialisé par exemple sous le nom d'Hémoccult®). Cette technique a démontré son utilité clinique. Lorsqu'elle est utilisée tous les 2 ans chez les personnes âgées de 50 à 74 ans, elle peut réduire de 15 à 20% la mortalité par cancer colorectal². Pour cela, il faut que plus de la moitié de la population concernée participe régulièrement au dépistage et qu'une colonoscopie soit faite en cas de test positif, suivie éventuellement d'un traitement adapté.

Néanmoins, cette technique de dépistage souffre d'un certain nombre de handicaps :
- L'inconvénient majeur de ce test est sa sensibilité médiocre, tout spécialement pour les adénomes (lésion dysplasique pré-cancéreuse), qui s'ils sont de grande taille conduiront dans 1 cas sur 10 au développement d'un cancer.
- Le test est également peu spécifique. L'apparition de sang dans les selles peut être liée à une affection non tumorale : hémorragies recto-coliques, hémorroïdes, fistules... Dans ce cas, une investigation par colonoscopie doit être réalisée avec les inconvénients décrits ci-après.
- Enfin les Hémoccult® sont délicats à interpréter, ils doivent donc être lus dans des centres spécialisés, par un personnel qualifié et compétent.

Des tests immunologiques spécifiques de l'hémoglobine humaine (Feca EIA®, Heme Select®, ...) ont également été décrits. Ils constituent probablement un progrès par rapport à l'Hémoccult® mais ils présentent par essence les mêmes problèmes. C'est ainsi que InSure^{™}, commercialisé par Enterix Inc., permet de détecter 87% des patients atteints de CCR et 47% de ceux ayant des polypes précancéreux. Il s'agit d'un test de détection de l'hémoglobine humaine dans les selles, et plus particulièrement de la portion de globine de cette molécule.

Une deuxième stratégie de dépistage est la réalisation systémique d'une colonoscopie après 50 ans, qui permet en théorie de réduire la mortalité par cancer colorectal. Mais l'acceptabilité de cet examen chez des sujets en bonne santé est trop faible pour qu'une politique de dépistage utilisant l'endoscopie diminue la mortalité (il y a une compliance aux alentours de 2% pour la colonoscopie dans les pays d'Europe ayant mis en place cette stratégie de dépistage). Il existe un risque non négligeable (1 ‰) de perforation et hémorragie du côlon et de décès (1/10 000), ainsi qu'un coût élevé pour la santé publique. De plus, la colonoscopie nécessite une préparation colique préalable très contraignante, qui explique en grande partie la mauvaise compliance.

Des marqueurs tumoraux dosables par immunoessais ont été décrits de longue date dans le cadre du cancer colorectal. Il s'agit notamment de l'antigène carcinoembryonnaire (ACE) et du CA19-9.

L'ACE est utilisé pour le suivi. Il ne peut pas être utilisé pour le dépistage ni pour le diagnostic précoce du cancer colorectal car sa sensibilité et sa spécificité sont insuffisantes. En effet, ce marqueur est exprimé par d'autres types de cancers, et dans des pathologies bénignes. Malgré tout, il est possible de gagner en sensibilité sans perdre en spécificité en associant à l'ACE un autre marqueur tumoral tel que le CA 19-9 ou le CA72-4.

Les causes de variations physiologiques du CA19-9 sont rares mais d'autres affections bénignes (hépatobiliaires, pancréatiques), ou malignes peuvent induire une élévation du CA19-9. Ce marqueur pris isolément ne présente donc pas non plus d'intérêt pour le diagnostic. Néanmoins, sa concentration sérique étant corrélée à la taille de la tumeur et à la présence de métastases, il peut permettre également un suivi thérapeutique ou la mise en évidence précoce de récidives.

Des tests commerciaux ont par ailleurs été proposés tels que :
➢ Colopath®/ColorectAlert^{MD}, commercialisé par Ambrilia, est un test de dépistage rapide et peu invasif pour le CCR. Colopath® détecte un plasmalogène (classe de lipides complexes faisant partie des phospholipides) dans le mucus rectal des individus avec une pathologie colorectale, tandis que le ColorectAlert^{MD} détecte l'antigène-T, un sucre complexe dans le mucus rectal. Le test Colopath®/ColorectAlert^{MD} implique l'application de mucus rectal sur une bande de test et le résultat positif ou négatif est basé sur une réaction de Schiff. Ambrilia a étudié 1 787 sujets et démontré que le Colopath®/ColorectAlert^{MD} détecte 54% des cas de cancer colorectal de stade précoce et 49% de tous stades confondus.
➢ *CQLARIS*, commercialisé par Myriad Genetics, est un test de détection dans le sang de mutations dans les gènes MLH1 et MSH2 pour le dépistage des cancers héréditaires du côlon non polyposiques (syndrome HNPCC). Le résultat du test est disponible en 3 semaines. Myriad utilise les techniques de séquençage les plus sensibles et les plus spécifiques existantes à l'heure actuelle. Le coût du test est élevé.
➢ *DR-70*®, commercialisé par AMDL, est un test de dépistage de différents types de cancers (poumon, côlon, sein, foie, estomac, ...). Il n'est donc pas spécifique du CCR. Son principe est basé sur la technique ELISA double sandwich (dosage de l'antigène DR-70). La révélation se fait par réaction enzymatique (anticorps couplés à la biotine et à la streptavidine). Une réaction colorée indique la présence de cancer.

La Demanderesse a maintenant mis en évidence de façon surprenante un nouveau marqueur tumoral qui peut être détecté dans les échantillons biologiques distants des tumeurs.

Ainsi, la présente invention a pour premier objet un procédé de diagnostic *in vitro* du cancer colorectal par détermination de la présence de l'Apolipoprotéine AII dans des échantillons biologiques issus de patients suspectés d'être atteints du cancer colorectal, et lesdits échantillons étant des échantillons distants des tumeurs.

Elle concerne également l'utilisation de ce procédé tant dans le diagnostic précoce, le dépistage, le suivi thérapeutique, le pronostic, que dans le diagnostic des rechutes dans le cadre du cancer colorectal.

Le procédé de l'invention permet donc de diagnostiquer de façon spécifique et précoce le cancer colorectal par un test simple consistant à rechercher la présence d'Apolipoprotéine AII dans un échantillon biologique prélevé chez un patient, ledit échantillon étant distant de la tumeur potentielle. En effet, la Demanderesse a montré de façon inattendue que la concentration d'Apolipoprotéine AII dans l'échantillon biologique dans lequel on met en oeuvre le procédé de l'invention était diminuée par rapport aux valeurs de référence déterminées pour les patients sains.

La détermination de la présence de l'Apolipoprotéine AII dans un échantillon biologique distant de la tumeur permet alors de conclure à la pathologie recherchée. Un des avantages du procédé de l'invention réside donc en la possibilité d'utiliser un échantillon distant de la tumeur potentielle à titre d'échantillon de diagnostic, ce qui permet un diagnostic simple et non invasif alors qu'un diagnostic tissulaire nécessite une biopsie prélevée de façon invasive. En effet, l'étude de marqueurs tissulaires, par exemple sur coupe de tissu (immunohistochimie), peut présenter un intérêt pronostique mais n'a aucun intérêt pour le dépistage ou le diagnostic du cancer colorectal.

Les Apolipoprotéines sont une famille de protéines constituées d'acides aminés polaires permettant le transport des lipides dans le sang par formation d'un complexe macromoléculaire hydrophile appelé lipoprotéine. Pour chacune des Apolipoprotéines plasmatiques humaines existent des isoformes issues de polymorphisme génétique et/ou de modifications post-traductionnelles dont la présence dans le sang peut être associée à certaines pathologies³. La concentration plasmatique des Apolipoprotéines est non négligeable, de l'ordre du mg/ml⁴.

Le marqueur Apolipoprotéine AII, (N° Swiss Prot P02652, également appelé ApoA II, Apo-AII, et Apo A2) est une protéine de 17380 Da composée de deux chaînes polypeptidiques de 77 acides aminés chacune reliées par un pont disulfure. Comme l'Apolipoprotéine AI, l'Apolipoprotéine AII est essentiellement synthétisée par le foie et l'intestin. Hachem et al.⁵ ont également dosé, outre l'Apo AI, l'Apo AII dans des sérums de patients ayant eu le cancer du foie suite à des métastases du cancer colorectal. Toutefois, les résultats ne sont pas significatifs et ne permettent pas une conclusion quant à la pathologie recherchée. La Demanderesse a montré quant à elle de façon surprenante la diminution de la concentration de cette protéine chez les patients atteints d'un cancer colorectal en fait un bon marqueur dans les échantillons biologiques issus d'un patient atteint d'un cancer colorectal, lesdits échantillons étant distants de la tumeur.

Par la détermination de la présence du marqueur tumoral, on entend la détermination de la présence de la protéine, de son ARN messager, ou de la détection de modification sur son gène dans les séquences codantes ou non codantes, comme des méthylations.

Par relarguage par les tumeurs coliques, on entend la sécrétion active ou passive ou la libération, quel qu'en soit le mécanisme, du marqueur tumoral par les cellules tumorales elles-mêmes ou par les cellules non tumorales voisines suite à des lésions ou des modifications de phénotype cellulaire résultant du développement tumoral.

Par échantillon biologique distant de la tumeur dans lequel on met en oeuvre le procédé de l'invention, on entend tout échantillon biologique non situé directement au niveau de la tumeur elle-même ou de ses métastases et susceptible de contenir le marqueur tumoral d'intérêt. A titre d'exemple d'échantillon biologique distant de la tumeur, on peut citer les fluides biologiques tels que le sang total ou ses dérivés, par exemple sérum ou plasma, les urines, la salive et les épanchements, la moelle osseuse et les selles, et les cellules purifiées à partir de ces échantillons liquides. On préfère le sang ou ses dérivés ainsi que les selles, les épanchements et les cellules purifiées à partir de ces échantillons liquides.

Le procédé de l'invention peut être amélioré en détectant, outre l'Apolipoprotéine AII, au moins un autre marqueur tumoral, le cas échéant également relargué par les tumeurs coliques hors des tissus cancéreux. Ainsi, la combinaison d'au moins deux marqueurs permet d'améliorer la spécificité et la sensibilité du test de diagnostic du cancer colorectal.

Ainsi, un autre objet de l'invention consiste également à déterminer la présence d'au moins un autre marqueur tumoral choisi parmi les deux groupes de marqueurs suivants, considérés seuls ou en association :
- Groupe A : Leucocyte Elastase Inhibitor, Ezrine, Aminoacylase 1, Liver Fatty Acid-Binding Protein, Intestinal Fatty Acid-Binding Protein, Apolipoprotéine AI et Plastine-I, certains de ces marqueurs étant de nouveaux marqueurs identifiés par la Demanderesse,
- Groupe B : marqueurs ayant un intérêt diagnostic supplémentaire, à savoir : Beta 2 Microglobuline, Protéasome 20S, Galectine-3, L-Lactate Deshydrogénase Chaîne B, Calréticuline, Regenerating Islet-Derived Protein 3 Alpha, Tumor-Associated Calcium Signal Transducer 1, Kératine type II Cytoskeletal 8, Kératine type I Cytoskeletal 18, Kératine type I Cytoskeletal 19, Epithelial-Cadhérine, ACE, Villine, CA19-9, CA 242, CA 50, CA 72-2, testostérone, TIMP-1, Cripto-1, Intélectine-1, Protéine Disulfide Isomérase, Cytokératine 20, Translationally-Controlled Tumor Protein, (Pro)défensine-A5, la détection de fragments d'ADN dans le sang ayant des altérations spécifiques de leur profil de méthylation, comme par exemple l'ADN méthylé du gène AXL4 (Aristaless-like Homeobox-4 Gene Methylation) ou l'ADN méthylé du gène Septin-9, la détection d'altérations spécifiques de fragments d'ADN dans les selles comme des mutations spécifiques de l'ADN dans les selles ou des altérations spécifiques du profil de méthylation de l'ADN dans les selles, la détection d'hémoglobine humaine dans les selles.

Le procédé de l'invention peut donc être amélioré en détectant au moins deux marqueurs, l'un étant l'Apolipoprotéine AII, l'autre étant un autre marqueur tumoral choisi dans le Groupe A, à savoir : Leucocyte Elastase Inhibitor, Ezrine, Aminoacylase 1, Liver Fatty Acid-Binding Protein, Intestinal Fatty Acid-Binding Protein, Apolipoproteine AI et Plastine-I.

Par marqueur tumoral nouvellement décrit, on entend la protéine, l'ARN messager ou des modifications spécifiques du gène correspondant, comme des mutations ou des méthylations.

Le marqueur tumoral Leucocyte Elastase Inhibitor (N° Swiss Prot P30740, également appelé LEI, Serpin B1, Monocyte/neutrophil elastase inhibitor, M/NEI ou EI) a été séquencé en 1992⁶. Le LEI inhibe spécifiquement les protéases ayant des propriétés de type Elastase ou Chymotrypsine par formation de complexe non dissociable sous l'action du SDS⁷. C'est ainsi que le LEI inhibe trois des protéases majeures produites par les neutrophiles : la Leucocyte Elastase, la proteinase-3 et la Cathepsine G. Ces protéases permettent au système immunitaire de défendre l'organisme par protéolyse de substrats extracellulaires ou phagocytés. Mais lorsque ces protéases sont en excès, elles sont responsables de réactions inflammatoires. Le LEI pourrait donc avoir un rôle de régulation et de limitation de l'action inflammatoire induite par les protéases cellulaires. La Demanderesse a montré quant à elle de façon surprenante que cette protéine est un bon marqueur dans les échantillons biologiques issus d'un patient atteint d'un cancer colorectal, lesdits échantillons étant distants ou non de la tumeur.

Le marqueur Ezrine (N° Swiss Prot P15311, également appelé p81, Cytovillin ou Villin-2) est une protéine assurant la liaison entre la membrane cellulaire et les filaments d'Actine du cytosquelette de la cellule, notamment dans les microvillosités des cellules épithéliales intestinales⁸. W.G. Jiang et S. Hiscox⁹ ont montré que les Interleukines IL-2, IL-8, IL-10, ... pouvaient inhiber l'expression d'Ezrine dans la lignée cellulaire de cancer colorectal humaine, HT29. Les mêmes auteurs¹⁰ ont montré que l'inhibition de l'expression d'Ezrine dans les lignées cellulaires de cancer colorectal, HT115 et HRT18, réduisait l'adhésion entre cellules et augmentait la mobilité et le comportement invasif des cellules. Ils ont conclu que l'Ezrine régulait les adhésions cellule/cellule et cellule/matrice, en interagissant avec les molécules d'adhésion cellulaire, E-Cadhérine et beta-Caténine. Ils ont suggéré que l'Ezrine pouvait jouer un rôle important dans le contrôle du potentiel invasif des cellules cancéreuses. Par ailleurs, T. Xiao et al.¹¹ ont utilisé un dosage ELISA pour quantifier l'Ezrine plasmatique de patients atteints d'un cancer du poumon. Toutefois, ils n'ont pas observé de différences par rapport à des sujets contrôles. La Demanderesse a montré quant à elle de façon surprenante que cette protéine est un bon marqueur dans les échantillons biologiques issus d'un patient atteint d'un cancer colorectal, lesdits échantillons étant distants ou non de la tumeur.

Le marqueur Aminoacylase 1 (N° Swiss Prot Q03154, également appelé EC 3.5.1.14, N-Acyl-L-Amino-Acid Amidohydrolase ou ACY-1) fait partie de la famille des Aminoacylases. Ce sont des enzymes qui catalysent l'hydrolyse des acides aminés acylés pour donner des acides gras et des acides aminés¹². Un dosage immunochimique de l'activité enzymatique Aminoacylase a été développé dès 1975 par K. Lorentz et al.¹³ et a été utilisé pour doser différents tissus et sérums¹⁴. L'étude a montré une augmentation de l'activité Aminoacylase en cas de pathologies hépatiques mais non en cas de cancer du côlon. Par ailleurs, le gène de l'Aminoacylase 1 a été identifié sur le chromosome 3p21.1¹⁵. La région 3p21.1 est réduite à l'homozygotie lors d'un cancer du poumon à petite cellule, et dans ce cas, l'expression de l'Aminoacylase est réprimée ou indétectable¹⁶. De même S. Balabanov et al.¹⁷ ont montré que l'expression de l'Aminoacylase était réprimée en cas de cancer du rein. La Demanderesse a montré quant à elle de façon surprenante que cette protéine est un bon marqueur dans les échantillons biologiques issus d'un patient atteint d'un cancer colorectal, lesdits échantillons étant distants ou non de la tumeur.

Le marqueur Liver Fatty Acid-Binding Protein (N° Swiss Prot P07148, également appelé L-FABP, FABP1, FABPL, protéine Z ou protéine transporteur de stérol) appartient à la famille des FABP qui comprend neuf isoformes. Chaque isoforme est dénommée d'après le tissu dans lequel elle a été détectée la première fois. Ces isoformes possèdent une communauté de fonction, des structures tridimensionnelles ressemblantes mais leur homologie de séquence n'est pas élevée. La L-FABP a été séquencée en 1985¹⁸. C'est une petite protéine de 15 kDa, abondante dans le cytosol, possédant la capacité de se fixer aux acides gras libres ainsi qu'à la bilirubine. Quelques études récentes semblent indiquer que les altérations de l'expression de la protéine L-FABP pourraient induire un processus de tumorigenèse. Pour le cancer de la prostate, le niveau d'expression des ARNm du L-FABP dans les biopsies de tissu tumoral était 10 fois plus élevé que dans le tissu normal¹⁹. Pour le cancer du côlon, plusieurs équipes ont identifié une diminution de l'expression de la protéine L-FABP au niveau du tissu tumoral comparée à la muqueuse colique normale, en utilisant des techniques d'électrophorèse en 2 dimensions²⁰. Ce résultat a aussi été confirmé par des techniques d'immunohistochimie. De plus, la protéine L-FABP est un marqueur pronostic de résection hépatique chez les patients atteints de cancer colorectal ayant métastasé dans le foie²¹. La Demanderesse a montré quant à elle de façon surprenante que cette protéine est un bon marqueur dans les échantillons biologiques issus d'un patient atteint d'un cancer colorectal, lesdits échantillons étant distants de la tumeur.

Le marqueur Intestinal Fatty Acid-Binding Protein (N° Swiss Prot P12104, également appelé I-FABP, FABP-2 ou FABPI) a été séquencé en 1987²². C'est une petite protéine de 15 kDa, abondante dans le cytosol, possédant la capacité de se fixer aux acides gras libres ainsi qu'à la bilirubine. La protéine I-FABP est exprimée au niveau des entérocytes de l'intestin grêle et peut constituer environ 2% du contenu protéique de ce type cellulaire. Au niveau tissulaire, le duodénum et le jejunum contiennent des quantités significativement plus élevées de I-FABP que le côlon (jéjunum : 4,8 µg/g, côlon : 0,25 µg/g)²³. La I-FABP n'a pas pu être détectée dans les échantillons de plasma de sujets sains. Par contre, dans certains contextes pathologiques comme l'ischémie intestinale, la maladie de Crohn ou la cirrhose biliaire primitive, il est possible de mettre en évidence une augmentation de la concentration de I-FABP plasmatique chez certains sujets²³. Pour le cancer de la prostate, il a été montré que le niveau d'expression des ARNm du I-FABP dans les biopsies de tissu tumoral était 7 fois plus élevé que dans le tissu normal¹⁹. Dans le modèle d'induction de tumeur colorectal par l'azoxyméthane chez le rat, le niveau d'expression des ARNm de la I-FABP est diminué de 2,92 à 3,97 fois lorsque les animaux ont une alimentation qui réduit l'incidence de cancer (protéines du soja ou hydrolysat de petit lait)²⁴. La Demanderesse a montré quant à elle de façon surprenante que cette protéine est un bon marqueur dans les échantillons biologiques issus d'un patient atteint d'un cancer colorectal, lesdits échantillons étant distants de la tumeur.

Le marqueur Apolipoprotéine AI (N° NCBI 490098, également appelé Apo A-I, Apo AI et Apo A1) est une protéine de 243 acides aminés et de 28 kDa. Il est essentiellement synthétisé par le foie et l'intestin. Cette protéine a été montrée sous-abondante dans les sérums de patients souffrant d'un cancer colorectal par rapport aux sujets sains par SELDI-TOF²⁵. Cependant, il est précisé dans cet article que la discrimination des patients atteints de CCR par rapport aux sujets sains est réalisée en combinant l'Apo AI à d'autres marqueurs protéiques. Par ailleurs, cet article précise que le dosage par immunoessai turbidimétrique de l'Apo AI réalisé par une autre équipe ne confirme pas la sous-abondance de cette protéine dans les sérums de patients atteints de CCR²⁶. Hachem et al.⁵ ont quant à eux dosé l'Apo AI dans des sérums de patients ayant eu le cancer du foie suite à des métastases du cancer colorectal. La Demanderesse a montré quant à elle de façon surprenante qu'un dosage par immunoessai permet de mettre en évidence la diminution de la concentration de cette protéine chez les patients atteints d'un cancer colorectal, contrairement à ce qui était avancé par Engwegen et al.²⁵ qui ont pu mettre en évidence cette diminution uniquement en mettant en oeuvre la technique SELDI-TOF. Le dosage par immunoessai de l'Apo AI dans les échantillons biologiques est un bon procédé de diagnostic du cancer colorectal, lesdits échantillons étant distants de la tumeur, dans la mesure où le dosage par immunoessai mis en oeuvre n'est pas la turbidimétrie comme utilisée par l'équipe de Zhang et al.²⁶.

Le marqueur Plastine-I (N° Swiss Prot Q14651, également appelé I-plastin, Intestine-specific plastin ou Plastin 1) appartient à la famille des Plastines humaines dont trois représentants sont connus : la Plastine-I, la Plastine-L et la Plastine-T. Certains auteurs appellent les Plastines « Fimbrines », d'autres auteurs encore réservent le nom de Fimbrine à la Plastine-I. Les Plastines sont des protéines se liant à l'Actine pour former le cytosquelette (squelette cellulaire). Ce sont des protéines de 70 kDa relativement bien conservées tout au long de l'évolution des Eucaryotes. Elles présentent une forte spécificité tissulaire, seulement une isoforme à la fois est présente dans les tissus normaux²⁷. L'utilisation des Plastines vis-à-vis du cancer a déjà été décrite dans le brevet US-A-5,360,715, qui propose une méthode pour déterminer si une cellule est hématopoïétique ou néoplasique, c'est-à-dire cancéreuse. Cette méthode revendique le dosage de la Plastine-L et de la Plastine-T au niveau cellulaire, et plus particulièrement le dosage de leurs ARNm. Toutefois, malgré ces propriétés, aucun travail antérieur n'a été réalisé pour évaluer l'intérêt des Plastines dans le cadre du diagnostic du cancer colorectal à partir d'un prélèvement de sérum ou de selles. De plus la Plastine-I n'a même jamais été envisagée comme un marqueur potentiel du cancer²⁸. La Demanderesse a montré quant à elle de façon surprenante que cette protéine est un bon marqueur dans les échantillons biologiques issus d'un patient atteint d'un cancer colorectal, lesdits échantillons étant distants ou non de la tumeur.

La concentration du marqueur tumoral choisi dans le Groupe A sera, selon le marqueur considéré, augmentée ou diminuée dans l'échantillon biologique dans lequel on met en oeuvre le procédé de l'invention par rapport aux valeurs de référence déterminées pour les patients sains.

Le procédé de l'invention peut également être amélioré en associant la détection de l'Apolipoprotéine AII et d'un autre marqueur tumoral choisi dans le Groupe B, à savoir : les marqueurs Beta 2 Microglobuline, Protéasome 20S, Galectine-3, L-Lactate Deshydrogénase Chaîne B, Calréticuline, Regenerating Islet-Derived Protein 3 Alpha, Tumor-Associated Calcium Signal Transducer 1, Keratine type II Cytoskeletal 8, Keratine type I Cytoskeletal 18, Keratine type I Cytoskeletal 19, Epithelial-Cadhéiine, ACE, Villine, CA19-9, CA 242, CA 50, CA 72-2, Testostérone, TIMP-1, Cripto-1, Intélectine-1, Protéine Disulfide Isomérase, Cytokératine 20, Translationally-Controlled Tumor Protein, (Pro)défensine-A5, la détection de fragments d'ADN dans le sang ayant des altérations spécifiques de leur profil de méthylation, comme par exemple l'ADN méthylé du gène AXL4 (Aristaless-like Homeobox-4 Gene Methylation) ou l'ADN méthylé du gène Septin-9, la détection d'altérations spécifiques de fragments d'ADN dans les selles comme des mutations spécifiques de l'ADN dans les selles ou des altérations spécifiques du profil de méthylation de l'ADN dans les selles, la détection d'hémoglobine humaine dans les selles. Bien entendu, le procédé de l'invention pourra également mettre en oeuvre la détection, dans le même dosage, de l'Apolipoprotéine AII, d'au moins un marqueur tumoral choisi dans le Groupe B et d'au moins un autre marqueur tumoral choisi dans le Groupe A.

Le marqueur Beta 2 Microglobuline (N° Swiss Prot P61769, également appelé β2 Microglobuline, β2M) est une protéine de basse masse moléculaire (11 à 12 kDa) trouvée à la surface de la plupart des cellules humaines nucléées. Le taux de β2 Microglobuline sérique augmente chez certains patients atteints de cancer, sans que cette augmentation soit spécifique, ni corrélée avec la nature de la tumeur, son stade ou la sévérité de la maladie. Une augmentation significative est également observée lors d'autres maladies telles que le lupus érythémateux, l'arthrite rhumatoïde, le syndrome de Sjogren, les maladies malignes du système lymphoïde (myélome multiple, lymphome à cellules B), certaines maladies virales (hépatites ou SIDA) et chez les patients hémophiles. La β2 Microglobuline étant filtrée par les glomérules rénaux et réabsorbée par les tubes contournés proximaux, sa concentration sanguine peut être modifiée en cas de pathologies rénales. C'est ainsi que le dosage de la β2 Microglobuline est le plus souvent réservé au diagnostic de pathologies rénales, ou au suivi d'infection par le virus de l'immunodéficience acquise. Toutefois, ce marqueur est connu comme un marqueur tumoral, notamment du cancer du côlon.

Le marqueur Protéasome 20S (également appelé Prosome) est la structure centrale du protéasome qui est lui-même un complexe moléculaire responsable de la dégradation intracellulaire des protéines ubiquitinylées²⁹. Le Protéasome est un complexe moléculaire de 700 kDa constitué de 28 sous-unités associées en 4 anneaux de 7 sous-unités. Chez l'homme, 7 unités alpha (α1, α2, α3, α4, α5, α6 et α7) et 10 unités bêta (β1, β2, β3, β4, β5, β6, β7, β1i, β2i et β5i) sont connues. Grâce à ses propriétés catalytiques, le Protéasome joue un rôle central dans les mécanismes de prolifération, de croissance, de régulation et d'apoptose cellulaire et donc dans les voies de cancérisation. L'inhibition du Protéasome par le Bortezomib (Velcade) est un traitement reconnu des myélomes multiples. Des essais thérapeutiques de phase II ou III sont en cours pour des cancers hématologiques ou des tumeurs. Lavabre-Bertrand et al.³⁰ ont montré que le taux sérique de Protéasome pouvait s'élever à l'occasion de certaines pathologies, notamment en cas de cancers (myélome, lymphome et tumeurs solides).

Le marqueur Galectine-3 (N° Swiss Prot P17931, également appelé Gal-3, Galactose-specific lectin 3, MAC-2 antigen, IgE-binding protein, 35 kDa lectin, Carbohydrate binding protein 35, CBP 35, Laminin-binding protein, Lectin L-29, L-31,Galactoside-binding protein ou GALBP), est une lectine capable de se lier à des structures beta-galactosidique de type N-acetyllactosamine. C'est une protéine à fonctions multiples impliquée dans diverses fonctions biologiques, incluant l'adhésion des cellules tumorales, la prolifération, la différentiation, l'angiogénèse, l'apoptose, la progression cancéreuse métastasique³¹. Différents travaux ont montré que Gal-3 pouvait se complexer avec de nombreuses molécules : ACE, IgE, Laminine, Mucine, Mac-2BP, LAMP1, LAMP2, Fibronectine, etc. Un dosage sérique de Gal-3 a été décrit par I. Iurisci et al.³². Gal-3 était capturée sur des microplaques revêtue de Mac-2-binding protein (une protéine liant Gal-3) puis révélée avec un anticorps de rat anti-Gal-3. Cette étude a montré une élévation sérique de Gal-3 en cas de cancers gastro-intestinaux, du sein, du poumon, de l'ovaire, de mélanomes et de lymphomes non-Hodgkinien.

Le marqueur L-Lactate Deshydrogénase Chaîne B (N° Swiss Prot P07195, également appelé LDH-B, LDH Heart Unit ou LDH-H) est une protéine pouvant se complexer sous forme d'homotétramère. Cette protéine peut également se complexer avec la protéine L-Lactate Deshydrogénase Chaîne A (N° Swiss Prot P00338, également appelé LDH-A, LDH Muscle Unit ou LDH-M) sous forme d'hétrotétramères. La dose sérique et/ou l'activité enzymatique sérique des complexes tétramèrique, baptisé LDH, augmente dans la circulation sanguine proportionnellement à la masse tumorale pour de nombreuses tumeurs solides. Son utilisation est recommandée en association avec la gonadotrophine chorionique humaine (beta-hCG) et la phosphatase alcaline placentaire pour le suivi des cancers séminaux. La LDH est considérée comme un marqueur d'intérêt pour le pronostic des lymphomes, de la leucémie et du cancer du côlon³³.

Le marqueur Calréticuline (N° Swiss Prot P27797, également appelé CRP55, Calregulin, HACBP, ERp60 ou grp60) est une protéine multifonctionnelle. C'est une lectine capable d'interagir transitoirement avec la quasi-totalité des protéines monoglycosylées du réticulum endoplasmique. C'est ainsi que D.J. McCool et al.³⁴ ont montré que la Calréticuline, était impliquée dans la maturation de la mucine colique MUC2. Une méthode de diagnostic du CCR utilisant un dosage de la Calréticuline dans un tissu, les selles ou un fluide corporel est décrit dans la demande de brevet WO03/065003.

Le marqueur Regenerating Islet-Delived Protein 3 Alpha (N° Swiss Prot Q06141, également appelé Reg III-alpha, Pancreatitis-associated protein 1 ou Pancreatis Associated Protein I (PAP 1)) est une protéine faiblement exprimée dans le pancréas sain. Elle est surexprimée durant les phases aiguës de pancréatite et chez certains patients souffrant de pancréatite chronique. Elle apparaît alors dans le liquide pancréatique et dans la circulation sanguine³⁵. Y. Motoo et al.³⁶ ont montré par dosage ELISA que le taux de PAP 1 sanguine augmentait chez certains patients atteints de cancer du côlon, de l'estomac, du foie ou du pancréas, ainsi qu'en cas d'insuffisance rénale. Ils ont pour ce faire utilisé le test ELISA (PANCEPAP) commercialisé par la société Dynabio (La Gaude, France).

Le marqueur Tumor-Associated Calcium Signal Transducer 1 (N° Swiss Prot P16422, également appelé Major gastrointestinal tumor-associated protein GA733-2, Epithelial cell surface antigen, EpCAM, Epithelial glycoprotein, EGP, Adenocarcinoma-associated antigen, KSA, KS 1/4 antigen, Cell surface glycoprotein Trop-1 ou CD326 antigen) a été caractérisé en 1979 par sa capacité à être reconnu par un anticorps dirigé contre des cellules de cancer colorectal³⁷. Cette protéine est connue sous différents noms, comme indiqué précédemment, mais l'usage le plus fréquent est de l'appeler EpCAM. C'est une protéine transmembranaire exprimée sur la surface basolatérale des cellules, dans certains épithéliums et de nombreux cancers³⁸. Dès 1982 Herlyn et al.³⁹ ont montré que l'injection d'un anticorps monoclonal anti-EpCAM pouvait inhiber la croissance tumorale de patients atteints de cancer colorectal. Ces résultats ont conduit au développement d'un traitement anti-tumoral à base d'un anticorps anti-EpCAM nommé Edrecolomab. Ce traitement est commercialisé sous le nom de Panorex™. Par ailleurs, H Abe et al.⁴⁰ ont montré par dosage ELISA qu'une forme soluble d'EpCAM, baptisée MK-1, était augmentée dans la circulation sanguine de 10% chez les patients cancéreux étudiés.

Les cytokératines font partie des protéines qui composent les filaments intermédiaires du cytosquelette des cellules épithéliales. Actuellement, plus de 20 cytokératines humaines ont été identifiées. Les cytokératines 8 (N° Swiss Prot P05787, également appelé Cytokeratin-8, CK-8, Keratin-8 ou K8), 18 (N° Swiss Prot P05783, également appelé Cytokeratin-18, CK-18, Keratin-18 ou K18), et 19 (N° Swiss Prot P08727, également appelé Cytokeratin-19, CK-19, Keratin-19 ou K19) sont les plus abondantes dans les cellules épithéliales et sont des outils utiles pour le diagnostic de pathologies cancéreuses⁴¹. Cet intérêt clinique est lié à la libération de cytokératines par les cellules épithéliales en phase d'apoptose ou de prolifération. En cas d'apoptose, cette libération se fait sous forme de fragments solubles qui semble apparaître sous l'action protéolytique de Caspases. Des formes de cytokératines non dégradées n'ont jamais été décrites dans la circulation sanguine. Les trois dosages de cytokératines les plus utilisés en clinique sont le dosage de l'antigène polypeptidique tissulaire (TPA), de l'antigène polypeptidique tissulaire spécifique (TPS), et CYFRA 21-1. TPA est un test de spectre large qui mesure les cytokératines 8, 18, et 19. Les dosages de TPS et de CYFRA 21-1 sont plus spécifiques et mesurent respectivement des fragments de la cytokératine 18 et de la cytokératine 19. Ces 3 dosages détectent des fragments solubles de cytokératines pouvant être présents isolément ou sous forme de complexes protéiques. TPA, TPS ou CYFRA-21-1 ont été utilisés pour le suivi thérapeutique des cancers colorectaux, du sein, du poumon, de la vessie, de l'ovaire, du pancréas, de la prostate et de certains cancers ORL. Le dosage sanguin des fragments solubles de cytokératines a en effet une valeur clinique pour dépister les récidives ou évaluer la réponse à la thérapie engagée (radiothérapie, chimiothérapie, traitement hormonal). Un dosage régulier permet notamment d'évaluer la progression de la masse tumorale. La dose de cytokératines sanguines solubles a également un aspect pronostique vis-à-vis du stade tumoral et de la formation de métastase. Actuellement le dosage sanguin de cytokératine le plus utilisé est CYFRA 21-1. Il est fortement recommandé pour le suivi de patients atteints de cancer du poumon non à petites cellules. Il existe divers dosages commerciaux pour TPA (AB Sangtec Medical Co., Byk-Roland...), TPS (IDL Biotech AB, BEKI Diagnostics...) et CYFRA-21-1 (Roche Diagnostics, CIS Bio-International, Fujirebio Diagnostics...). Par ailleurs, H. Kim et al.⁴² ont montré que le dosage dans les selles de cytokératine 19 (DiNonA Inc.) pouvait être utile au dépistage de maladies digestives en association avec un dosage de sang occulte dans les selles. Enfin, l'utilisation de la Cytokératine 20 (N° Swiss Prot P35900, également appelé Keratin, type I cytoskeletal 20, CK-20, Keratin-20, K20, ou Protein IT) en tant que marqueur dans le cancer colorectal est décrite dans la demande de brevet US2002/0160382.

Le marqueur Epithelial-Cadhérine (N° Swiss Prot P12830, également appelé E-cadherin, Uvomorulin, Cadherin-1, CAM 120/80 ou CD324 antigen) est une protéine transmembranaire médiatrice de l'adhésion cellulaire calcium dépendante. Elle est spécifiquement exprimée dans les cellules épithéliales, où elle est impliquée dans le maintien de leur phénotype. Le domaine cytoplasmique de l'E-Cadhérine se lie à la β-Caténine, qui est elle-même liée aux réseaux de filaments d'actine du cytosquelette. Cette liaison l'E-Cadhérine/β-Caténine joue un rôle critique pour stabiliser les adhésions cellules/cellules du tissu épithélial. La perte d'E-Cadhérine peut donc réduire l'adhésion cellulaire et augmenter le pouvoir invasif des cellules cancéreuses. Une réduction d'expression d'E-Cadhérine ou de β-Caténine est généralement associée avec une dédifférenciation et une agressivité plus importante de la tumeur, notamment pour les cancers digestifs. C'est ainsi que F. Roca et al.⁴³ ont montré que les patients atteints d'un cancer colorectal et sous-exprimant l'E-Cadhérine avaient un pronostic plus péjoratif que les patients ayant un niveau d'expression normal. Dès 1983, Damsky et al.⁴⁴ ont montré qu'une forme soluble d'E-Cadhérine pouvait être libérée par la lignée cellulaire du cancer du sein MCF-7. Cette forme soluble correspond au clivage de la partie extracellulaire de l'E-Cadhérine. Plus tard, M. Katayama et al.⁴⁵ ont montré que la forme soluble de l'E-Cadhérine pouvait être libérée dans la circulation sanguine en cas de cancer et C. Willmanns et al.⁴⁶ ont montré que l'augmentation de la dose d'E-Cadhérine sanguine était corrélée au stade tumoral pour les cancers colorectaux. Un kit commercial est par ailleurs proposé par la société Takara BioChemicals (Tokyo, Japon).

Le dosage de l'ACE (Antigène Carcino-Embryonnaire) pour le diagnostic du cancer colorectal a été proposé depuis 1965 par P. Gold et S. Freedman⁴⁷, mais un dosage sanguin de ce marqueur a une sensibilité médiocre pour le diagnostic de cancers colorectaux à un stade peu avancé. C'est ainsi que le dosage de l'ACE sérique est surtout recommandé pour évaluer le risque de métastases hépatiques⁴⁸ et pour le suivi thérapeutique. En outre, c'est un marqueur peu spécifique du cancer colorectal, il peut en effet être augmenté dans de nombreux autres cancers (poumon, sein, ...). En revanche, le dosage d'ACE dans les selles semble plus sensible et plus spécifique que le dosage de l'ACE sérique ou que le dosage de sang dans les selles⁴⁹. Ce dosage n'est toutefois pas encore proposé en routine.

Les déterminants antigéniques 1116-NS-19-9 réactifs, plus communément baptisés CA19-9 (Carbohydrate Antigen 19.9), sont portés par des protéines de poids moléculaires élevés⁵⁰. Le dosage sanguin de CA 19-9 est plus spécifique que celui de l'ACE. Le taux de CA 19-9 sanguin augmente en cas de cancer colorectal, du pancréas et du foie (cholangiocarcinome), mais aussi en cas de pathologies non cancéreuses (cholangites...). Son usage en association avec l'ACE est recommandé tant au moment du diagnostic d'un cancer que pour le suivi de la pathologie.

J. Holmgren et al.⁵¹ ont montré que la dose sérique d'antigène CA 50 était augmentée en cas de cancer colorectal. L'antigène CA 50 est défini par sa faculté à être reconnu par un anticorps monoclonal spécifique.

S'agissant du marqueur CA 72, T.L. Klug et al.⁵² ont montré que la dose sérique d'antigène CA 72 était augmentée en cas de cancer colorectal. L'antigène CA 72 est défini par sa faculté à être reconnu par un anticorps monoclonal spécifique.

De même, P. Kuusela et al.⁵³ ont montré que la dose sérique d'antigène CA 242 était augmentée en cas de cancer colorectal. L'antigène CA 242 est défini par sa faculté à être reconnu par un anticorps monoclonal spécifique.

Le dosage de la Testostérone pour le diagnostic du cancer colorectal a été proposé chez les hommes par M. Holland et al.⁵⁴. Ces auteurs ont montré un effondrement du taux de Testostérone sanguine en cas de cancer colorectal.

S'agissant du marqueur TIMP-1, ou Tissue Inhibitor of Matrix Metalloproteinase Type-1, la demande de brevet US 2007/0020707 décrit notamment le dosage de TIMP-1 pour le diagnostic du cancer colorectal à l'aide d'un dosage dans un fluide corporel.

F. Model et al.⁵⁵ ont montré en juillet 2006, lors du congrès World Congress on Gastrointestinal Cancer, qu'il était possible de détecter des formes méthylées du gène de la Septin-9 dans le plasma de patients atteints de cancer colorectal.

M.P. Ebert et al.⁵⁶ ont montré que le gène ALX4, ou Aristaless-like homeobox-4, était plus souvent méthylé dans les sérums de patients atteints d'un cancer colorectal que dans les sérum contrôles (*P* < 0,0001). En utilisant une valeur seuil de 41,4 pg/mL, ils ont obtenu une sensibilité de 83,3% et une spécificité de 70%.

La Villine est décrite en tant que marqueur sanguin pour le diagnostic du cancer colorectal dans la demande de brevet FR2581456.

C. Bianco et al.⁵⁷ ont montré que la dose sérique de Cripto-1 était augmentée en cas de cancer colorectal.

Le dosage de l'Intélectine-1 (N° Swiss Prot Q8WWA0, également appelé Intestinal lactoferrin receptor, Galactofuranose-binding lectin, Endothelial lectin HL-1 ou Omentin) pour le diagnostic du cancer colorectal a été décrit dans la demande de brevet US2003/0082533.

L'utilisation de la Protéine Disulfide Isomérase (N° Swiss Prot P07237, également appelé EC 5.3.4.1, PDI, Prolyl 4-hydroxylase subunit beta, Cellular thyroid hormone-binding protein ou p55), de la Translationally-Controlled Tumor Protein (N° Swiss Prot P13693, également appelé TCTP, p23, Histamine-releasing factor, HRF ou Fortilin) et de la (Pro)défensine-A5 (N° Swiss Prot Q01523) en tant que marqueurs dans le cancer colorectal est décrite respectivement dans les demandes de brevet EP1724586, US2003/0172388 et US2006/0179496. Par (Pro)défensine, on entend, le précurseur, à savoir la Prodéfensine avant clivage, le propeptide, à savoir la moitié N-terminale après clivage de la Prodéfensine, et la protéine mature, à savoir la Défensine, correspondant à la moitié C-terminale après clivage.

Enfin, le dosage d'hémoglobine humaine dans les selles est connu et peut être mis en oeuvre comme décrit précédemment.

La concentration du marqueur tumoral choisi dans le Groupe B sera, selon le marqueur considéré, augmentée ou diminuée dans l'échantillon biologique dans lequel on met en oeuvre le procédé de l'invention par rapport aux valeurs de référence déterminées pour les patients sains.

De préférence, le ou les marqueurs tumoraux du groupe B sont choisis parmi : les marqueurs : Beta 2 Microglobuline, Protéasome 20S, Galectine-3, L-Lactate Deshydrogénase Chaîne B, Calréticuline, Regenerating Islet-Derived Protein 3 Alpha, Tumor-Associated Calcium Signal Transducer 1, Epithelial-Cadhérine, ACE, CA19-9, Testostérone, TIMP-1, Intélectine-1, Protéine Disulfide Isomérase, Cytokératine 20, Translationally-Controlled Tumor Protein, (Pro)défensine-A5, détection d'hémoglobine humaine dans les selles.

Bien entendu, le procédé de l'invention peut également inclure la détection de tout autre marqueur du cancer colorectal connu de l'homme du métier.

Comme indiqué précédemment, on détecte le ou les marqueurs tumoraux d'intérêt soit sous forme de protéine, soit sous forme d'ARN messager, soit par altération de l'ADN correspondant (mutation ou modification des méthylations).

La détermination de la présence, dans l'échantillon biologique, du marqueur tumoral d'intérêt « protéine » peut être mis en oeuvre par tout procédé de détermination de la présence d'une protéine dans un échantillon, connu de l'homme du métier, comme par exemple par un test biochimique, y compris un dosage immunologique, ou par spectrométrie de masse.

Le test biochimique peut être tout test largement connu de l'homme du métier impliquant des interactions moléculaires, à savoir des réactions entre ledit marqueur tumoral et un ou des partenaire(s) de liaison spécifique(s) ou non dudit marqueur tumoral.

De préférence, le test biochimique est un dosage immunologique connu de l'homme du métier impliquant des réactions immunologiques entre le marqueur tumoral qui est l'antigène et un ou des partenaire(s) de liaison spécifique(s) que sont les anticorps dirigés contre cet antigène.

Les partenaires de liaison spécifiques ou non du ou des marqueurs tumoraux recherchés dans le procédé de l'invention sont tout partenaire susceptible de se lier à ce ou ces marqueurs. Ils sont dits spécifiques quand ils sont capables de se lier à ces marqueurs avec une spécificité élevée, voire une spécificité de 100%. Ils sont dits non spécifiques lorsque leur spécificité de liaison à ces marqueurs est faible et qu'ils sont alors capables de se lier à d'autres ligands, tels que des protéines. A titre d'exemple, on peut citer les anticorps, les fractions d'anticorps, les récepteurs et toute autre molécule capable de se lier à ce marqueur.

Les anticorps partenaires de liaison sont par exemple soit des anticorps polyclonaux, soit des anticorps monoclonaux.

Les anticorps polyclonaux peuvent être obtenus par immunisation d'un animal avec le marqueur tumoral concerné, suivie de la récupération des anticorps recherchés sous forme purifiée, par prélèvement du sérum dudit animal, et séparation desdits anticorps des autres constituants du sérum, notamment par chromatographie d'affinité sur une colonne sur laquelle est fixé un antigène spécifiquement reconnu par les anticorps, notamment ledit marqueur.

Les anticorps monoclonaux peuvent être obtenus par la technique des hybridomes dont le principe général est rappelé ci-après.

Dans un premier temps, on immunise un animal, généralement une souris avec le marqueur tumoral d'intérêt, dont les lymphocytes B sont alors capables de produire des anticorps contre ledit antigène. Ces lymphocytes producteurs d'anticorps sont ensuite fusionnés avec des cellules myélomateuses "immortelles" (murines dans l'exemple) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clone, chacun conduisant à la production d'un anticorps monoclonal dont les propriétés de reconnaissance vis-à-vis dudit marqueur tumoral pourront être testées par exemple en ELISA, par immunotransfert (Western blot) en une ou deux dimensions, en immunofluorescence, ou à l'aide d'un biocapteur. Les anticorps monoclonaux ainsi sélectionnés, sont par la suite purifiés notamment selon la technique de chromatographie d'affinité décrite ci-dessus.

Les anticorps monoclonaux peuvent être également des anticorps recombinants obtenus par génie génétique, par des techniques bien connues de l'homme du métier.

Des exemples d'anticorps anti-Leucocyte Elastase Inhibitor sont connus et sont disponibles notamment dans le catalogue Abcam, anticorps polyclonal de lapin anti-LEI, Cat. No. Ab47731. Un anticorps monoclonal anti-LEI Clone ELA-1 a été décrit dans l'article de R. Yasumatsu et al.⁵⁸.

Des exemples d'anticorps anti-Ezrine sont connus et sont disponibles notamment dans le catalogue Abcam, anticorps monoclonal anti-Ezrine Clone 3C12, Cat. No. Ab4069 et anticorps polyclonal de lapin anti-Ezrine, Cat. No. Ab47418.

Des exemples d'anticorps anti-Aminoacylase 1 sont connus et sont disponibles notamment dans le catalogue Abnova, anticorps monoclonal anti-Aminoacylase 1 Clone 4F1-B7, Cat. No. H00000095-M01, et dans le catalogue Abcam, anticorps polyclonal de poule anti-Aminoacylase 1, Cat. No. Ab26173.

Des exemples d'anticorps anti-Liver Fatty Acid-Binding Protein sont connus et sont disponibles notamment dans le catalogue Abcam, anticorps monoclonal anti-L-FABP Clone 6B6, Cat. No. Ab10059 et anticorps polyclonal de lapin anti-L-FABP, Cat. No. Ab7807.

Des exemples d'anticorps anti-Intestinal Fatty Acid-Binding Protein sont connus et sont disponibles notamment dans le catalogue R&D Systems, anticorps monoclonal anti-I-FABP Clone 323701, Cat. No. MAB3078, et dans le catalogue Abcam, anticorps polyclonal de lapin anti-I-FABP, Cat. No. Ab7805.

Des exemples d'anticorps anti-Apolipoprotéine AI sont connus et sont disponibles notamment dans le catalogue Biodesign Meridian Life Sciences, anticorps monoclonal anti-Apo AI Clone 4A90, Cat. No. H45402M et anticorps polyclonal de chèvre anti-Apo AI, Cat. No. K45252P.

Des exemples d'anticorps anti-Apolipoprotéine AII sont connus et sont disponibles notamment dans le catalogue US Biological, anticorps monoclonal anti-Apo AII Clone 1402, Cat. No. A2299-31C et dans le catalogue Biodesign Meridian Life Sciences anticorps polyclonal de chèvre anti-Apo AII, Cat. No. K74001P.

Des exemples d'anticorps polyclonaux anti-Plastine-I sont connus et sont disponibles notamment dans le catalogue Santa Cruz Biotechnology. L'anticorps polyclonal de lapin H-300 (Cat. No. sc-28531) réagit avec les Plastines-I, L et T. La Demanderesse a mis au point des anticorps monoclonaux dirigés contre la Plastine-I.

Des exemples d'anticorps anti-Beta2 Microglobuline, anti-ACE, anti-CA19-9 et anti-Testostérone sont connus et sont notamment utilisés dans les trousses de dosage de la Demanderesse, respectivement Vidas® 2 Microglobulin, Vidas® ACE, Vidas® CA19-9™ et Vidas® Testostérone.

Des exemples d'anticorps anti-Protéasome 20S sont connus et sont disponibles notamment dans le catalogue d'Affinitiy Research Products.

Des exemples d'anticorps anti-Galectine-3, anti-L-Lactate Deshydrogénase Chaîne B, anti-Calréticuline, anti-Tumor-Associated Calcium Signal Transducer 1, anti-Keratine type II Cytoskeletal 8, anti-Keratine type I Cytoskeletal 18, anti-Keratine type I Cytoskeletal 19, anti-Epithelial-Cadhérine, anti-Villine et anti-TIMP-1 sont connus et sont disponibles notamment dans le catalogue Abcam.

Des exemples d'anticorps anti-Regenerating Islet-Derived Protein 3 Alpha sont connus et sont notamment utilisés dans les trousses de dosage de Dynabio (La Gaude, France).

Des exemples d'anticorps anti-CA 242, anti-CA 50, anti-CA 72-4 sont connus et sont disponibles notamment dans le catalogue Fujirebio.

Des exemples d'anticorps anti-Intélectine-1 sont connus et sont disponibles notamment dans le catalogue Alexis Biochemicals, anticorps monoclonal anti-Intélectine-1 Clone Saly-1, Cat. No. ALX-804-850-C100 et anticorps polyclonal de lapin anti-Intélectine-1, Cat. No. ALX-210-941.

Des exemples d'anticorps anti-Protein Disulfide Isomérase sont connus et sont disponibles notamment dans le catalogue Abcam, anticorps monoclonal anti-PDI Clone RL77, Cat. No. Ab5484 et anticorps polyclonal de lapin anti-PDI, Cat. No. Ab3672.

Des exemples d'anticorps anti-Cytokératine 20 sont connus et sont disponibles notamment dans le catalogue Abcam, anticorps monoclonal anti-Cytokératine 20 Clone Ks20.8, Cat. No. Ab962 et anticorps polyclonal de lapin anti-Cytokératine 20, Cat. No. Ab36756.

Des exemples d'anticorps anti-TCTP sont connus et sont disponibles notamment dans le catalogue Abnova, anticorps monoclonal anti-TCTP Clone 3C7, Cat. No. 157H00007178-M01 et anticorps polyclonal anti-TCTP, Cat. No. 157H00007178-A01.

Des exemples d'anticorps anti-Défensine-A5 sont connus et sont disponibles notamment dans le catalogue Santa Cruz Biotechnology, anticorps monoclonal anti-Défensine-A5 Clone 8C8, Cat. No. sc-53997, et dans le catalogue Alpha Diagnostic International Inc., anticorps polyclonal de lapin anti-Défensine-A5, Cat. No. HDEFA51-A.

Les partenaires de liaison spécifiques ou non du ou des marqueurs tumoraux recherchés dans le procédé de l'invention peuvent être utilisés comme réactif de capture, comme réactif de détection ou comme réactifs de capture et de détection.

La visualisation des réactions immunologiques, c'est-à-dire de la liaison marqueur tumoral/partenaire de liaison, peut être effectuée par tout moyen de détection, tels que des moyens directs ou indirects.

Dans le cas de la détection directe, c'est-à-dire sans l'intermédiaire d'un marquage, on observe les réactions immunologiques par exemple par résonance plasmonique de surface ou par voltamétrie cyclique sur une électrode portant un polymère conducteur.

La détection indirecte se fait par l'intermédiaire d'un marquage, soit du partenaire de liaison dit réactif de révélation, soit du marqueur tumoral d'intérêt lui-même. On parle alors dans ce dernier cas de méthode de compétition.

Par marquage, on entend la fixation d'un réactif marqueur capable de générer directement ou indirectement un signal détectable. Une liste non limitative de ces réactifs marqueurs consiste en :
- les enzymes qui produisent un signal détectable par exemple par colorimétrie, fluorescence, luminescence, comme la peroxydase de raifort, la phosphatase alcaline, la β-galactosidase, la glucose-6-phosphate déshydrogénase,
- les chromophores comme les composés fluorescents, luminescents, colorants,
- les molécules radioactives comme le ³²P, le ³⁵S ou le ¹²⁵I, et
- les molécules fluorescentes telles que les Alexa ou les phycocyanines.

Des systèmes indirects de détection peuvent être aussi utilisés, comme par exemple des ligands capables de réagir avec un anti-ligand. Les couples ligand/anti-ligand sont bien connus de l'homme du métier, ce qui est le cas par exemple des couples suivants : biotine/streptavidine, haptène/anticorps, antigène/anticorps, peptide/anticorps, sucre/lectine, polynucléotide/complémentaire du polynucléotide. Dans ce cas, c'est le ligand qui porte le partenaire de liaison. L'anti-ligand peut être détectable directement par les réactifs marqueurs décrits au paragraphe précédent ou être lui-même détectable par un ligand/anti-ligand.

Ces systèmes indirects de détection peuvent conduire, dans certaines conditions, à une amplification du signal. Cette technique d'amplification du signal est bien connue de l'homme du métier, et l'on pourra se reporter aux demandes de brevet antérieures FR98/10084 ou WO-A-95/08000 de la Demanderesse ou à l'article de Chevalier et al.⁵⁹.

Selon le type de marquage utilisé, l'homme du métier ajoutera des réactifs permettant la visualisation du marquage.

A titre d'exemple de tests immunologiques tels que définis ci-dessus, on peut citer les méthodes « sandwich » telles qu'ELISA, IRMA et RIA, les méthodes dites de compétition et les méthodes d'immunodétection directe comme l'immunohistochimie, l'immunocytochimie, le Western-blot et le Dot-blot.

La spectrométrie de masse peut également être utilisée pour la détection dans le fluide biologique du ou des marqueurs tumoraux recherchés dans le procédé de l'invention. Le principe de la spectrométrie est largement connu de l'homme du métier et est décrit par exemple dans Patterson, S.⁶⁰.

Pour ce faire, l'échantillon biologique préalablement traité ou non est passé dans un spectromètre de masse et on compare le spectre obtenu avec celui du ou des marqueurs tumoraux recherchés dans le procédé de l'invention. Un exemple de traitement préalable de l'échantillon consiste à le faire passer sur un support d'immunocapture, comportant un des partenaires de liaison du ou des marqueurs tumoraux recherchés dans le procédé de l'invention, par exemple un anticorps dirigé contre le ou les marqueurs tumoraux recherchés dans le procédé de l'invention. Un autre exemple de traitement préalable de l'échantillon peut être le pré-fractionnement de l'échantillon biologique, afin de séparer entre elles les protéines de l'échantillon. Dans des techniques bien connues de l'homme du métier, on peut par exemple tout d'abord dépléter les protéines majoritaires de l'échantillon.

Grâce à des avancées technologiques récentes, il est également devenu possible de quantifier des protéines dans les milieux biologiques complexes en utilisant la spectrométrie de masse en tandem (MS/MS) mise en oeuvre en utilisant un analyseur triple quadripôle fonctionnant en mode MRM (multiple reaction monitoring). Ce mode de fonctionnement présente une double sélectivité (deux analyseurs, sélections de l'ion parent et de l'ion produit) et la sensibilité de détection est améliorée par rapport à d'autres modes de balayage. La faisabilité technique de cette approche a été démontrée récemment par Anderson et Hunter⁶¹ qui ont réussi à détecter des protéines dont la concentration est de l'ordre de la centaine de ng/ml dans lé plasma, après immunodépletion des protéines les plus abondantes.

La détermination de la présence, dans l'échantillon biologique, du marqueur tumoral d'intérêt « ARNm » peut être mis en oeuvre par tout procédé de détermination de la présence d'ARNm dans un échantillon, à savoir soit la détection directe de l'ARNm, soit la détection indirecte de l'ARNm, ou tout autre procédé de détermination de la présence d'un ARN dans un échantillon, connu de l'homme du métier.

Par détection directe de l'ARNm, on entend la mise en évidence de l'ARNm lui-même dans l'échantillon biologique.

La détection directe de l'ARNm dans l'échantillon biologique peut être mise en oeuvre par tout moyen connu de l'homme du métier, comme par exemple par hybridation avec un partenaire de liaison spécifique de l'ARNm, le cas échéant après amplification par la technique PCR ou NASBA.

Par hybridation, on entend le processus au cours duquel, dans des conditions appropriées, deux fragments nucléotidiques se lient avec des liaisons hydrogènes stables et spécifiques pour former un complexe double brin. Ces liaisons hydrogène se forment entre les bases complémentaires Adénine (A) et Thymine (T) (ou Uracile (U)) (on parle de liaison A-T) ou entre les bases complémentaires Guanine (G) et Cytosine (C) (on parle de liaison G-C). L'hybridation de deux fragments nucléotidiques peut être totale (on parle alors de fragments nucléotidiques ou de séquences complémentaires), c'est-à-dire que le complexe double brin obtenu lors de cette hybridation comprend uniquement des liaisons A-T et des liaisons C-G. Cette hybridation peut être partielle (on parle alors de fragments nucléotidiques ou de séquences suffisamment complémentaires), c'est-à-dire que le complexe double brin obtenu comprend des liaisons A-T et des liaisons C-G permettant de former le complexe double brin, mais également des bases non liées à une base complémentaire. L'hybridation entre deux fragments nucléotidiques dépend des conditions opératoires qui sont utilisées, et notamment de la stringence. La stringence est définie notamment en fonction de la composition en bases des deux fragments nucléotidiques, ainsi que par le degré de mésappariement entre deux fragments nucléotidiques. La stringence peut également être fonction des paramètres de la réaction, tels que la concentration et le type d'espèces ioniques présentes dans la solution d'hybridation, la nature et la concentration d'agents dénaturants et/ou la température d'hybridation. Toutes ces données sont bien connues et les conditions appropriées peuvent être déterminées par l'homme du métier. En général, selon la longueur des fragments nucléotidiques que l'on souhaite hybrider, la température d'hybridation est comprise entre environ 20 et 70°C, en particulier entre 35 et 65°C dans une solution saline à une concentration d'environ 0,5 à 1 M. Les partenaires de liaison spécifiques ou non de l'ARNm sont tout partenaire susceptible de se lier à cet ARNm. A titre d'exemple, on peut citer les sondes nucléiques, les amorces d'amplification, et toute autre molécule capable de se lier à cet ARNm.

Par sonde d'hybridation, on entend un fragment nucléotidique comprenant de 5 à 100 motifs nucléiques, notamment de 10 à 35 motifs nucléiques, possédant une spécificité d'hybridation dans des conditions déterminées pour former un complexe d'hybridation avec le matériel spécifique du gène cible d'intérêt. La sonde d'hybridation peut comprendre un marqueur permettant sa détection.

Au sens de la présente invention, on entend par amorce d'amplification, un fragment nucléotidique comprenant de 5 à 100 motifs nucléiques, préférentiellement de 15 à 30 motifs nucléiques permettant l'initiation d'une polymérisation enzymatique, telle que notamment une réaction d'amplification enzymatique. Par réaction d'amplification enzymatique, on entend un processus générant de multiples copies d'un fragment nucléotidique par l'action d'au moins une enzyme. De telles réactions d'amplification sont bien connues de l'homme du métier et on peut citer notamment les techniques suivantes :
- PCR (Polymerase Chain Reaction), telle que décrite dans les brevets US 4,683,195, US 4,683,202 et US 4,800,159,
- NASBA (Nucleic Acid Sequence-Based Amplification) avec la demande de brevet WO 91/02818, et
- TMA (Transcription Mediated Amplification) avec le brevet US 5,399,491.

Par détection, on entend soit une méthode physique, soit une méthode chimique avec un agent colorant intercalant tel que SYBR® Green I ou le bromure d'éthydium, soit une méthode de détection à l'aide d'un marqueur. De nombreuses méthodes de détection existent pour la détection des acides nucléiques⁶². Les marqueurs appropriés sont tels que définis précédemment.

Au sens de la présente invention, la sonde d'hybridation peut être une sonde dite de détection. Dans ce cas, la sonde dite de détection est marquée au moyen d'un marqueur tel que défini précédemment. Grâce à la présence de ce marqueur, on peut détecter la présence d'une réaction d'hybridation entre une sonde de détection donnée et le transcrit à détecter.

La sonde de détection peut être notamment une sonde de détection « molecular beacons »⁶³. Ces « molecular beacons » deviennent fluorescentes lors de l'hybridation. Elles possèdent une structure de type tige-boucle et contiennent un fluorophore et un groupe « quencher ». La fixation de la séquence de boucle spécifique avec sa séquence complémentaire d'acide nucléique cible provoque un déroulement de la tige et l'émission d'un signal fluorescent lors de l'excitation à la longueur d'onde qui convient.

La sonde d'hybridation peut être également une sonde dite de capture. Dans ce cas, la sonde dite de capture est immobilisée ou immobilisable sur un support solide par tout moyen approprié, c'est-à-dire directement ou indirectement, par exemple par covalence ou adsorption. Les supports solides appropriés sont connus de l'homme du métier et on peut citer à titre d'exemples les matériaux de synthèse ou les matériaux naturels, les latex, les particules magnétiques, les dérivés métalliques, les gels, etc. Le support solide peut être sous la forme d'une plaque de microtitration, d'une membrane comme décrit dans la demande WO-A-94/12670, d'une particule. On peut également immobiliser sur le support plusieurs sondes de capture différentes, chacune étant spécifique d'un transcrit cible. En particulier, on peut utiliser comme support une biopuce sur laquelle peuvent être immobilisées un grand nombre de sondes.

L'immobilisation des sondes sur le support est également connue de l'homme du métier et on peut citer un dépôt de sondes par transfert direct, la micro-déposition, la synthèse *in situ* et la photolithographie.

La mise en évidence, dans l'échantillon biologique, des modifications ou anomalies d'ADN au niveau du gène codant pour le marqueur tumoral d'intérêt peut être réalisée par tout procédé de détermination des altérations de l'ADN dans un échantillon, à savoir soit la détection directe de mutations, soit la mise en évidence d'altérations dans le profil de méthylation des loci d'intérêt, ou tout autre procédé de détermination d'altérations de l'ADN dans un échantillon, connu de l'homme du métier.

Les mutations peuvent inclure des substitutions ponctuelles d'un nucléotide par un autre, des délétions de un ou plusieurs nucléotides et des insertions de un ou plusieurs nucléotides. Les mutations peuvent être situées dans la partie codant du gène du marqueur tumoral d'intérêt, ou dans les parties 5' et 3' non codant comme la région promotrice ou la région terminatrice de transcription.

Les stratégies de mise en évidence de mutation s'appuient sur les techniques de la biologie moléculaire et comprennent des étapes d'extraction d'ADN, d'amplification par PCR ou autre technique d'amplification, d'hybridation et/ou de séquençage. Dans le cas du cancer colorectal, le procédé suivant a été utilisé avec succès pour réaliser la détection de mutations dans l'ADN des selles : concentration de l'ADN par précipitation, enrichissement en cible en utilisant des oligonucléotides de capture sur billes magnétiques, amplification PCR des gènes d'intérêt, séquençage en phase solide pour identifier les mutations ponctuelles⁶⁴. Les délétions ont été identifiées par rapport à la différence de taille entre le fragment de référence attendu et le fragment muté. Imperiale et al.⁶⁴ ont décrit un panel de 21 mutations situées dans les gènes K-*ras*, APC, et p53 qui permet de détecter 16/31 des cancers invasifs.

D'autres marqueurs ADN utilisés sont la délétion BAT-26, qui est un marqueur d'instabilité des microsatellites et l'ADN hautement amplifiable appelé long ADN (L-ADN), qui n'est pas un marqueur spécifique mais qui semble refléter l'apoptose désordonné des cellules tumorales exfoliées dans le lumen colique⁶⁵. Ces marqueurs ne sont satisfaisants ni par rapport à leur sensibilité, ni par rapport à leur spécificité.

Comme indiqué précédemment, les altérations de l'ADN peuvent aussi correspondre à une modification du profil de méthylation du gène correspondant au marqueur tumoral d'intérêt. La modification du profil de méthylation peut correspondre à une hypométhylation (diminution du nombre de méthylations) ou à une hyperméthylation (augmentation du nombre de méthylations). Les motifs altérés peuvent être situés dans la partie codant du gène du marqueur tumoral d'intérêt, ou dans les parties 5' et 3' non codant comme la région promotrice ou la région terminatrice de transcription.

L'analyse de la méthylation de l'ADN peut être effectuée en utilisant des techniques basées sur la PCR qualitative et/ou quantitative comme la MSP (methylation-specific PCR), le séquençage bisulfite, la digestion par une enzyme de restriction sensible aux méthylations couplée avec la PCR, COBRA (combined bisulfite restriction analysis) et Ms-SNuPE (methylation-sensitive single nucleotide primer extension). L'ensemble de ces techniques a été revu en détail et de façon comparée dans un article de méthodologie⁶⁶.

Dans la littérature plusieurs gènes hyperméthylés en cas de cancer colorectal ont été rapportés. A titre d'exemple on peut citer le gène ALX4 (Aristaless-like homeobox-4)⁵⁶, la région promotrice du gène TPEF/HHP1 (transmembrane protein containing epidermel growth factor and follistatin domain)⁶⁷ ou encore le gène Septin-9⁶⁸.

Lorsque, dans le procédé de l'invention, on détecte au moins deux marqueurs, ils peuvent être mis en évidence de façon séparée, par exemple à l'aide de kits différents, ou bien de façon simultanée en multiplex.

Lorsque, dans le procédé de l'invention, on détecte deux marqueurs de nature différente, par exemple un marqueur protéique et un marqueur ARNm, on peut utiliser deux procédés de détection différents, choisis parmi ceux décrits précédemment. On peut également les détecter simultanément, dans le même milieu de détection et dans les mêmes conditions réactionnelles, comme décrit dans la demande de brevet WO03/104490. Les étapes du procédé de détection décrit dans cette demande de brevet, qui consiste à détecter simultanément des réactions d'hybridation et immunologiques dans un échantillon susceptible de contenir des analytes cibles constitués d'au moins un acide nucléique et d'au moins un autre ligand de nature différente, consistent à :
(i) déposer une quantité connue en volume de l'échantillon dilué dans un tampon de réaction, sur une surface de capture préalablement revêtue des partenaires de capture desdits analytes cibles, lesdits partenaires de capture consistant en au moins une sonde nucléique et au moins un anti-ligand,
(ii) mettre à réagir à une température comprise entre 15°C et 60°C et
(iii) visualiser les réactions d'hybridation et immunologiques ainsi obtenues.

L'échantillon biologique peut nécessiter un traitement particulier car il peut contenir le ou les marqueurs tumoraux recherchés dans le procédé de l'invention en tant que tels, ou bien il peut contenir des cellules tumorales circulantes qui contiennent les marqueurs recherchés dans le procédé de l'invention et/ou des cellules tumorales circulantes qui sont capables de sécréter le ou les marqueurs recherchés dans le procédé de l'invention.

Ainsi, selon un mode de réalisation de l'invention, l'échantillon biologique est préalablement traité pour isoler les cellules tumorales circulantes contenues dans le dit fluide.

Par isoler les cellules tumorales circulantes, on entend obtenir une fraction cellulaire enrichie en cellules tumorales circulantes.

Le traitement de l'échantillon biologique pour isoler les cellules tumorales circulantes peut être effectué par tri cellulaire dans un cytomètre de flux, par enrichissement sur Ficoll, par enrichissement par billes magnétiques recouvertes d'anticorps spécifiques, ou par toute autre méthode d'enrichissement spécifique connue de l'homme du métier.

Dans le cas du sang à titre d'échantillon biologique, les cellules tumorales circulantes peuvent être isolées grâce à une technique de séparation cellulaire sur Ficoll associée à une déplétion des cellules sanguines utilisant des anticorps anti-CD45 couplés à des billes magnétiques (Dynal Biotech ASA, Norvège).

La détection du ou des marqueurs tumoraux recherchés dans le procédé de l'invention peut alors être effectuée directement à partir de cellules tumorales circulantes isolées de l'échantillon biologique, par exemple par marquage immunocytochimique de ces cellules avec un anticorps anti-marqueur(s) tumoral(aux) recherché(s) dans le procédé de l'invention, après avoir déposé les cellules tumorales circulantes sur une lame par cytospin. La détection du ou des marqueurs tumoraux recherchés dans le procédé de l'invention peut également être effectuée directement dans les cellules tumorales circulantes en utilisant la méthode de cytométrie de flux telle que décrite dans Métézeau et al.⁶⁹.

Dans ces conditions, lesdites cellules circulantes peuvent être traitées dans des conditions permettant le blocage du ou des marqueurs tumoraux recherchés dans le procédé de l'invention à l'intérieur desdites cellules. Un tel traitement est décrit par Mathieu et al.⁷⁰.

La détection du ou des marqueurs tumoraux recherchés dans le procédé de l'invention se fait alors après avoir rendu perméable la membrane des cellules pour faire rentrer les partenaires de liaison spécifique du ou des marqueurs recherchés dans le procédé de l'invention.

La détection directe du ou des marqueurs tumoraux utilisés dans le procédé de l'invention à partir des cellules circulantes peut également être effectuée à l'aide d'un procédé ELISPOT, par exemple à l'aide du procédé décrit dans la demande de brevet WO03/076942 déposée par la Demanderesse. Ce procédé est un procédé de détection et/ou quantification de cellules tumorales circulantes d'un échantillon biologique, lesquelles sont capables de relarguer ou sécréter *in vitro* un ou plusieurs marqueurs tumoraux, comprenant les étapes consistant à :
(i) déposer une quantité desdites cellules au fond d'une surface de culture sur laquelle est fixé au moins un partenaire de liaison spécifique dudit ou desdits marqueurs tumoraux,
(ii) cultiver lesdites cellules en conditions telles qu'elles relarguent ou sécrètent lesdits marqueurs tumoraux qui sont immunocapturés au fond de la surface de culture,
(iii) éliminer les cellules par lavage,
(iv) ajouter au moins un conjugué marqué spécifique desdits marqueurs tumoraux et
(v) visualiser le marquage ainsi obtenu.

La détection directe du ou des marqueurs tumoraux utilisés dans le procédé de l'invention dans les cellules tumorales peut encore être effectuée dans le milieu de culture desdites cellules après les avoir cultivées dans des conditions telles qu'elles sécrètent du ou des marqueurs tumoraux utilisés dans le procédé de l'invention.

Les conditions de culture pour le relarguage ou l'expression des marqueurs tumoraux sont des conditions classiques telles que 37°C sous atmosphère humide et à 5% de CO₂.

Lorsque l'échantillon biologique est un échantillon solide, la présence du ou des marqueurs tumoraux peut également être montrée *in vivo, in situ* dans les tumeurs.

Pour montrer la présence d'un marqueur tumoral au sein d'une tumeur *in vivo,* on peut utiliser toute méthode d'imagerie connue de l'homme du métier. Pour cela, on peut coupler un partenaire de liaison dudit marqueur tumoral à un traceur d'imagerie.

Par couplage des partenaires de liaison à un traceur d'imagerie, on entend la fixation d'un traceur capable d'être détecté par toute méthode d'imagerie connue de l'homme du métier, et de générer directement ou indirectement un signal détectable. Ainsi, le traceur peut être un traceur radioactif comme le technétium-99. Dans ce cas, l'organe atteint du cancer primitif ou des métastases va fixer le marqueur tumoral et son traceur. Le rayonnement émis par l'organe peut être filmé par une caméra spéciale, par exemple une gamma-caméra. L'appareil recueille les scintillations générées par la substance radioactive et permet ainsi de visualiser l'organe.

Dans un autre procédé de l'invention, le traceur peut comprendre un corps radioactif émettant des positrons (Fluor 18). Les images seront ensuite acquises par un système de Tomographie par Émission de Positrons.

Dans un autre procédé préféré de l'invention, le partenaire du ou des marqueurs tumoraux peut être couplé à des nanoparticules. A titre d'exemple, il peut s'agir de nanoparticules supramagnétiques. Par exemple des nanoparticules magnétiques anioniques pour l'application au marquage cellulaire direct et la détection *in vivo* par imagerie par résonance magnétique nucléaire. Il peut également s'agir de nanoparticules d'or.

Grâce aux procédés de l'invention permettant la détection du marqueur tumoral *in vivo,* on pourra visualiser les zones de l'organisme où il y a eu fixation du partenaire de liaison du marqueur tumoral.

Le procédé de l'invention peut être utilisé tant pour le diagnostic précoce, que pour le dépistage, le suivi thérapeutique, le pronostic et le diagnostic des rechutes dans le cadre du cancer colorectal puisque la diminution de la concentration d'Apolipoprotéine AII dans l'échantillon distant a lieu quelque soit le stade du cancer colorectal, ce qui constitue un autre objet de l'invention.

L'invention sera mieux comprise à l'aide des exemples suivants donnés à titre illustratif et non limitatif, ainsi qu'à l'aide des Figures 1 à 21 annexées, sur lesquelles :
- la figure 1 est un graphe relatif au dosage par ELISA du LEI, en ng/ml, dans le sérum de patients présentant un cancer colorectal (CCR+) et de patients sains (CCR-),
- la figure 2 est un graphe relatif au dosage par ELISA de l'Ezrine, en ng/ml, dans le sérum de patients présentant un cancer colorectal (CCR+) et de patients sains (CCR-),
- la figure 3 est un graphe relatif au dosage par ELISA de l'Aminoacylase 1, en ng/ml, dans le sérum de patients présentant un cancer colorectal (CCR+) et de patients sains (CCR-),
- la figure 4 est un graphe relatif au dosage par ELISA du L-FABP, en ng/ml, dans le sérum de patients présentant un cancer colorectal (CCR+) et de patients sains (CCR-),
- la figure 5 est un graphe relatif au dosage par ELISA du I-FABP, en pg/ml, dans le sérum de patients présentant un cancer colorectal (CCR+) et de patients sains (CCR-),
- la figure 6 est un graphe relatif au dosage par ELISA de l'Apolipoprotéine AI, en µg/ml, dans le sérum de patients présentant un cancer colorectal (CCR+) et de patients sains (CCR-), soit en microplaque ELISA (figure 6A), soit avec le kit Lincoplex (figure 6B),
- la figure 7 est un graphe relatif au dosage par le kit multiplex de Linco de l'Apolipoprotéine AII, en µg/ml, dans le sérum de patients présentant un cancer colorectal (CCR+) et de patients sains (CCR-),
- la figure 8 est un graphe relatif au dosage par ELISA de la Plastine-I, en RFV (Relative Fluorescence Value), dans le sérum de patients présentant un cancer colorectal (CCR+) et de patients sains (CCR-),
- la figure 9 est un graphe relatif au dosage par ELISA de la béta2 Microglobuline, en ng/ml, dans le sérum de patients présentant un cancer colorectal (CCR+) et de patients sains (CCR-),
- la figure 10 est un graphe relatif au dosage par ELISA d'ACE, en ng/ml, dans le sérum de patients présentant un cancer colorectal (CCR+) et de patients sains (CCR-),
- la figure 11 est un graphe relatif au dosage par ELISA du CA 19-9, en U/ml, dans le sérum de patients présentant un cancer colorectal (CCR+) et de patients sains (CCR-),
- la figure 12 est un graphe relatif au dosage par ELISA de la Testostérone, en ng/ml, dans le sérum de patients présentant un cancer colorectal (CCR+) et de patients sains (CCR-),
- la figure 13 est un graphe relatif au dosage par ELISA de la E-Cadhérine, en ng/ml, dans le sérum de patients présentant un cancer colorectal (CCR+) et de patients sains (CCR-),
- la figure 14 est un graphe relatif au dosage par ELISA de la PAP1, en ng/ml, dans le sérum de patients présentant un cancer colorectal (CCR+) et de patients sains (CCR-),
- la figure 15 est un graphe relatif au dosage par ELISA de la Galectine-3, en RFV, dans le sérum de patients présentant un cancer colorectal (CCR+) et de patients sains (CCR-),
- la figure 16 est un graphe relatif au dosage par ELISA de la LDH, en ng/ml, dans le sérum de patients présentant un cancer colorectal (CCR+) et de patients sains (CCR-),
- la figure 17 est un graphe relatif au dosage par ELISA du Protéasome 20S, en ng/ml, dans le sérum de patients présentant un cancer colorectal (CCR+) et de patients sains (CCR-),
- la figure 18 est un graphe relatif au dosage par ELISA d'Aminoacylase 1, en ng/ml, dans les selles de patients présentant un cancer colorectal (CCR+) et de patients sains (CCR-),
- la figure 19 est un graphe relatif au dosage par ELISA de Galectine-3, en RFV, dans les selles de patients présentant un cancer colorectal (CCR+) et de patients sains (CCR-),
- la figure 20 est un graphe relatif au dosage par ELISA de Protéasome 20S, en RFV, dans les selles de patients présentant un cancer colorectal (CCR+) et de patients sains (CCR-), et
- la figure 21 est une représentation graphique d'un dosage ELISPOT du LEI, de l'Ezrine et de la Galectine-3, en nombre de spots par 10⁶ cellules cancéreuses des lignées Caco-2, HT-29 et HT29-B6.

### Exemple 1 : Clonage des gènes codant pour les marqueurs tumoraux et expression desprotéines recombinantes

### 1. Amplification de l'ADNc et clonage

La lignée de cancer colorectal Caco-2 est cultivée dans le milieu DMEM contenant 2 mM de L-Glutamine, sans SVF (sérum de veau foetal) (tous Gibco).

Pour le clonage des gènes LEI, L-FABP et Gal-3, les ARN messagers ont été extraits à partir d'un culot de 10⁸ cellules Caco-2 en utilisant le kit FastTrack 2.0 de Invitrogen (Cat. No. 45-0019), en suivant le protocole fourni par le fabricant. Les étapes de transcription inverse et de PCR sont réalisées en une seule fois à partir de 450 ng d'ARNm Caco-2, avec le kit Superscript III One Step RT-PCR System (Invitrogen Cat. No. 12574-018) utilisant l'enzyme Platinum Taq DNA polymérase en suivant le protocole fourni par le fabricant. Les amorces de PCR utilisées pour l'amplification des gènes sont données dans le Tableau 1.

**Tableau 1.**

| Gènes et Amorces | Oligonucléotides |
|---|---|
| *LEI* | |
| OL215 (SEQ ID°1) | 5'-ATGGAGCAGCTGAGCTCAGCAAAC-3' |
| OL216 (SEQ ID°2) | 5'-CTAAGGGGAAGAAAATCTCCCCAA-3' |
| *L-FABP* | |
| Forward (SEQ ID°3) | |
| Reverse (SEQ ID°4) | |
| *Gal-3* | |
| OL217 (SEQ ID°5) | 5'-ATGGCAGACAATTTTTCGCTCC-3' |
| OL218 (SEQ ID°6) | 5'-TTATATCATGGTATATGAAGCACTGG-3' |

Les fragments d'ADN obtenus ont été clonés dans le vecteur pCR2.1 TOPO (LEI et Gal-3) avec le TA Cloning kit (Invitrogen Cat. No. K4520-01) ou le vecteur pCMV6-XL4 d'Origene (L-FABP) après digestion par Bsm BI et Xba I. Les plasmides ont été séquencés afin de vérifier que l'ADNc est bien conforme à la séquence attendue.

Pour le clonage du gène codant pour l'Aminoacylase 1, les ARN totaux ont été extraits à partir d'un culot de 10⁸ cellules Caco-2 en utilisant le kit RNA Easy Mini de Qiagen, en suivant le protocole fourni par le fabricant. La transcription inverse est réalisée à partir de 10 ng de ARN Caco-2, avec l'enzyme Superscript II (Invitrogen) en suivant le protocole fourni par le fabricant. L'amorce de transcription inverse est un oligo(dT).

L'ADNc issu de cette réaction a été utilisé comme matrice dans une réaction de PCR utilisant le kit AccuPrime Pfx (Invitrogen Cat. No. 12344-024) en suivant le protocole fourni par le fabricant. Les amorces de PCR sont : ACY-1 Fwd2 (SEQ ID°7 : 5'-GCGAATTCTTTAAGAAGGAGATATACATATGACGAGCAAAGGTCCGGAA GAGGAGCACCCATCG-3') et ACY-1 Rev (SEQ ID°8 : 5'-GCAAGCTTCAGCTGTCACTGGGCAGGGC-3')

Dans ces conditions, il a été possible d'amplifier un fragment de 1,3 kb qui a été cloné dans un vecteur de clonage de type Zero Blunt TOPO PCR Cloning kit (Invitrogen Cat. No. K2820-20). Ce plasmide a été séquencé afin de vérifier que l'ADNc est bien conforme à la séquence attendue.

Le fragment d'ADN suivant (SEQ ID°9) contenant le cadre de lecture ouvert *I-FABP* a été obtenu par synthèse chimique, effectué par la société Geneart. SEQ ID°9 :

### 2. Construction des vecteurs d'expression

Les gènes codant pour le LEI et la Galectine-3 ont été sous-clonés dans le vecteur d'expression procaryote pMR78⁷¹ et le gène de L-FABP dans le vecteur pET3d (New England Biolabs). Les sites de restriction nécessaire au clonage ont été introduits par PCR en utilisant comme matrice les plasmides pCR2.1 TOPO-LEI, pCR2.1 TOPO-Gal-3 et pCMV6-LFABP. L'enzyme de PCR est la Pfu DNA polymérase de Promega, la réaction de PCR a été réalisée selon les instructions du fabricant avec les amorces données dans le Tableau 2.

**Tableau 2**

| Gènes et Amorces | Oligonucléotides |
|---|---|
| *LEI* | |
| OL228 (SEQ ID°10) | 5'-ATGGGAATTCAGGAGCAGCTGAGCTCAGCAA-3' |
| OL229 (SEQ ID°11) | 5'-CGATAAGCTTAAGGGGAAGAAAATCTCCCC-3' |
| *L-FABP* | |
| Forward (SEQ ID°12) | |
| Reverse (SEQ ID°13) | |
| *Gal-3* | |
| OL230 (SEQ ID°14) | 5'-ATGGGAATTCAGGCAGACAATTTTTCGCTCC-3' |
| OL231 (SEQ ID°15) | |

Les produits de PCR contenant les cadres de lecture ouverts codant pour le LEI ou la Galectine-3 ont été digérés par les enzymes de restriction Eco RI et Hind III. Les fragments ont été introduits dans le vecteur pMR78 restreint par les mêmes enzymes (plasmides pMR-LEI et pMR-Gal-3). Le vecteur pMR78 contient une séquence 6-histidine en phase avec la protéine à exprimer qui permet la purification par chromatographie d'affinité métal-chélate. Le produit de PCR L-FABP a été cloné dans le vecteur pET3d, au niveau des sites de restriction Nco I et Bam HI.

Pour l'Aminoacylase 1, le vecteur de clonage TOPO a été directement digéré par les enzymes de restriction Eco RI et Hind III afin de générer un fragment de 1,3 kb contenant le cadre de lecture ouvert *acyl*, qui a été introduit dans le vecteur pStabyl (Eurogentec). Le plasmide recombinant est appelé pStabyl-ACY.

Pour la I-FABP, le vecteur de clonage fourni par Geneart a été digéré par les enzymes de restriction Eco RI et Sal I afin de générer un fragment d'environ 400 pb contenant la séquence codante qui a été introduite dans le vecteur pMRCH79 (dérivé du vecteur pMR78, bioMérieux). Le plasmide recombinant est appelé pMRCH-*IFABP*.

Les plasmides pGEX-Ezrine et pGEX-Plastine-I, permettant d'exprimer respectivement l'Ezrine et la Plastine-I en fusion avec la GST (Glutathione S-transférase), ont été fournis par l'Institut Curie.

### 3. Expression et purification des protéines recombinantes

Les plasmides d'expression permettant de produire les marqueurs tumoraux recombinants sont introduits dans des bactéries E. coli BL21 et dérivées (Stratagene). Les cultures sont réalisées à température ambiante sous agitation. Les conditions de culture précises pour chaque protéine sont récapitulées dans le Tableau 3. L'IPTG est l'isopropyl beta-D-1-thiogalactosidase.

Les culots bactériens sont repris en tampon PBS 2X (phosphate buffered saline) et passé dans un désintégrateur de cellules à 1,5 kbar (Constant System). Les lysats sont centrifugés à 3000 *g* pendant 30 min à 4°C. Le surnageant contient les protéines solubles. Le culot contient les corps d'inclusion. Le tampon de solubilisation des corps d'inclusion dépend de la protéine.

Pour le LEI, la purification se fait à partir de la fraction soluble, sur une colonne contenant 5 mL de résine Ni-NTA-Sepharose (Qiagen) et la protéine est éluée avec du PBS 2X contenant de l'imidazole 450 mM, pH 7,5.

Pour la Galectine-3, les corps d'inclusions sont solubilisés en PBS 2X, urée 1M, passés sur 5 mL de résine Ni-NTA-Sepharose (Qiagen) et la protéine Gal-3 est éluée avec du PBS 2X contenant de l'imidazole 450 mM, urée 1M pH 7,5.

Pour la L-FABP, la purification se fait à partir de la fraction soluble, en utilisant le kit Ni-IDA de Macherey-Nagel.

**Tableau 3**

| | Souche | Volume Culture | Induction IPTG | Purification |
|---|---|---|---|---|
| LEI | BL21 | 250 mL | 0,1 mM | Ni-NTA |
| Gal-3 | BL21-Codon | 400 mL | 0,5 mM | Ni-NTA |
| | plus (DE3)-RIPL | | | |
| L-FABP | BL21 | 500 mL | 0,1 mM | Ni-IDA |
| GST-Ezrin | BL21 | 250 mL | 0,1 mM | GST |
| ACY-1 | BL21-Codon | 500 mL | 0,1 mM | Autre |
| | plus (DE3)-RIPL | | | |

Pour la GST-Ezrine, la purification se fait à partir des corps d'inclusions solubilisés dans le tampon Tris 100 mM, urée 8M, DTT 10 mM par chromatographie d'affinité GST. On utilise une colonne contenant 5 mL de gel Glutathione Sepharose 4 fast flow (Amersham). Le tampon d'équilibration et de lavage est du PBS 2x, Tween 20 0,05%. Le tampon d'élution est du Tris-HCl 50 mM, glutathione réduite 20 mM, pH 8.

Pour l'Aminoacylase 1, la fraction soluble de la culture est passée sur une colonne Amersham HiTrap Q FF et la protéine ACY-1 a été éluée avec 0.3M NaCl à pH 7,5. Comme plusieurs autres protéines ont été co-éluées dans ces conditions, la purification a été poursuivie sur une colonne à interaction hydrophobe (HIC Phenyl HP, Amersham). La protéine ACY-1 a été éluée par 0,5M NaCl à pH 7.

La protéine recombinante GST-Plastine-I a été fournie par l'Institut Curie sous forme purifiée.

La protéine recombinante Calréticuline a été produite par la société Proteus Services for Industry (Dijon, France). La séquence codant pour la Calréticuline a été obtenue par synthèse chimique.

### Exemple 2: Obtention d'anticorps monoclonaux dirigés contre les marqueurs tumoraux

### 1. Modèle animal

Les expériences d'immunisation ont été réalisées chez des souris BALB/c (H-2^{d}) femelles âgées de 6 à 8 semaines au moment de la première immunisation.

### 2. Immunogènes et immunisations

Afin d'augmenter les réponses immunes obtenues chez les souris et pouvoir générer des anticorps monoclonaux, les marqueurs tumoraux ont été produits sous forme de protéines recombinantes produites selon les modes opératoires décrits dans l'exemple 1. La protéine LDH a été obtenue auprès de la société SciPac (Cat. No. 103-133). Ces protéines ont été mélangées volume pour volume avec l'adjuvant de Freund (Sigma), préparé sous forme d'émulsion eau-dans-huile et dont il est connu qu'il présente un bon pouvoir immunogène. Pour chaque marqueur tumoral, 3 souris ont été immunisées. Les souris ont reçu 3 doses successives de 10 µg des immunogènes à 0, 2 et 4 semaines. Toutes les injections ont été réalisées par voie sous-cutanée. La première injection est faite en mélange avec l'adjuvant de Freund complet, les deux suivantes se font en mélange avec l'adjuvant de Freund incomplet. Entre J50 et J70 après la première injection, les réponses humorales ont été restimulées avec une injection intraveineuse de 100 µg de la protéine recombinante.

### 3. Suivi de l'apparition de la réponse humorale

Afin de suivre l'apparition des anticorps, on effectue régulièrement sur les souris des prélèvements de sang. La présence des anticorps anti-marqueur tumoral est testée en utilisant un ELISA. La protéine d'intérêt est utilisée en capture (1 µg/puits), après saturation on fait réagir avec l'antigène différentes dilutions des sérums à tester (incubation à 37°C, pendant 1h). Les anticorps spécifiques présents dans le sérum sont révélés par un anticorps de chèvre anti-IgG de souris AffiniPure conjugué à la phosphatase alcaline (H+L, Jackson Immunoresearch, Cat no. 115-055-146), qui se lie aux anticorps recherchés (0,1 µg/puits).

### 4. Obtention d'anticorps monoclonaux

Trois jours après la dernière injection, pour chaque marqueur tumoral, une des souris immunisée a été sacrifiée ; le sang et la rate ont été prélevés. Les splénocytes obtenues à partir de la rate ont été mises en culture avec les cellules de myélome Sp2/0-Agl4 pour qu'elles fusionnent et s'immortalisent, selon le protocole décrit par Köhler et Milstein^{72,73}. Après une période d'incubation de 12-14 jours les surnageants des hybridomes obtenus ont été criblés pour déterminer la présence d'anticorps anti-marqueur tumoral en utilisant le test ELISA décrit dans le point 3 de cet exemple. Lorsque des protéines de fusion GST ont été utilisées comme immunogène, les clones dirigés contre la GST sont éliminés en effectuant un criblage ELISA avec en capture la GST non couplée. Les colonies d'hybridome positives ont été sous-clonées deux fois selon la technique de la dilution limite, bien connue par l'homme du métier.

### 5. Caractérisation des anticorps monoclonaux par immunoblot

La liste des anticorps monoclonaux obtenus contre les différents marqueurs tumoraux est présentée dans le Tableau 4. Ces anticorps monoclonaux ont été analysés par la technique du Western blot.

**Tableau 4**

| Marqueurs Tumoraux | Nom des Anticorps Monoclonaux |
|---|---|
| Leucocyte Elastase Inhibitor (LEI) | 21B10A5 et 10E1H1 |
| Ezrine | 4A7A6C1 et 4A9H5 |
| Aminoacylase 1 | 2A7F6 et 11H7D9 |
| Plastine-I | 3D11D10, 8C8C5, 3A3H2, 8G2D2 |
| Calréticuline | 5C10H10 et 11B6D11 |
| L-lactate déshydrogénase chaîne B (LDH) | 3F11E11 et 12F10G8 |
| Galectine-3 | 12F8A12 et 14A5G1 |

### 5.1. Méthodologie

Les extraits de culture cellulaires de lignées Caco-2 et HT-29 sont préparés en lysant directement le culot cellulaire par 600 µl d'une solution en eau d'urée 8,3M, thiourée 2M, sulfonate de 3-[(3-cholamidopropyl)-dimethylammonio]-1-propane (CHAPS) 4%, DTT 100 mM, Servalyte 4-9 (Serva, Heidelberg, Allemagne) 2%, Orange G 0.1g/l, puis traités selon le protocole de préparation d'échantillon des gels NuPAGE Novex (Invitrogen). Pour obtenir les extraits de tissu, les biopsies tumeur et muqueuse des patients GHBD001, GHBD004 et CLSP109 ont été dissociées au scalpel, puis ont subi 10 cycles d'extraction dans le système Medimachine (Becton Dickinson) en utilisant des Medicons 50 µm avec 1 ml de tampon PBS, 2,5 mM EDTA, inhibiteurs des protéases (pastilles Roche). Ces 10 ml de suspension cellulaire sont poolés, complétés à 25 ml puis centrifugés 15 min à 600*g*. Le surnageant correspond à l'extrait de tissu qui est traité selon le protocole de préparation d'échantillon des gels NuPAGE Novex. On utilise des échantillons réduits, à une concentration finale en protéine totale de 0,4 mg/ml. Le volume de dépôt est de 20 µl par puits, sur un gel NuPAGE Novex Bis-Tris 4-12%, tampon de migration MOPS. Après migration (sous 200V, pendant 1 heure) et transfert sur membrane de PVDF (sous 400 mA, pendant 45 min), la qualité du transfert est appréciée par une coloration à l'amidoblack.

Les membranes sont saturées par 5% de lait écrémé (Régilait) dans une solution de TNT (Tris 15 mM, NaCl 0,14M, Tween 20 0,5% pH8) à température ambiante pendant 1 heure. Après saturation, les membranes sont incubées pendant 1 heure avec les différents anticorps à tester dilués à 10 µg/ml dans la solution de saturation. Après rinçages au TNT, les membranes sont incubées 1 heure à température ambiante avec un conjugué anti-souris-peroxydase de raifort dilué au 1 : 5000, (Cat No. 115-035-062, Jackson Immunoresearch) dans la solution de saturation. Après rinçage, la révélation est réalisée avec le kit Substrat Supersignal West Dura Extended (Cat No. 34076, Pierce) suivant les données d'utilisation recommandées.

Le signal de chemiluminescence sur les membranes a été mesuré avec le système d'imagerie VersaDoc de Biorad. A partir de l'image du Western blot, les volumes des bandes qui correspondent aux différents marqueurs tumoraux ont été évalués avec le logiciel QuantityOne (Bio-Rad). Le volume correspond à l'intensité du signal de chemiluminescence multipliée par la surface de la bande.

### 5.2. Résultats

Les résultats de Western blot récapitulés dans le Tableau 5 qui donne le volume des bandes correspondant au marqueur tumoral d'intérêt sur les analyses par Western blot, en fonction des différents échantillons testés. Ces résultats montrent que les marqueurs tumoraux testés sont bien exprimés par les lignées de cancer du côlon Caco-2 et HT-29, ainsi que dans les tissus, comme montré avec les extraits de tumeur et muqueuse, obtenus à partir des patients. L'intensité du signal obtenu avec un anticorps sur un échantillon peut être comparée aux signaux obtenus avec les autres échantillons et le même anticorps. La technique utilisée permet de confirmer la présence ou l'absence du marqueur dans le tissu (échantillon non distant) et la spécificité des anticorps vis-à-vis des marqueurs. Cette technique n'a pas été mise en oeuvre dans cet exemple dans les échantillons distants car elle ne permettrait pas de conclure à la présence ou non du marqueur tumoral dans les échantillons distants, ni de déterminer si sa concentration sera augmentée ou diminuée dans ceux-ci. De plus, le schéma expérimental utilisé ne permet pas de comparer la réactivité d'un anticorps à l'autre.

**Tableau 5**

| Marqueur tumoral et Anticorps | Caco-2 | HT-29 | Tissu tumeur GHBD001 | Tissu muqueuse GHBD004 | Tissu tumeur GHBD004 | Tissu muqueuse CLSP109 | Tissu tumeur CLSP109 |
|---|---|---|---|---|---|---|---|
| *LEI* | | | | | | | |
| 21B10A5 | 8365 | 7678 | NT | 60200 | 36506 | NT | NT |
| 10E1H1 | 0 | 0 | NT | 13357 | 6893 | NT | NT |
| *Ezrine* | | | | | | | |
| 4A9H5 | 7066 | 4742 | NT | NT | NT | 1588 | 2446 |
| 4A7A6C1 | 123436 | 116448 | 42480 | 15303 | 67439 | NT | NT |
| *Aminoacylase 1* | | | | | | | |
| 2A7F6 | 10687 | 4787 | NT | NT | NT | 4477 | 7238 |
| 11H7D9 | 217664 | 232005 | 36093 | 10513 | 30233 | NT | NT |
| *Plastine-I* | | | | | | | |
| 3D11D10 | 136725 | NT | NT | NT | NT | 275477 | 246564 |
| 8C8C5 | 557 | 1110 | 4364 | 77 | 0 | NT | NT |
| *Calréticuline* | | | | | | | |
| 5C10H10 | 2842 | 3040 | NT | NT | NT | 2503 | 3294 |
| 11B6D11 | 3261 | 2937 | NT | NT | NT | 2070 | 2764 |
| *LDH* | | | | | | | |
| 3F11E11 | 45391 | NT | NT | NT | NT | 30411 | 13942 |
| 12F10G8 | 122907 | 154593 | 11841 | 15811 | 53285 | NT | NT |
| *Galectine-3* | | | | | | | |
| 12F8A12 | 245712 | 65790 | 18262 | 12961 | 7307 | NT | NT |
| 14A5G1 | 254531 | 120010 | 79833 | 98361 | 45872 | NT | NT |

| | | | | | | | |
|---|---|---|---|---|---|---|---|
| NT: non testé. | | | | | | | |

### 5.3. Anticorps monoclonaux dirigés contre la Plastine-I

Chez le patient GHBD004, l'anticorps 8C8C5 n'allume pas ou que très faiblement la bande qui correspond à la Plastine-I. La présence de Plastine-I dans ces échantillons peut être mise en évidence en utilisant par exemple l'anticorps 8G2D2 qui présente une meilleure affinité pour la Plastine-I en blot.

Comme la Plastine-I est membre d'une famille de protéines comprenant 2 autres isoformes (Plastines L et T) avec lesquels elle présente plus de 70% d'homologie, nous avons testé l'ensemble des clones d'anticorps monoclonaux obtenus pour leur réactivité vis-à-vis des protéines GST-Plastine-L et GST-Plastine-T (fournies par l'Institut Curie). A l'issu de ce criblage, nous avons sélectionné les clones 3D11D10, 8C8C5, 3A3H2 et 8G2D2 qui ne présentent pas de réactivité croisée avec les autres membres de la famille. Ces anticorps sont bien spécifiques de l'isoforme Plastine-I.

### Exemple 3 : Dosages sériques des marqueurs tumoraux

### 1. Patients et prélèvements

La collecte des échantillons de sang se fait au niveau d'un réseau de 8 centres cliniques répartis dans toute la France, dans le cadre de 2 protocoles loi Huriet.

Pour l'obtention de sérum, le prélèvement sanguin se fait sur tube sec. Pour l'obtention du plasma, le prélèvement sanguin se fait sur tube EDTA. Après coagulation, le tube est centrifugé 10 min à 1000 *g,* le sérum est prélevé, aliquoté et conservé à -80°C. Le tube de plasma est directement centrifugé 10 min à 1000 *g,* le plasma est prélevé, aliquoté et conservé à -80°C. Les échantillons sont parfaitement documentés pour l'histoire clinique des patients.

### 2. Dosage sérique du marqueur tumoral LEI

La protéine LEI a été dosée à l'aide des anticorps décrit dans l'exemple 2 et d'un test ELISA utilisant l'automate Vidas® (bioMérieux). Pour ce faire, le test ELISA a été construit en utilisant les réactifs du kit Vidas® HBs Ag Ultra (bioMérieux, Cat. No. 30315). Les réactifs ont été utilisés tels que décrits dans la notice correspondante (réf. 11728 D - FR - 2005/05), avec les modifications suivantes :
1. Les cônes ont été sensibilisés avec l'anticorps de capture 10E1H1 à une concentration de 10 µg/ml.
2. Le contenu du deuxième puits de la cartouche HBs Ag Ultra a été remplacé par 300 µl d'anticorps de révélation 21B10A5, couplé à la biotine, dilué à 1 µg/ml dans le tampon du deuxième puits du kit Vidas® HBs Ag Ultra (tampon avec sérum de chèvre et azoture de sodium à 1 g/l).
3. Les échantillons de sérum, de plasma ou de selle (50µl) ont été dilués directement dans le deuxième puits de la cartouche HBs Ag Ultra, pur ou après une dilution préalable au 1/20 dans le tampon du deuxième puits du kit Vidas@ HBs Ag Ultra (tampon avec sérum de chèvre et azoture de sodium à 1 g/l).
4. La réaction ELISA a été réalisée à l'aide de l'automate Vidas® et du protocole du kit HBs Ag Ultra.
5. Les résultats ont été obtenus sous forme de valeurs brutes après soustraction du bruit de fond (lecture du substrat avant réaction).

Une courbe étalon a été établie en dosant une gamme de concentrations du marqueur tumoral sous forme de protéine recombinante. La courbe étalon a été tracée en reportant en abscisse la concentration du marqueur tumoral et en ordonnée le signal lu par Vidas® (RFV ou Relative Fluorescence Value). La concentration de marqueur tumoral présente dans le fluide corporel à doser (sang, sérum, plasma, selle) a été calculée en reportant la concentration correspondant au signal RFV lu par Vidas@.

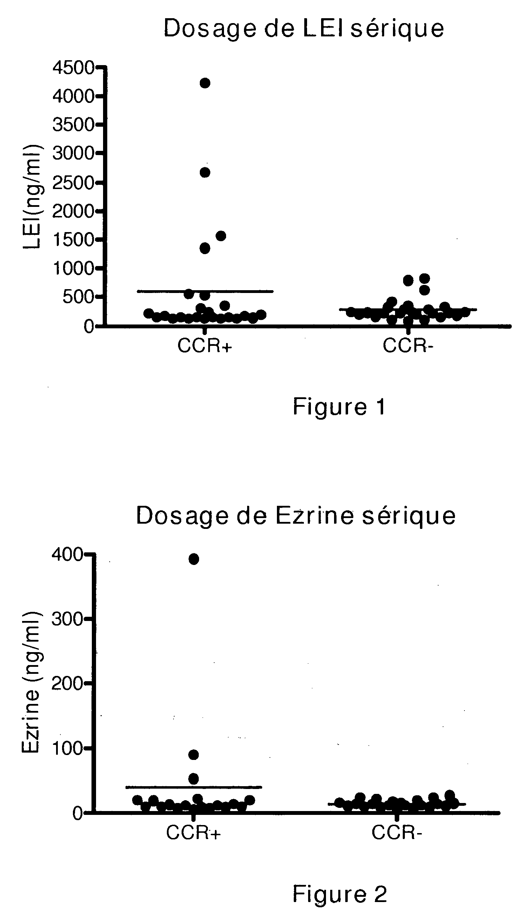

Les doses obtenues pour les patients analysés sont reportées sur la Figure 1. On peut noter sur cette figure que 3 sérums de patients ayant un cancer colorectal de stade IV et 1 sérum de patient ayant un cancer colorectal de stade III montrent une nette augmentation de leur dose de LEI sérique.

### 3. Dosage sérique du marqueur tumoral Ezrine

La protéine Ezrine a été dosée à l'aide des anticorps décrit dans l'exemple 2 et d'un test ELISA utilisant l'automate Vidas® (bioMérieux). Pour ce faire, le test ELISA a été construit en utilisant les réactifs du kit Vidas@ HBs Ag Ultra (bioMérieux, Cat. No. 30315). Les réactifs ont été utilisés tels que décrits dans la notice correspondante (réf. 11728 D - FR - 2005/05), avec les modifications suivantes :
1. Les cônes ont été sensibilisés avec l'anticorps de capture 4A9H5 à une concentration de 30 µg/ml.
2. Le contenu du deuxième puits de la cartouche HBs Ag Ultra a été remplacé par 300 µl d'anticorps de révélation 4A7A6C1, couplé à la biotine, dilué à 1 µg/ml dans le tampon du deuxième puits du kit Vidas@ HBs Ag Ultra (tampon avec sérum de chèvre et azoture de sodium à 1 g/l).
3. Les échantillons de sérum, de plasma ou de selle (50µl) ont été dilués directement dans le deuxième puits de la cartouche HBs Ag Ultra.
4. La réaction ELISA a été réalisée à l'aide de l'automate Vidas® et du protocole HBS AG ULTRA dont l'étape d'incubation de l'échantillon avec les anticorps de capture et de révélation avait été porté à 100 cycles.
5. Les résultats ont été obtenus sous forme de valeurs brutes après soustraction du bruit de fond (lecture du substrat avant réaction).

La concentration du marqueur tumoral présente dans le fluide corporel à doser (sang, sérum, plasma, selle) a été calculée selon le mode opératoire décrit dans le paragraphe 2 concernant le dosage du LEI.

Les doses obtenues pour les patients analysés sont reportées sur la Figure 2. On peut noter sur cette figure que 3 sérums de patients ayant un cancer colorectal de stade IV montrent une nette augmentation de leur dose d'Ezrine sérique.

### 4. Dosage sérique du marqueur tumoral Aminoacylase 1

La protéine Aminoacylase 1 a été dosée à l'aide des anticorps décrit dans l'exemple 2 et d'un test ELISA utilisant l'automate Vidas® (bioMérieux). Pour ce faire, le test ELISA a été construit en utilisant les réactifs du kit Vidas® HBs Ag Ultra (bioMérieux, Cat. No. 30315). Les réactifs ont été utilisés tels que décrits dans la notice correspondante (réf. 11728 D - FR - 2005/05), avec les modifications suivantes :
1. Les cônes ont été sensibilisés avec l'anticorps de capture 2A7F6 à une concentration de 20 µg/ml.
2. Le contenu du deuxième puits de la cartouche HBs Ag Ultra a été remplacé par 300 µl d'anticorps de révélation 11H7D9, couplé à la biotine, dilué à 1 µg/ml dans le tampon du deuxième puits du kit Vidas® HBs Ag Ultra (tampon avec sérum de chèvre et azoture de sodium à 1 g/l).
3. Les échantillons de sérum, de plasma ou de selle (100 µl) ont été dilués directement dans le deuxième puits de la cartouche HBS Ag Ultra.
4. La réaction ELISA a été réalisée à l'aide de l'automate Vidas® et du protocole HBS AG ULTRA dont l'étape d'incubation de l'échantillon avec les anticorps de capture et de révélation avait été porté à 100 cycles.
5. Les résultats ont été obtenus sous forme de valeurs brutes après soustraction du bruit de fond (lecture du substrat avant réaction).

La concentration du marqueur tumoral présente dans le fluide corporel à doser (sang, sérum, plasma, selle) a été calculée selon le mode opératoire décrit dans le paragraphe 2 concernant le dosage du LEI.

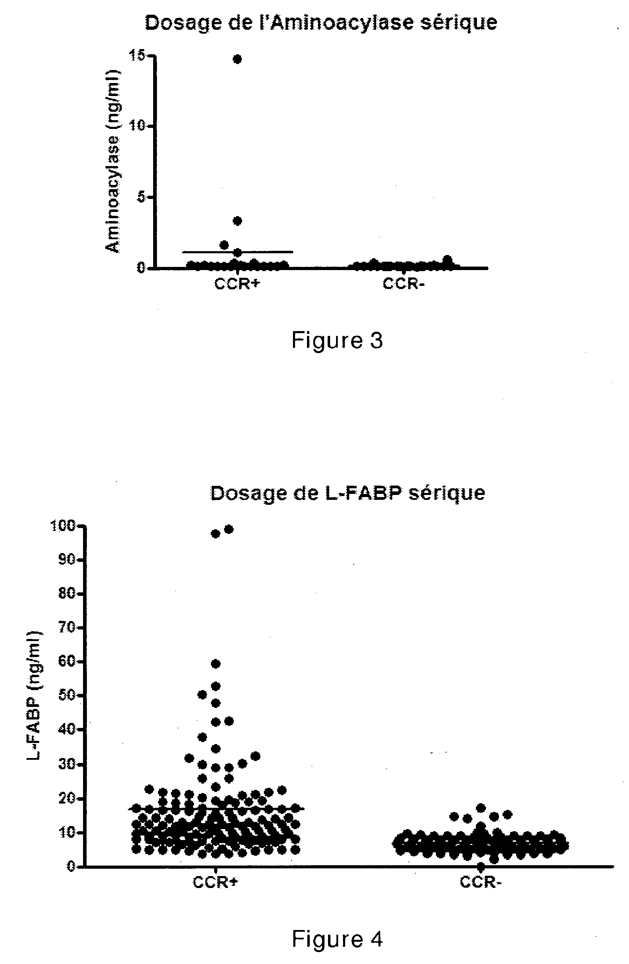

Les doses obtenues pour les patients analysés sont reportées sur la Figure 3. On peut noter sur cette figure que 1 sérum de patients ayant un cancer colorectal de stade II, 1 sérum de patients ayant un cancer colorectal de stade III et 2 sérums de patients ayant un cancer colorectal de stade IV montrent une nette augmentation de leur dose d'Aminoacylase 1 sérique.

### 5. Dosage sérique du marqueur tumoral L-FABP

Nous avons utilisé un kit ELISA commercialisé par la société Hycult biotechnology pour doser la protéine L-FABP humaine (Cat. No. HK404). Ce kit permet de quantifier la protéine L-FABP dans les surnageants de culture cellulaire, dans le sérum, le plasma ou l'urine, afin de déterminer la présence de lésions au niveau hépatique. Nous avons suivi le mode opératoire préconisé par le fabricant avec 2 modifications : les incubations ont été effectuées à 37°C et non à température ambiante, les sérums ont été dilués au 1/10e avant le dosage. Le dosage de la protéine L-FABP peut être effectué par des techniques alternatives, bien connues des hommes du métier.

La Figure 4 présente les résultats de ce dosage. Dans le panel de sérum que nous avons testé, 41 patients sur 141 ayant un cancer colorectal ont une concentration sérique de L-FABP supérieure à 17 ng/ml, alors que dans le groupe témoin, aucun sujet ne dépasse cette valeur. Parmi ces 41 patients, on retrouve 8 patients ayant un cancer colorectal de stade I, 8 ayant un cancer colorectal de stade II, 13 ayant un cancer colorectal de stade III et 12 ayant un cancer colorectal de stade IV. La concentration moyenne de L-FABP sérique observée pour 141 patients avec un cancer colorectal est de 16,6 ± 1,3 ng/ml. La valeur moyenne est de 6,6 ± 0,2 ng/ml pour 112 individus sains (témoins négatifs). Cette différence est statistiquement significative (*P*<0,0001, *t*-test unilatéral avec correction de Welch pour variances inégales).

### 6. Dosage du marqueur tumoral I-FABP

Nous avons utilisé un kit ELISA commercialisé par la société Hycult biotechnology pour doser la protéine I-FABP humaine (Cat. No. HK406). Ce kit permet de quantifier la protéine I-FABP dans les surnageants de culture cellulaire, dans le sérum, le plasma ou l'urine, afin de déterminer la présence de lésions ischémiques au niveau de l'intestin grêle. Nous avons suivi le mode opératoire préconisé par le fabricant. Le dosage de la protéine I-FABP peut être effectué par des techniques alternatives, bien connues des hommes du métier.

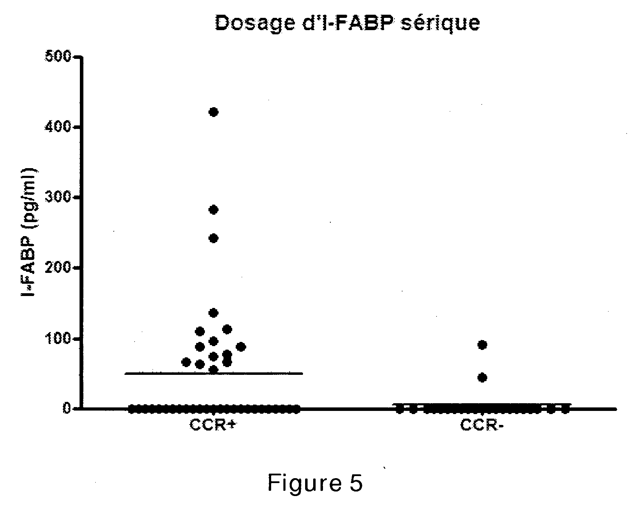

La figure 5 présente les résultats de ce dosage. Dans le panel de sérum que nous avons testé, 15 patients sur 40 ayant un cancer colorectal ont une concentration sérique d'I-FABP supérieure à 40 pg/ml, alors que dans le groupe témoin uniquement 2 sujets sur 24 dépassent cette valeur. Plus nettement, 3 sérums de patients ayant un cancer colorectal de stade I, 2 sérums de patients ayant un cancer colorectal de stade III et 1 sérum de patient ayant un cancer colorectal de stade IV ont une concentration sérique d'I-FABP supérieure à 100 pg/ml. Aucune concentration supérieure à cette valeur n'a été trouvée dans le groupe témoin CCR-.

### 7. Dosage sérique du marqueur tumoral Apolipoprotéine AI

Le dosage de l'Apolipoprotéine AI sérique a été réalisé par deux techniques différentes d'immunoessai. Dans un premier temps, nous avons utilisé un ELISA sandwich en microplaque. Les plaques 96 puits ont été coatées avec l'anticorps monoclonal anti-Apo AI Clone 1404 (Biodesign Cat. No. H45404) à 1µg par puits. Après 3 lavages PBS-Tween 20 0,05% (PBS-T), les plaques sont saturées par 10% de lait dans du PBS-T pendant 1h à 37°C. On lave encore 3 fois en PBS-T, on dépose sur les plaques 100 µl des dilutions de la gamme étalon ou 100 µl de la dilution au 1/100 000 des échantillons de sérum à tester, et on incube 2h à 37°C. La gamme étalon est réalisée en diluant la protéine Apo AI (Biodesign Cat. No. A50620H) dans du PBS-T, BSA 1% (1,6 à 100 ng/ml). Après 3 lavages PBS-T, l'anticorps de détection polyclonal couplé à la peroxydase de raifort (Biodesign Cat. No. K45452P) est rajouté à 0,1 µg par puits et on incube 2h à 37°C. On effectue encore 3 lavages PBS-T, avant de rajouter le substrat OPT EIA (BD), 100 µl/puits. Au bout de 20 min, lorsque le développement de la coloration a lieu, on stoppe la réaction par de l'acide sulfurique 2N et on mesure-l'absorbance à 450 nm.

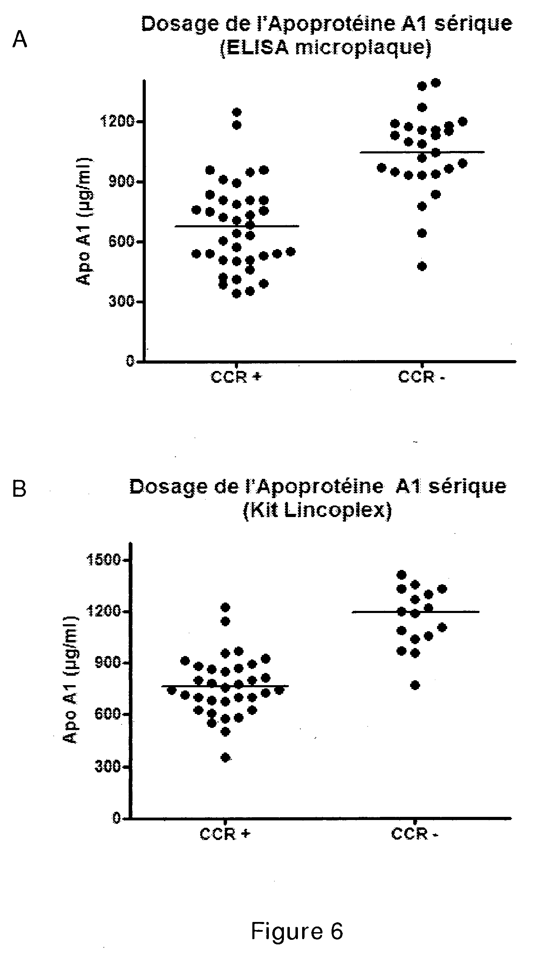

La Figure 6A présente les résultats de ce dosage. Nous avons mis en évidence une diminution de la concentration sérique d'Apo AI chez les sujets ayant un cancer colorectal. La concentration moyenne chez 38 sujets ayant un CCR de stade I à IV est de 675 ± 36 µg/ml alors qu'elle est bien plus élevée chez 27 sujets sains (témoins) : 1040 ± 39 µg/ml. Cette différence est statistiquement très significative (*P*<0,0001, *t-*test unilatéral). A titre de comparaison, chez 13 sujets ayant un cancer du foie, la concentration sérique moyenne d'Apo AI est de 1175 ± 87 µg/ml avec la technique ELISA sandwich utilisée. La diminution de la concentration sérique met en évidence que Apo AI est donc un marqueur spécifique du cancer colorectal, cette diminution pouvant être mis en évidence par un dosage immunoessai.

La deuxième technique de dosage qui a été utilisée est un dosage multiplex commercialisé par la société Linco qui permet de doser plusieurs Apolipoprotéines dont AI et AII simultanément, dans le même échantillon (Cat. No. APO-62K). Le mode opératoire préconisé par le fabricant a été appliqué.

La Figure 6B présente les résultats de ce dosage. On confirme par cette deuxième technique la diminution de la concentration sérique d'Apo AI chez les patients ayant un CCR. La concentration moyenne d'Apo AI chez 34 sujets ayant un CCR de stade I à IV est de 768 ± 30 µg/ml alors qu'elle est bien plus élevée chez 17 sujets sains (témoins): 1194 ± 51 µg/ml. Cette différence est statistiquement très significative (*P*<0,0001, *t*-test unilatéral).

### 8. Dosage sérique du marqueur tumoral Apolipoprotéine AII

Le dosage de l'Apolipoprotéine AII sérique a été réalisé avec le kit multiplex de Linco et les résultats sont donnés dans le Tableau 6.

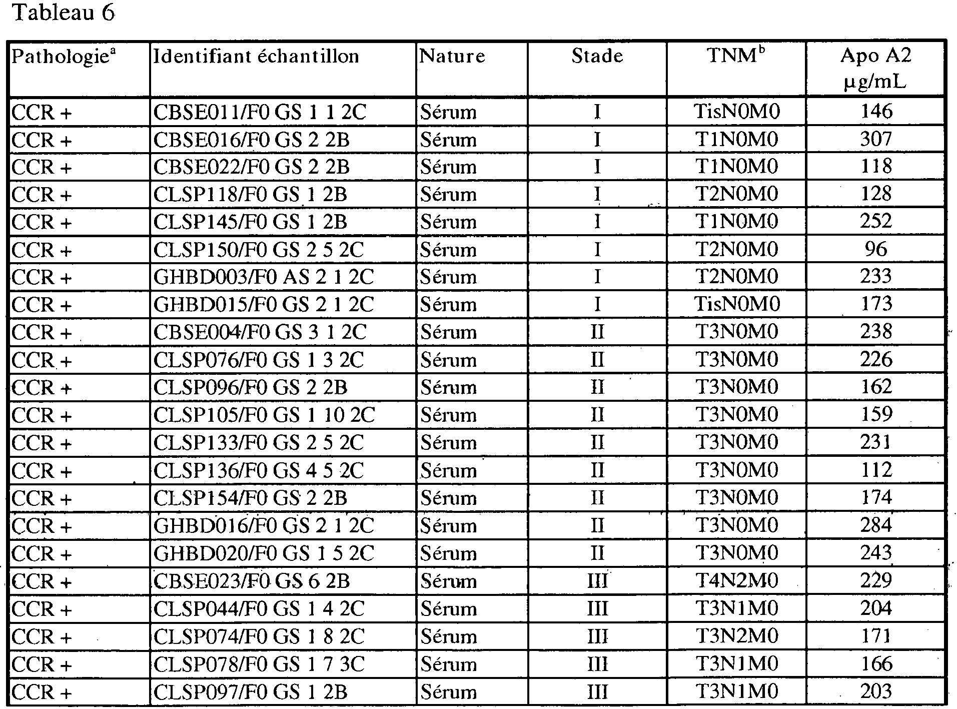

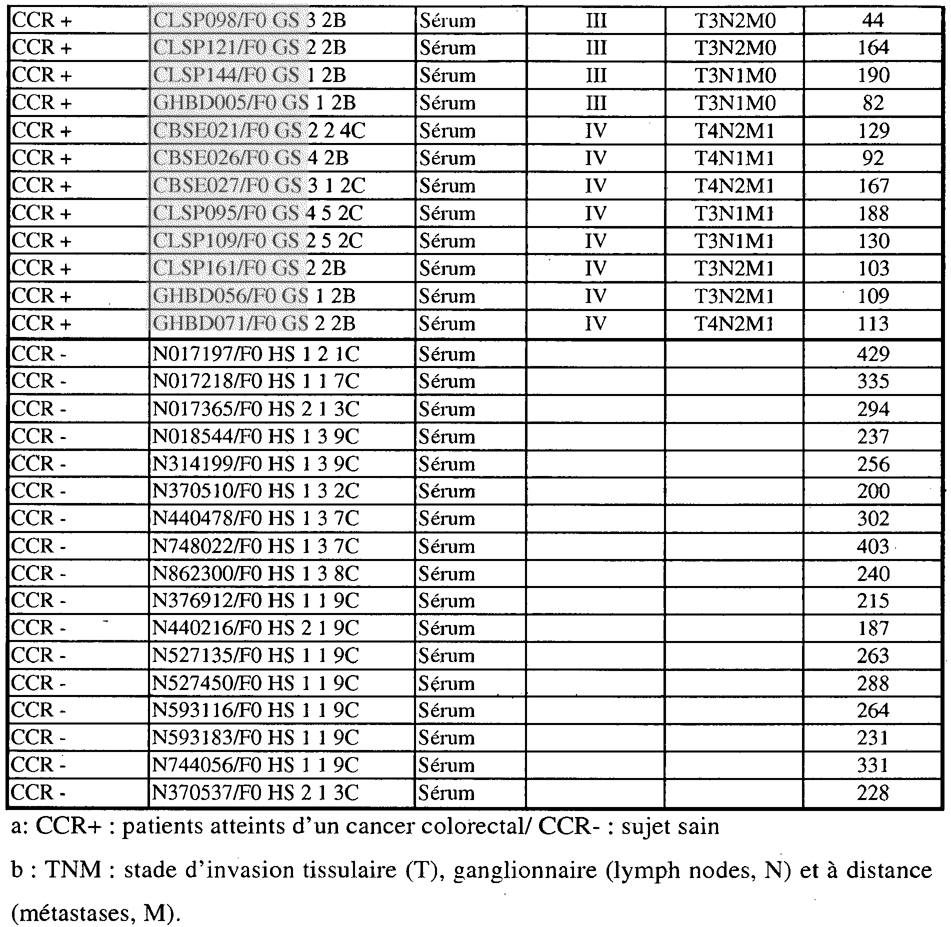

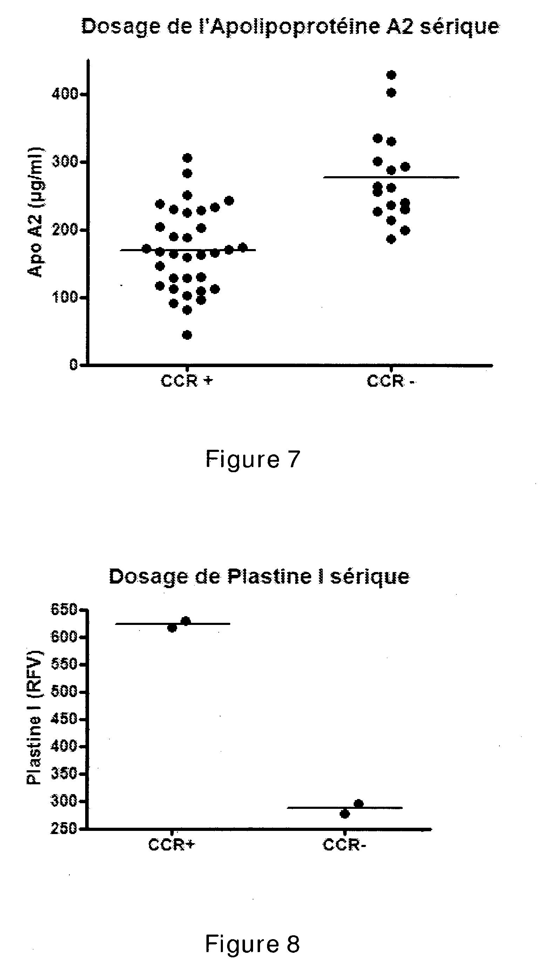

La Figure 7 présente les résultats de ce dosage. Nous avons mis en évidence une diminution de la concentration sérique d'Apo AII chez les sujets ayant un cancer colorectal. La concentration moyenne d'Apo AII chez 34 sujets ayant un CCR de stade I à IV est de 170 ± 11 µg/ml alors qu'elle est bien plus élevée chez 17 sujets sains (témoins) : 277 ± 16 µg/ml. Cette différence est statistiquement très significative (*P*<0,0001, *t*-test unilatéral).

### 9. Dosage sérique du marqueur tumoral Plastine-I

La protéine Plastine-I a été dosée à l'aide des anticorps décrits dans l'exemple 2 et d'un test ELISA utilisant l'automate Vidas® (bioMérieux). Pour ce faire, le test ELISA a été construit en utilisant les réactifs du kit Vidas@ HBs Ag Ultra (bioMérieux, Cat. No. 30315). Les réactifs ont été utilisés tels que décrits dans la notice correspondante (réf. 11728 D - FR - 2005/05), avec les modifications suivantes :
1. Les cônes ont été sensibilisés avec l'anticorps de capture 3D11D10 à une concentration de 15 µg/ml.
2. Le contenu du deuxième puits de la cartouche HBs Ag Ultra a été remplacé par 300 µl d'anticorps de révélation 8C8C5, couplé à la biotine, dilué à 1 µg/ml dans le tampon du deuxième puits du kit Vidas@ HBs Ag Ultra (tampon avec sérum de chèvre et azoture de sodium à 1 g/l).
3. Les échantillons de sérum, de plasma ou de selle (100 µl) ont été dilués directement dans le deuxième puits de la cartouche HBs Ag Ultra.
4. La réaction ELISA a été réalisée à l'aide de l'automate Vidas® et du protocole HBS AG ULTRA.
5. Les résultats ont été obtenus sous forme de valeurs brutes après soustraction du bruit de fond (lecture du substrat avant réaction).

La concentration du marqueur tumoral présente dans le fluide corporel à doser (sang, sérum, plasma, selle) a été calculée selon le mode opératoire décrit dans le paragraphe 2 concernant le dosage du LEI.

Les doses obtenues pour les patients analysés sont reportées sur la Figure 8. Les 2 sérums de patients ayant un cancer colorectal testés montrent une nette augmentation de leur dose de Plastine-I sérique.

### 10. Dosage sérique des marqueurs tumoraux du groupe B

Les marqueurs tumoraux Beta2 Microglobuline, ACE, CA19-9 et Testostérone ont été dosés à l'aide des trousses de dosage de la Demanderesse, respectivement Vidas® Beta2 Microglobulin, Vidas® ACE, Vidas@ CA19-9™ et Vidas® Testostérone, en suivant le protocole opératoire propre à chaque trousse.

La protéine E-Cadhérine a été dosée à l'aide du kit E-Cadhénne EIA kit (Takara Biochemicals, Tokyo, Japon) en suivant le protocole opératoire du kit.

La protéine Regenerating islet-derived protein 3 alpha, autrement nommée Pancreatitis Associated Protein (PAP1), a été dosée à l'aide du kit ELISA PANCREPAP (DynaBio, Marseille, France) en suivant le protocole opératoire du kit.

Les protéines Galectine-3 et LDH ont été dosées à l'aide des anticorps décrits dans l'exemple 2. Le Protéasome 20 S a été dosé à l'aide des anticorps décrits dans le brevet EP0434670. Pour ce faire, les tests ELISA ont été construits en utilisant l'automate Vidas@ (bioMérieux) et les réactifs du kit Vidas® HBs Ag Ultra (bioMérieux, Cat. No. 30315). Les réactifs ont été utilisés tels que décrits dans la notice correspondante (réf. 11728 D - FR - 2005/05), avec les modifications suivantes :
1. Les cônes ont été sensibilisés avec l'anticorps de capture à une concentration entre 5 et 30 µg/ml.
2. Le contenu du deuxième puits de la cartouche HBs Ag Ultra a été remplacé par 300 µl d'anticorps de révélation, couplé à la biotine, dilué à 1 µg/ml dans du tampon avec sérum de chèvre et azoture de sodium à 1 g/l.
3. Les échantillons de sérum, de plasma ou de selle ont été dilués directement dans le deuxième puits de la cartouche HBs Ag Ultra après, si nécessaire, une dilution en tampon du deuxième puits.
4. La réaction ELISA a été réalisée à l'aide de l'automate Vidas@ et du protocole HBS Ag Ultra. L'étape d'incubation de l'échantillon avec les anticorps de capture et de révélation a été comprise entre 14 et 100 cycles.
5. Les résultats ont été obtenus sous forme de valeurs brutes après soustraction du bruit de fond (lecture du substrat avant réaction).

La concentration du marqueur tumoral présente dans le fluide corporel à doser (sang, sérum, plasma, selle) a été calculée selon le mode opératoire décrit dans le paragraphe 2 concernant le dosage du LEI. Les conditions de dosage pour différents marqueurs tumoraux ont été récapitulées dans le Tableau 7.

**Tableau 7**

| Protéine | Galectine-3 | LDH-B | Protéasome 20 S |
|---|---|---|---|
| Anticorps de capture | 12F8A12 à 15 µg/mL | 3F11E11 à 10 | GD6 à 30 µg/mL |
| Anticorps de révélation | 14A5G1 | 12F10G8 | 7A11 |
| Sérum de chèvre dans le tampon de dilution | Avec | Avec | Sans |
| Volume de selles | 50 µL | 50 µL | 200µL |
| Volume de sérum | 50 µL | 50 µL | 100 µL |
| Dépôt échantillon | 2ème puits | 2ème puits | 1er puits |
| Durée d'incubation | 100 cycles | 14 cycles | 14 cycles |

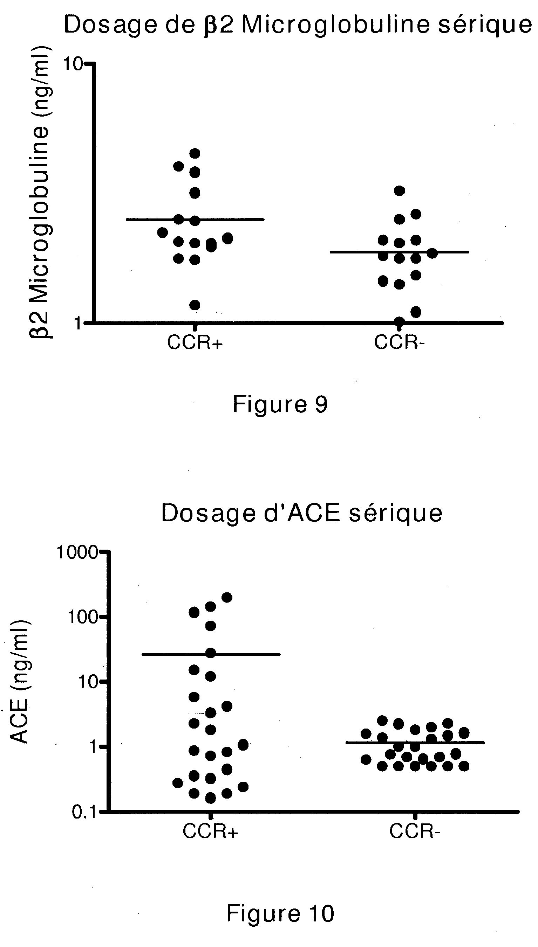

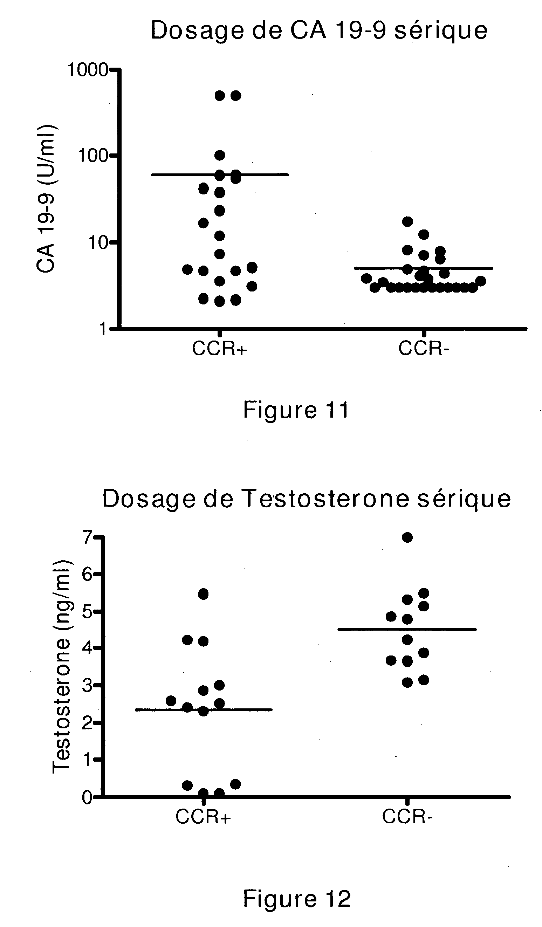

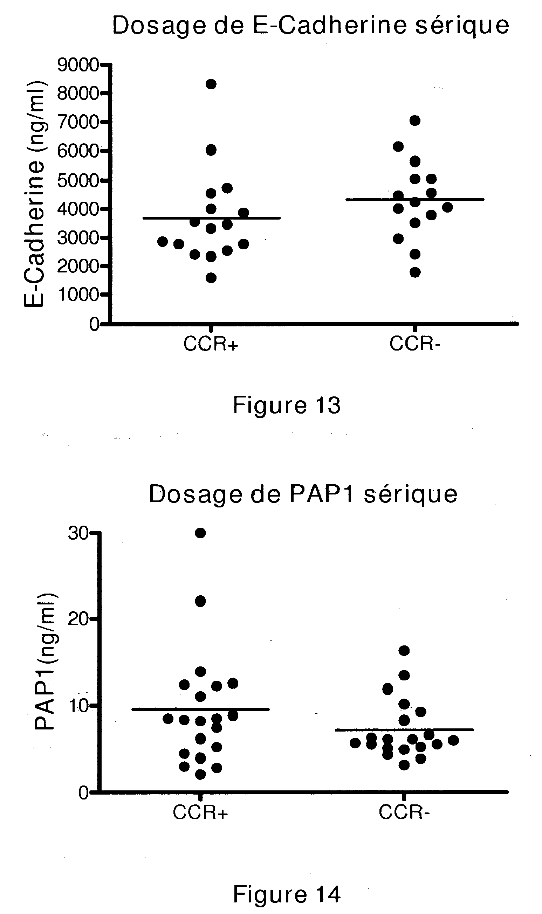

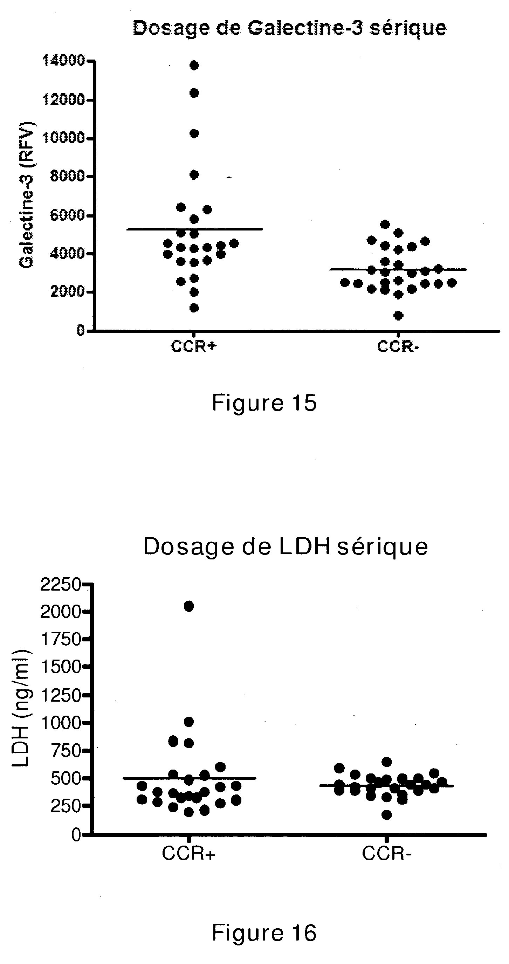

Les doses obtenues pour les patients analysés avec les marqueurs tumoraux Beta2 Microglobuline, ACE, CA19-9, Testostérone, E-Cadhérine, Regenerating islet-derived protein 3 alpha, Galectine-3, LDH et Protéasome 20S ont été reportées respectivement sur les Figures 9 à 17.

Trois sérums de patients ayant un cancer colorectal montrent une augmentation de leur dose de β2 Microglobuline sérique.

Dix sérums de patients ayant un cancer colorectal montrent une augmentation de leur dose d'ACE sérique. Plus nettement, 1 sérum de patient ayant un cancer colorectal de stade III et 7 sérums de patients ayant un cancer colorectal de stade IV montrent une élévation importante de leur dose d'ACE sérique.

Neuf sérums de patients ayant un cancer colorectal montrent une augmentation de leur dose de CA 19-9 sérique. Plus nettement, 1 sérum de patient ayant un cancer colorectal de stade III et 7 sérums de patients ayant un cancer colorectal de stade IV montrent une élévation importante de leur dose de CA 19-9 sérique.

Dix sérums de patients ayant un cancer colorectal montrent une diminution de leur dose de Testostérone sérique. Plus nettement, 1 sérum de patient ayant un cancer colorectal de stade II, 1 sérum de patient ayant un cancer colorectal de stade III et 2 sérums de patients ayant un cancer colorectal de stade IV montrent un effondrement de leur dose de Testostérone sérique.

Deux sérums de patients ayant un cancer colorectal montrent une augmentation de leur dose de Regenerating islet-delived protein 3 alpha sérique.

Quatre sérums de patients ayant un cancer colorectal de stade IV, 2 sérums de patients ayant un cancer colorectal de stade III et 1 sérum de patient ayant un cancer colorectal de stade II montrent une nette augmentation de leur dose de Galectine-3 sérique.

### Exemple 4: Utilisation des dosages sériques des marqueurs tumoraux en combinaison

La Demanderesse a montré dans l'exemple 3 que des doses anormalement élevées ou anormalement diminuées de marqueurs tumoraux pouvaient être observées dans la circulation sanguine de certains patients atteints d'un cancer colorectal. De façon surprenante, l'augmentation ou la diminution de la dose sanguine de deux marqueurs donnés n'est pas systématiquement observée chez les mêmes patients. De ce fait, la combinaison de plusieurs marqueurs tumoraux permet d'augmenter le nombre de patients identifiés comme ayant un cancer colorectal. C'est ainsi qu'un patient A peut présenter une augmentation ou une diminution d'un ou plusieurs marqueurs tumoraux (groupe X), les dit marqueurs du groupe X pouvant être normaux chez un patient B ; chez ce même patient B un ou plusieurs autres marqueurs tumoraux (groupe Y) peuvent être élevés ou diminués, les dit marqueurs du groupe Y pouvant être normaux chez le patient A.

Les différents marqueurs tumoraux dosés par la Demanderesse peuvent ainsi être combinés au moyen de divers algorithmes mathématiques bien connus de l'homme du métier. A titre d'illustration et sans que cet exemple ait un caractère exhaustif, il a été mis en oeuvre le procédé suivant :
1. Une valeur seuil a été fixée pour chaque marqueur tumoral.
2. Lorsque la dose sanguine du marqueur tumoral était augmentée en cas de cancer colorectal, la dose sanguine obtenue pour un patient donné a été divisée par sa valeur seuil. Lorsque la dose sanguine du marqueur tumoral était diminuée en cas de cancer colorectal, la dose sanguine obtenue pour un patient donné a été inversée puis multipliée par sa valeur seuil.
3. Lorsque le ratio, dose sanguine divisée par valeur seuil, était supérieur à 1, le ratio a été multiplié par un coefficient, par exemple 10. La valeur ainsi obtenue a été baptisée « score » pour le patient étudié du marqueur tumoral considéré.
4. Les scores obtenus pour différents marqueurs tumoraux ont été ajoutés en les pondérant d'un facteur propre à chaque marqueur. Dans le cas de l'exemple ci-dessous tous les facteurs de pondération ont été fixés à 1.
5. La somme des scores a été divisée par le nombre total de scores sommés et la valeur ainsi obtenue a été baptisée « score total ».
6. Le patient est diagnostiqué comme ayant un cancer colorectal lorsque sont score total est augmenté par rapport à un score seuil.

Les scores totaux pour une sélection de 2, 4 et 8 marqueurs comprenant Apolipoprotéine AII sont donnés dans le Tableau 8.

L'association des marqueurs tumoraux Apolipoprotéine AII et Galectine-3 permet ainsi d'obtenir pour le même groupe de 13 patients des scores totaux « 2 » augmentés chez 9 patients atteints d'un cancer colorectal alors que le dosage seul de Apolipoprotéine AII ou Galectine-3 était diminué ou augmenté respectivement chez 7 et 5 patients seulement. Conformément aux enseignements de l'exemple 3, le marqueur Apolipoprotéine AII est diminué en cas de cancer colorectal et le marqueur Galectine-3 est augmenté en cas de cancer colorectal.

L'association des marqueurs tumoraux Apolipoprotéine AII, Apolipoprotéine AI, E-Cadhérine et Galectine-3 permet ainsi d'obtenir pour le même groupe de 13 patients des scores totaux « 4 » augmentés chez 10 patients atteints d'un cancer colorectal alors que le dosage seul d'Apolipoprotéine AII, Apolipoprotéine AI, E-Cadhérine ou Galectine-3 était diminué ou augmenté respectivement chez 7, 6, 1 et 5 patients seulement. Conformément aux enseignements de l'exemple 3, les marqueurs Apolipoprotéine AI et Apolipoprotéine AII sont diminués en cas de cancer colorectal et les marqueurs E-Cadhérine et Galectine-3 sont augmentés en cas de cancer colorectal.

L'association des marqueurs tumoraux Apolipoprotéine AII, Apolipoprotéine AI, Testostérone, LEI, LDH-B, L-FABP, E-Cadhérine et Galectine-3 permet ainsi d'obtenir pour le même groupe de 13 patients atteints d'un cancer colorectal des scores totaux «8» augmentés chez 11 patients alors que le dosage seul d'Apolipoprotéine AII, Apolipoprotéine AI, Testostérone, LEI, LDH-B, L-FABP, E-Cadhérine ou Galectine-3 était diminué ou augmenté respectivement chez 7, 6, 4, 3, 1, 6, 1 et 5 patients seulement. Conformément aux enseignements de l'exemple 3, les marqueurs Apolipoprotéine AI, Testostérone, et Apolipoprotéine AII sont diminués en cas de cancer colorectal et les marqueurs LEI, LDH-B, L-FABP, E-Cadhérine et Galectine-3 sont augmentés en cas de cancer colorectal.

**Tableau 8**

| Pathologie | Identifiant échantillon | Stade | TNM | Apo AII | Score total | Score total | Score total |
|---|---|---|---|---|---|---|---|
| | | | | µg/mL | 2^{a} | 4^{b} | 8^{c} |
| CCR+ | CLSP076/F0 | II | T3N0M0 | 226,00 | 0,51 | 0,51 | 16,09 |
| CCR+ | CBSE021/F0 | IV | T4N2M1 | 129 | 12,41 | 9,07 | 14,20 |
| CCR+ | CBSE026/F0 | IV | T4N1M1 | 92 | 16,79 | 15,66 | 66,80 |
| CCR+ | CLSP074/F0 | III | T3N2M0 | 171 | 5,47 | 2,97 | 3,96 |
| CCR+ | CLSP076/F0 | II | T3N0M0 | 226 | 5,58 | 3,09 | 12,24 |
| CCR+ | CLSP078/F0 | III | T3N1M0 | 166 | 12,81 | 6,75 | 39,48 |
| CCR+ | CLSP095/F0 | IV | T3N1M1 | 188 | 0,50 | 0,45 | 0,60 |
| CCR+ | CLSP133/F0 | II | T3N0M0 | 231 | 0,40 | 2,88 | 3,84 |
| CCR+ | CLSP136/F0 | II | T3N0M0 | 112 | 8,35 | 7,01 | 9,34 |
| CCR+ | CLSP161/F0 | IV | T3N2M1 | 103 | 9,08 | 7,35 | 9,80 |
| CCR+ | GHBD015/F0 | I | TisN0M0 | 173 | 5,40 | 5,28 | 7,03 |
| CCR+ | GHBD016/F0 | II | T3N0M0 | 284 | 0,33 | 0,32 | 0,43 |
| CCR+ | GHBD020/F0 | II | T3N0M0 | 243 | 9,60 | 5,15 | 12,60 |
| CCR - | N017197/F0 | | | 429 | 0,61 | 0,45 | 0,61 |
| CCR - | N017218/F0 | | | 335 | 0,28 | 0,28 | 0,38 |
| CCR - | N017365/F0 | | | 294 | 0,32 | 0,30 | 0,39 |
| CCR - | N018544/F0 | | | 237 | 0,39 | 0,36 | 0,48 |
| CCR - | N314199/F0 | | | 256 | 0,58 | 0,72 | 0,59 |
| CCR - | N370510/F0 | | | 200 | 0,97 | 0,92 | 0,79 |
| CCR - | N370537/F0 | | | 228 | 0,41 | 0,37 | 0,49 |
| CCR - | N376912/F0 | | | 215 | 0,43 | 0,39 | 0,53 |
| CCR - | N440216/F0 | | | 187 | 0,50 | 0,45 | 0,60 |
| CCR - | N440478/F0 | | | 302 | 0,31 | 0,30 | 0,40 |
| CCR - | N527135/F0 | | | 263 | 0,36 | 0,33 | 0,44 |
| CCR - | N527450/F0 | | | 288 | 0,32 | 0,35 | 0,46 |
| CCR - | N593116/F0 | | | 264 | 0,35 | 0,32 | 0,43 |
| CCR - | N593183/F0 | | | 231 | 0,40 | 0,38 | 0,50 |
| CCR - | N744056/F0 | | | 331 | 0,28 | 0,47 | 0,47 |
| CCR - | N748022/F0 | | | 403 | 0,23 | 0,23 | 0,31 |
| CCR - | N862300/F0 | | | 240 | 0,39 | 0,40 | 0,53 |
| Seuil | | | | 187,00 | 0,97 | 0,92 | 0,79 |

| | | | | | | | |
|---|---|---|---|---|---|---|---|
| a : association Apolipoprotéine AII et Galectine-3 b : association Apolipoprotéine AII, Apolipoprotéine AI, E-Cadhérine et Galectine-3 c : association Apolipoprotéine AII, Apolipoprotéine AI, Testostérone, LEI, LDH-B, L-FABP, E-Cadhérine et Galectine-3 | | | | | | | |

### Exemple 5 : Dosages des marqueurs tumoraux dans les selles

L'extraction des selles est réalisée à partir d'un morceau pesant approximativement 1g, auquel on ajoute 10 ml de tampon phosphate de sodium 100 mM, pH 7,2 contenant 1 g/L d'azide. On homogénéise sur un Vortex pendant 1 min. L'échantillon subit ensuite 4 cycles d'ultrasons de 7s sur la glace. La fraction non solubilisée est éliminée par centrifugation à 2000 *g*, pendant 10 min, à 4°C. Le surnageant est conservé à -30°C jusqu'au dosage.

Les dosages ELISA décrits dans l'exemple 3 ont été utilisés pour rechercher les marqueurs tumoraux dans les selles après, si nécessaire, une dilution adaptée des selles dans le tampon du premier puits de la cartouche HBs Ag Ultra.

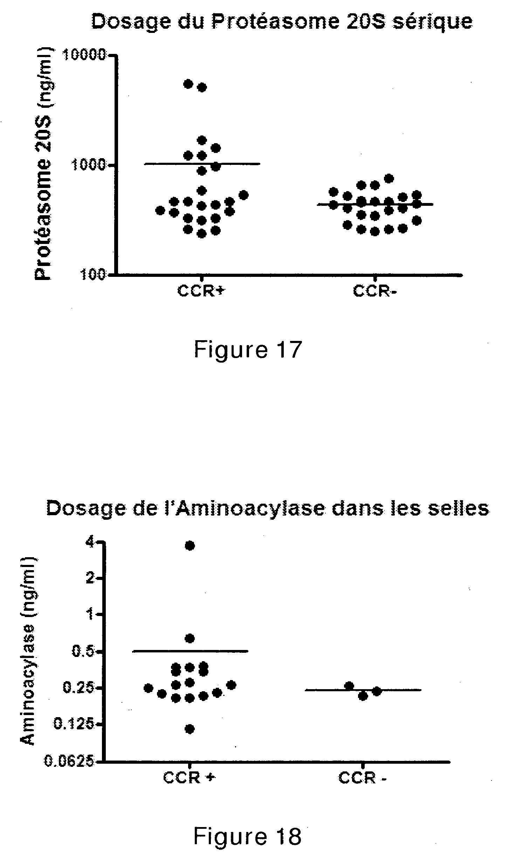

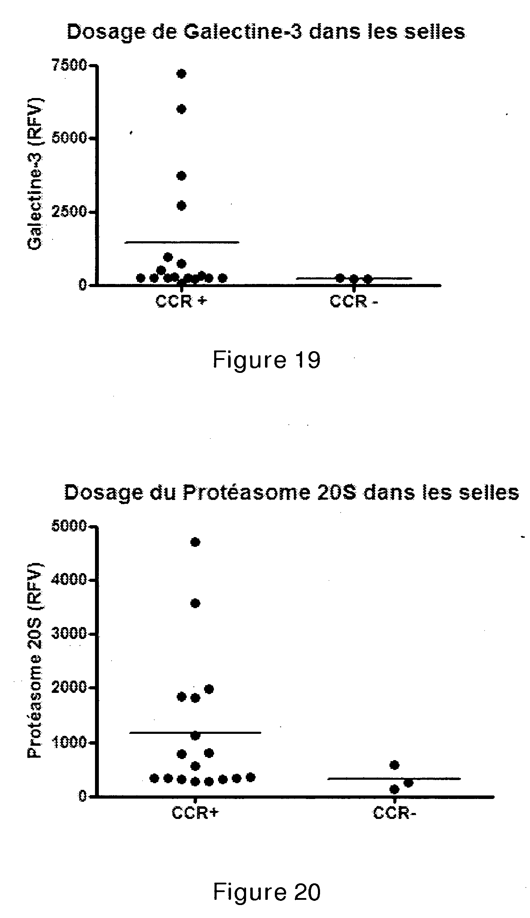

Les dosages obtenus avec les tests, Aminoacylase 1, Galectine-3 et Protéasome 20S ont été représentés respectivement sur les Figures 18 à 20. Une augmentation de la dose d'Aminoacylase 1, de Galectine-3 et de Protéasome 20S est observée respectivement pour 10, 14 et 8 selles de patients atteints d'un cancer colorectal.

### Exemple 6 : Détection par la technique ELISPOT des marqueurs tumoraux

### 1. Culture cellulaire

La lignée de cancer de la prostate LnCAP est cultivée dans le milieu RPMI 1640 supplémenté avec 2 mM L-Glutamine, 10 mM HEPES, 1 mM sodium pyruvate et 10% SVF(tous Gibco). Ces cellules sont utilisées comme témoin négatif.

La lignée de cancer colorectal Caco-2 est cultivée dans le milieu DMEM contenant 2 mM de L-Glutamine, sans SVF (tous Gibco).

La lignée de cancer colorectal HT-29 est cultivée dans le milieu MEM contenant 2 mM de L-Glutamine et 10% SVF (tous Gibco).

La lignée de cancer colorectal HT-29/B6 est cultivée dans le milieu DMEM contenant 4 mM de L-Glutamine, sans SVF (tous Gibco).

Les cellules sont maintenues à 37°C, dans une étuve avec 5% de CO₂.

### 2. La technique ELISPOT

Ce mode opératoire permet de déterminer le nombre de cellules sécrétant la protéine. Les plaques d'ELISPOT 96 puits avec des membranes de PVDF (Multiscreen IP, Millipore) sont coatées par l'anticorps monoclonal de souris anti-marqueur tumoral à 10µg/ml (anticorps de capture, voir le Tableau 9 ci-dessous qui donne les anticorps utilisés en ELISPOT), 100 µl par puits, dans du PBS stérile, sur la nuit à +4°C. Les plaques sont ensuite lavées au PBS et saturées avec du milieu de culture contenant 10% SVF. Parallèlement, les cellules sont trypsinées, comptées, puis diluées à 105 cellules/ml. On distribue 200 µl de cette suspension cellulaire par puits, ainsi que des dilutions en cascade de cette solution mère. Les plaques sont alors incubées 20 h à 37°C en atmosphère humide à 5 % CO₂, puis lavées au PBS contenant 0,05% de Tween-20. Les cellules restantes sont alors lysées par un traitement à l'eau glacée pendant 10 minutes, puis les plaques sont lavées encore. On ajoute ensuite l'anticorps de révélation, le monoclonal biotinylé dirigé contre le marqueur tumoral à doser (Tableau 9), à 0,1µg/puits (incubation 2h à température ambiante). Les spots sont révélés par l'ajout d'extravidine-phosphatase alcaline (Sigma) et du substrat 5-bromo-4-chloro-3-indolyl phosphate/nitro blue tetrazolium (BCIP/NBT, Biorad). Le bruit de fond correspond au nombre de spots mesuré dans les puits LnCap et varie entre 0 et 8 spots dans les conditions de lecture utilisées. Le nombre moyen de spots non spécifiques a été soustrait du signal spécifique.

**Tableau 9**

| Marqueur | Ac capture | Ac détection |
|---|---|---|
| LEI | 10E1H1 | 21B10A5 |
| Ezrine | 4A9H5 | 4A7A6C1 |
| Galectine-3 | 12F8A12 | 14A5G1 |

### 3. Résultats

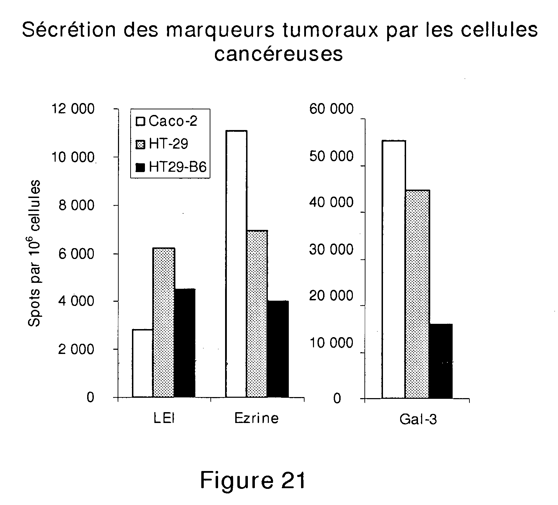

Le nombre de cellules Caco-2, HT-29 et HT-29 B6 sécrétant le marqueur tumoral d'intérêt par 1 million de cellules incubées est présenté dans la Figure 21. La technique ELISPOT permet de confirmer le relarguage ou la sécrétion des marqueurs tumoraux par les lignées de cancer de côlon. Il sera possible d'effectuer une recherche de cellules tumorales circulantes chez les patients en utilisant cette technique, selon le procédé de la demande de brevet WO03/076942 déposé par la Demanderesse.

### Exemple 7 : Détection des marqueurs tumoraux à partir de tissus coliques par immunohistochimie

### 1. Méthodologie

Dans un premier temps, les lames de tissue-micro-array sont déparaffinées. Pour cela, elles sont incubées successivement dans les bains suivants pendant 10 minutes : methycyclohexane (2 fois), éthanol 100%, éthanol 95%, éthanol 70% et eau. Les lames sont ensuite rincées au TBS 0,1% Tween 20 (TBS-T), pendant 10 min, sous agitation. Les antigènes sont réactivés dans le tampon citrate 10 mM pH6, en chauffant jusqu'à 90°C pendant 40 min, puis en laissant refroidir à température ambiante pendant 30 min. Les peroxydases endogènes sont inhibées par incubation dans du TBS-T contenant 3% de H₂O₂, pendant 5 min. Les lames sont ensuite saturées par 3% de BSA en TBS-T, pendant 1h à 37°C, en chambre humide.

Puis, les lames sont incubées pendant 2h avec l'anticorps primaire anti-Leucocyte Elastase Inhibitor (clone 3D9C2), anti-Ezrine (clone 5G2D12), anti-Aminoacylase 1 (clone 8A8A10) ou anti Plastine-I (clone 8D6A3) dilué à 10µg/ml dans du TBS-T contenant 3% de BSA (incubation à 37°C en chambre humide). Après 3 lavages de 10 min au TBS-T, les lames sont incubées 2h à 37°C en chambre humide avec l'anticorps secondaire anti-souris couplé à la peroxydase de raifort (Cat. No. 115-035-003 Jackson Immunoresearch) dilué au 1/400 dans la solution de saturation. Les lames sont lavées 3 fois 10 minutes dans du TBS-T, puis encore 3 fois 10 min dans du PBS. La révélation est réalisée avec le substrat Sigma Fast (Cat. No. D-4168, Sigma-Aldrich) pendant 5 min. La coloration est arrêtée par lavage dans le PBS. On effectue une contre-coloration à l'hématoxyline de Harris (Cat. No. MHS16, Sigma-Aldrich) pendant 30 sec. Après lavages à l'eau et au PBS, les lames sont montées pour observation au microscope.

Les anticorps utilisés pour le marquage par immunohistochimie ont été sélectionnés spécifiquement pour cette application, indépendamment de leur réactivité en ELISA ou en Western blot.

### 2. Détection du Leucocyte Elastase Inhibitor par immunohistochimie

Des lames de tissue-micro-array ont été utilisées pour cribler un grand nombre de prélèvements. Il s'agit de tissus coliques spottés sur lames. Les caractéristiques des patients (caractéristiques des spots de tissu colique présents sur le tissue-micro-array cancer colorectal), ainsi que les résultats des immunomarquages par l'anticorps anti-Leucocyte Elastase Inhibitor sont récapitulés dans le Tableau 10.

**Tableau 10**

| Diagnostic | Histologie et caractérisation génétique | Marquage dans les cellules épithéliales | Marquage dans le stroma |
|---|---|---|---|
| Tumeur maligne | Adénocarcinome conservé | Positif | Négatif |
| Tumeur maligne | Adénocarcinome conservé | Positif | Négatif |
| Normal | Muqueuse normale | Négatif | Négatif |
| Normal | Muqueuse normale | Négatif | Négatif |
| Normal | Muqueuse normale | Négatif | Négatif |
| Normal | Muqueuse normale | Négatif | Négatif |
| Tumeur bénigne | Adénome | Négatif | Négatif |
| Tumeur maligne | Adénocarcinome conservé | Positif | Négatif |
| Tumeur maligne | Adénocarcinome LOH | Positif | Négatif |
| Tumeur maligne | Adénocarcinome LOH | Positif | Négatif |
| Normal | Muqueuse normale | Négatif | Négatif |
| Normal | Muqueuse normale | Négatif | Négatif |
| Normal | Muqueuse normale | Négatif | Négatif |
| Normal | Muqueuse normale | Négatif | Négatif |
| Tumeur maligne | Adénocarcinome LOH | Négatif | Négatif |
| Tumeur maligne | Adénocarcinome LOH | Positif | Négatif |
| Tumeur maligne | Adénocarcinome MSI High | Positif | Négatif |
| Tumeur maligne | Adénocarcinome MSI High | Positif | Négatif |
| Tumeur maligne | Adénocarcinome Colloïde | Négatif | Négatif |
| Tumeur maligne | Adénocarcinome Colloïde | Négatif | Négatif |
| Normal | Muqueuse normale | Négatif | Négatif |
| Normal | Muqueuse normale | Négatif | Négatif |

Les résultats dans le tableau mettent en évidence que, dans les biopsies de muqueuses coliques saines, il n'y a pas de marquage (10 négatifs). Le marquage est également négatif dans l'adénome (1/1). Le marquage est positif dans les cellules épithéliales des adénocarcinomes coliques (+ chez 8/11 patients). Il n'y a aucun marquage dans le stroma.

### 3. Détection de l'Ezrine par immunohistochimie

Des lames de tissue-micro-array ont été utilisées pour cribler un grand nombre de prélèvements. Il s'agit de tissus coliques spottés sur lames. Pour chaque patient avec un adénocarcinome colique, 3 prélèvements au centre de la tumeur, 3 prélèvements au niveau du front d'invasion et 3 prélèvements dans le tissu sain ont été effectués. Le Tableau 11 présente les résultats des immunomarquages par l'anticorps anti-Ezrine, le niveau de marquage indiqué est le maximum d'intensité sur les 3 prélèvements analysés.

**Tableau 11**

| Identifiant patient | Centre de la tumeur | Front invasion tumoral | Tissu sain |
|---|---|---|---|
| 55 | + | ++ | 0 |
| 127 | + | + | 0 |
| 329 | + | ++ | + |
| 475 | + | ++ | + |
| 544 | + | ++ | + |
| 726 | + | + | + |
| 1203 | ++ | ++ | + |
| 1310 | ++ | +++ | + |
| 2003 | + | 0 | + |
| 2296 | ++ | ++ | 0 |
| 2301 | + | ++ | + |
| 2377 | + | + | 0 |
| 3095 | + | + | 0 |
| 3430 | + | + | 0 |
| 3636 | + | + | 0 |
| 3748 | + | + | 0 |
| 3839 | + | ++ | 0 |
| 3891 | 0 | 0 | 0 |
| 4054 | + | + | 0 |
| 4322 | + | ++ | 0 |
| 445 | 0 | ++ | + |
| 4474 | ++ | ++ | 0 |
| 4792 | + | + | + |
| 4958 | ++ | ++ | + |
| 5101 | + | ++ | + |
| 5318 | ++ | +++ | 0 |
| 5374 | + | + | 0 |
| 5472 | + | 0 | + |
| 6340 | ++ | + | 0 |
| 6353 | ++ | + | 0 |

Dans un échantillonnage du 30 patients, 25 ont une surexpression de l'Ezrine au niveau tumoral (centre de la tumeur ou front d'invasion) par rapport au tissu sain adjacent.

### 4. Détection de l'Aminoacylase 1 par immunohistochimie

Des lames de tissue-micro-array ont été utilisées pour cribler un grand nombre de prélèvements. Il s'agit de tissus coliques spottés sur lames. Pour chaque patient avec un adénocarcinome colique, 3 prélèvements au centre de la tumeur, 3 prélèvements au niveau du front d'invasion et 3 prélèvements dans le tissu sain ont été effectués. Le Tableau 12 présente les résultats des immunomarquages par l'anticorps anti-Aminoacylase, le niveau de marquage indiqué est le maximum d'intensité sur les 3 prélèvements analysés.

**Tableau 12**

| Identifiant patient | Centre de la tumeur | Front invasion tumoral | Tissu sain |
|---|---|---|---|
| 55 | ++ | ++ | 0 |
| 127 | 0 | 0 | 0 |
| 329 | ++ | ++ | 0 |
| 475 | ++ | ++ | + |
| 544 | + | 0 | 0 |
| 726 | 0 | 0 | 0 |
| 1203 | 0 | + | 0 |
| 1310 | 0 | + | + |
| 2003 | ++ | 0 | 0 |
| 2296 | + | + | 0 |
| 2301 | + | + | + |
| 2377 | + | + | + |
| 3095 | + | + | 0 |
| 3430 | + | + | + |
| 3636 | ++ | + | + |
| 3748 | ++ | ++ | 0 |
| 3839 | ++ | ++ | + |
| 3891 | ++ | ++ | 0 |
| 4054 | + | ++ | 0 |
| 4322 | +++ | +++ | + |
| 445 | + | ++ | + |
| 4474 | + | ++ | + |
| 4792 | ++ | ++ | + |
| 4958 | + | + | + |
| 5101 | + | + | ++ |
| 5318 | +++ | ++ | 0 |
| 5374 | + | + | 0 |
| 5472 | ++ | ++ | + |
| 6340 | ++ | ++ | + |
| 6353 | ++ | ++ | ++ |

Dans un échantillonnage du 30 patients, 21 ont une surexpression de l'Aminoacylase au niveau tumoral (centre de la tumeur ou front d'invasion) par rapport au tissu sain adjacent.

### 5. Détection de la Plastine-I par immunohistochimie

Des lames de tissue-micro-array ont été utilisées pour cribler un grand nombre de prélèvements. Il s'agit de tissus coliques et rectaux spottés sur lames. Les caractéristiques des patients (caractéristiques des spots de tissu colique présents sur le tissue-micro-array cancer colorectal), ainsi que les résultats des immunomarquages par l'anticorps anti-Plastine-I sont récapitulés dans le Tableau 13.

**Tableau 13**

| Diagnostic | Histologie et caractérisation génétique | Marquage dans les cellules épithéliales | Marquage dans le stroma |
|---|---|---|---|
| Tumeur maligne côlon | Adénocarcinome conservé | ++ | Négatif |
| Tumeur maligne côlon | Adénocarcinome conservé | ++ | Négatif |
| Normal côlon | Muqueuse normale | + | Négatif |
| Normal côlon | Muqueuse normale | + | Négatif |
| Normal côlon | Muqueuse normale | + | Négatif |
| Tumeur bénigne côlon | Adénome | + | Négatif |
| Tumeur maligne côlon | Adénocarcinome conservé | + | Négatif |
| Tumeur maligne côlon | Adénocarcinome LOH | ++ | Négatif |
| Tumeur maligne côlon | Adénocarcinome LOH | ++ | Négatif |
| Normal côlon | Muqueuse normale | + | Négatif |
| Normal côlon | Muqueuse normale | + | Négatif |
| Normal côlon | Muqueuse normale | + | Négatif |
| Tumeur maligne côlon | Adénocarcinome LOH | ++ | Négatif |
| Tumeur maligne côlon | Adénocarcinome LOH | ++ | Négatif |
| Tumeur maligne côlon | Adénocarcinome MSI High | + | Négatif |
| Normal côlon | Muqueuse normale | + | Décollé |
| Normal côlon | Muqueuse normale | + | Décollé |
| Tumeur maligne côlon | Adénocarcinome Colloïde | + | Négatif |
| Normal côlon | Muqueuse normale | ++ | Négatif |
| Normal côlon | Muqueuse normale | ++ | Négatif |
| Normal rectum | Muqueuse normale rectum | ++ | Négatif |
| Normal rectum | Muqueuse normale rectum | Non spécifique | Négatif |
| Normal rectum | Muqueuse normale rectum | Non spécifique | Négatif |
| Normal rectum | Muqueuse normale rectum | Non spécifique | Négatif |
| Tumeur maligne rectum | Adénocarcinome LOH | + | Négatif |
| Tumeur maligne rectum | Adénocarcinome LOH | ++ | Négatif |
| Tumeur maligne rectum | Adénocarcinome LOH | ++ | Négatif |
| Tumeur maligne rectum | Adénocarcinome LOH | ++ | Négatif |
| Tumeur bénigne rectum | Adénome dysplasie de bas grade | ++ | Négatif |
| Tumeur bénigne rectum | Adénome dysplasie de bas grade | ++ | Négatif |
| Tumeur bénigne rectum | Adénome dysplasie de bas grade | + | Négatif |
| Tumeur bénigne rectum | Adénome dysplasie de bas grade | + | Négatif |
| Tumeur bénigne rectum | Adénome dysplasie de bas grade | + | Négatif |
| Tumeur bénigne rectum | Adénome dysplasie de bas grade | + | Négatif |
| Tumeur bénigne rectum | Adénome dysplasie de bas grade | ++ | Négatif |
| Tumeur bénigne rectum | Adénome dysplasie de bas grade | ++ | Négatif |
| Tumeur bénigne rectum | Adénome dysplasie de bas grade | ++ | Négatif |

Les résultats dans le tableau mettent en évidence que :
- dans les biopsies de muqueuses coliques saines, le marquage est faible dans 8 prélèvements (+) et 2 prélèvements sont à ++. Le marquage est également faible à + dans l'adénome colique (1/1). Le marquage est positif fort ++ dans les cellules épithéliales des adénocarcinomes coliques (++ chez 6/9 patients et 3 faibles + dont les adénocarcinomes colloïdes coliques). Il n'y a aucun marquage dans le stroma.
- dans les biopsies de muqueuses rectales saines, le marquage est présent au niveau de l'épithélium de surface de manière non spécifique (3/4) et à ++ dans un prélèvement. Le marquage est positif fort à ++ dans les adénomes rectaux (5/9) ou discret à + (4/9). Le marquage est également fort ++ dans les cellules épithéliales des adénocarcinomes rectaux (++ chez 3/4 patients, 1 faible +). Il n'y a aucun marquage dans le stroma.

### Exemple 8 : Détection des marqueurs tumoraux par la technique LC-MRM-MS

### 1. Méthodologie

Afin de pouvoir baisser la limite de détection à quelques ng/ml, un procédé amélioré de MRM-MS a été mis en oeuvre. Les étapes successives de ce procédé sont : 1) immunodéplétion des protéines abondantes, 2) digestion trypsique, 3) fractionnement SPE (solid-phase extraction) des peptides, 4) chromatographie liquide (LC) couplée à la MRM-MS.

La mise au point a été réalisée sur des échantillons surchargés (spike) en ajoutant les protéines recombinantes ACY, Ezrine, L-FABP, PDI ou Plastine I dans un pool de sérum contrôle, à une concentration de 10-250 ng/ml. Les Apolipoprotéines A1 et A2 sont naturellement présentes dans le sérum.

*Immunodéplétion.* La déplétion des protéines abondantes du sérum a été réalisée en utilisant le kit commercial Vivapure anti-HSA de Vivascience. Alternativement, le kit Proteoextract Albumin/IgG de Calbiochem et le Aurum^{™} serum Protein Minikit de Bio-Rad ont aussi été utilisés. Il est aussi possible de produire les résines spécifiques au laboratoire, en couplant un anticorps monoclonal dirigé contre la protéine à dépléter sur une résine Sepharose 4B activée au CNBr (Amersham Bioscience), en suivant les instructions du fabricant.

*Digestion enzymatique*. Les échantillons de sérums déplétés sont dénaturés dans une solution d'urée 6M tamponnée par 10 mM de Tris pH 8 et contenant 30 mM de dithiothreitol, pendant 40 minutes à 40°C, puis alkylés par de l'iodoacétamide 50 mM, à température ambiante, pendant 40 minutes, à l'obscurité. Ils sont dilués 6 fois dans l'eau, puis la digestion trypsique est réalisée à 37°C, sur la nuit, en utilisant un ratio enzyme substrat de 1:30 (Promega). La digestion est arrêtée par ajout d'acide formique à une concentration finale de 0,5%. Les échantillons digérés sont dessalés par extraction sur phase solide (SPE, solid phase extraction) en utilisant les cartouches en phase inverse Oasis HLB 3cc (60 mg) (Waters). Après application de l'échantillon, les cartouches sont lavées par 1 ml d'acide formique à 0,1%, puis l'élution a été réalisée par un mélange méthanol/eau (80/20 v/v) contenant 0,1% d'acide formique. Les éluats sont séchés sous vide.

*Fractionnement SPE.* Les échantillons secs sont repris dans 1 ml de tampon acétate et chargés sur les cartouches mixtes (hydrophobe et échange de cation) Oasis MCX (mixed cation exchange) 60 mg (Waters) équilibrées au préalable en tampon acétate et méthanol. Les cartouches sont lavées par 1 ml de tampon acétate et 1 ml de méthanol. Les peptides d'intérêt (Tableau 14) sont élués par 1 ml d'un mélange méthanol/tampon acétate (50/50 v/v)). Le pH du tampon acétate est choisi en fonction du point isoélectrique du peptide d'intérêt. Les éluats sont séchés sous vide, dissous dans 200 µl d'une solution d'acétonitrile/eau (3/97 v/v) contenant 0,1% d'acide formique. Un aliquot de 50 µl a été injecté dans la LC couplée à un système MS-MS.

*Chromatographie liquide et spectrométrie de masse.* L'analyse LC-MS a été effectuée sur un système chromatographique haute pression (HPLC) de type HP 1100 series avec pompe binaire et injecteur (Agilent Technologies) couplé à un spectromètre de masse, soit un Sciex API 2000 triple quadripôle, soit un Sciex API 4000 Qtrap (MS hybride triple quadripôle - trappe ionique) (MDS Sciex) pour une meilleure sensibilité. La séparation LC a été effectuée sur une colonne C₁₈ Symmetry (Waters), à un débit d'élution de 300 µl/min. (Eluent A = 0,1% acide formique dans l'eau, éluent B = 0,1% acide formique dans l'acétonitrile, gradient linéaire de 5%B à 50%B en 25 min, puis de 50%B à 100%B en 3 min). L'analyse MS est réalisée en mode d'ionisation positive à une tension de 5500 V appliquée à l'aiguille permettant l'ionisation dans la source. Le contrôle de l'instrument et l'acquisition des données sont réalisés avec le logiciel Analyst 1.4.1. Les débits du gaz de nébulisation (air) et du gaz rideau (azote) sont de 30 et 20 psi, respectivement. La source ionique Turbo V^{™} est réglée à 400°C, le flux d'azote auxiliaire à 40 psi. Les transitions MRM enregistrées pour chaque peptide sont récapitulées dans le Tableau 14. L'énergie de collision (CE), la tension d'orifice (DP,declustering potential) et la tension à la sortie de la cellule de collision (CXP, collision cell exit potential) sont optimisées pour chacune des transitions MRM sélectionnées.

### 2. Résultats

Pour chaque marqueur tumoral (protéines du Tableau 14), la liste des transitions MRM théoriques a été générée en utilisant le logiciel MIDAS (MRM-initiated Detection and Sequencing). Cette liste comprend tous les ions parents di- ou tri-chargés des peptides tryptiques théoriques dans un intervalle de masse allant de 800 à 3000 Da et tous les ions fragments possibles de type y ou b. Pour chaque protéine, chaque transition possible a été testée afin de déterminer les transitions les plus sensibles et les plus spécifiques. Le résultat de cette sélection est récapitulé dans le Tableau 14. En utilisant un peptide lourd de type AQUA (Sigma) ou encore une protéine recombinante lourde qui serviront d'étalon de dosage, il est possible de quantifier de manière absolue le marqueur tumoral d'intérêt dans un milieu biologique complexe.

**Tableau 14**

| *Apolipoprotéine A1* | | | | | | |
|---|---|---|---|---|---|---|
| Séquence (SEQ ID N°) | pI | Q1 | Q3 | DP | CE | CXP |
| AKPALEDLR (SEQ ID N°16) | 6,38 | 338,2 | 288,2 | 14 | 20 | 5 |
| | | | 403,2 | 14 | 15 | 10 |
| | | | 532,3 | 14 | 15 | 25 |
| ATEHLSTLSEK (SEQ ID N°17) | 5,53 | 405,9 | 173,1 | 20 | 20 | 10 |
| | | | 363,2 | 20 | 25 | 20 |
| LSPLGEEMR (SEQ ID N°18) | 4,54 | 516,3 | 201,1 | 20 | 20 | 5 |
| | | | 831,4 | 20 | 20 | 40 |
| | | | 621,3 | 30 | 30 | 15 |
| QGLLPVLESFK (SEQ ID N°19) | 6,11 | 615,9 | 299,2 | 20 | 30 | 15 |
| | | | 819,5 | 20 | 35 | 40 |
| | | | 186,1 | 20 | 35 | 5 |
| THLAPYSDELR (SEQ ID N°20) | 5,39 | 651,3 | 239,1 | 20 | 30 | 5 |
| | | | 352,2 | 20 | 35 | 15 |
| | | | 879,4 | 20 | 35 | 25 |
| VQPYLDDFQK (SEQ ID N°21) | 3,71 | 626,8 | 228,1 | 15 | 25 | 5 |
| | | | 1025,5 | 15 | 20 | 30 |
| | | | 513,2 | 15 | 25 | 25 |
| DYVSQFEGSALGK (SEQ ID N°22) | 4,13 | 700,8 | 279,1 | 15 | 35 | 5 |
| | | | 378,2 | 15 | 20 | 10 |
| | | | 1023,5 | 15 | 20 | 25 |
| | | | 204,1 | 15 | 25 | 5 |

| *Apolipoprotéine A2* | | | | | | |
|---|---|---|---|---|---|---|
| EQLTPLIK (SEQ ID N°23) | 6,4 | 471,3 | 260,2 | 20 | 30 | 15 |
| | | | 147,1 | 20 | 30 | 25 |
| | | | 684,5 | 20 | 20 | 35 |

| *Aminoacylase* | | | | | | |
|---|---|---|---|---|---|---|
| Séquence | pI | Q1 | Q3 | DP | CE | CXP |
| AVGVPALGFSPMNR (SEQ ID N°24) | 10,35 | 708,4 | 517,3 | 20 | 30 | 23 |
| | | | 808,4 | 20 | 35 | 28 |
| | | | 228,1 | 20 | 32 | 7 |
| | | | 1089,6 | 20 | 32 | 32 |
| VVNSILAFR (SEQ ID N°25) | 10,35 | 509,8 | 199,1 | 15 | 25 | 5 |
| | | | 820,5 | 15 | 25 | 28 |
| | | | 506,3 | 15 | 25 | 20 |
| | | | 393,2 | 15 | 25 | 15 |
| EGSVTSVNLTK (SEQ ID N°26) | 6,99 | 567,8 | 248,2 | 15 | 28 | 7 |
| | | | 661,4 | 15 | 28 | 25 |
| | | | 762,4 | 15 | 28 | 32 |
| GPEEEHPSVTLFR (SEQ ID N°27) | 4,58 | 749,3 | 819,5 | 20 | 38 | 35 |
| | | | 956,5 | 20 | 38 | 30 |

| *Ezrine* | | | | | | |
|---|---|---|---|---|---|---|
| Séquence | pI | Q1 | Q3 | DP | CE | CXP |
| IGFPWSEIR (SEQ ID N°28) | 7,04 | 552,8 | 787,2 | 20 | 27 | 25 |
| | | | 288 | 20 | 27 | 10 |
| | | | 690,1 | 20 | 27 | 22 |
| | | | 504,1 | 20 | 27 | 20 |
| ELSEQIQR (SEQ ID N°29) | 4,31 | 501,8 | 303,0 | 20 | 25 | 15 |
| | | | 416,2 | 20 | 25 | 18 |
| | | | 760,2 | 20 | 25 | 25 |
| SGYLSSER (SEQ ID N°30) | 10,1 | 449,7 | 478,2 | 20 | 25 | 20 |
| | | | 591,4 | 20 | 22 | 20 |
| | | | 391,0 | 20 | 22 | 15 |
| APDFVFYAPR (SEQ ID N°31) | 6,76 | 591,8 | 272,2 | 20 | 28 | 10 |
| | | | 1111,6 | 20 | 28 | 35 |

| *L-FABP* | | | | | | |
|---|---|---|---|---|---|---|
| Séquence | pI | Q1 | Q3 | DP | CE | CXP |
| AIGLPEELIQK (SEQ ID N°32) | 4,54 | 605,8 | 856,5 | 32 | 29 | 22 |
| | | | 1026,58 | 32 | 30 | 27 |
| | | | 185,1 | 32 | 30 | 5 |
| | | | 242,2 | 32 | 33 | 5 |
| GVSEIVQNGK (SEQ ID N°33) | 6,36 | 515,8 | 658,4 | 28 | 30 | 35 |
| | | | 545,3 | 28 | 30 | 30 |
| | | | 157,1 | 28 | 30 | 10 |
| | | | 446,2 | 28 | 30 | 25 |
| TVVQLEGDNK (SEQ ID N°34) | 4,13 | 551,8 | 261,2 | 35 | 28 | 10 |
| | | | 675,33 | 35 | 28 | 34 |
| | | | 201,1 | 35 | 28 | 10 |
| FTITAGSK | 9,0 | 412,7 | 576,3 | 23 | 27 | 30 |
| (SEQ ID N°35) | | | 463,3 | 23 | 27 | 30 |
| | | | 249,1 | 23 | 27 | 10 |

| *PDI* | | | | | | |
|---|---|---|---|---|---|---|
| Séquence | pI | Q1 | Q3 | DP | CE | CXP |
| EADDIVNWLK (SEQ ID N°36) | 3,74 | 601,8 | 560,3 | 20 | 27 | 18 |
| | | | 659,4 | 20 | 27 | 22 |
| DHENIVIAK (SEQ ID N°37) | 5,24 | 519,8 | 253,1 | 20 | 25 | 10 |
| | | | 331,2 | 20 | 25 | 13 |
| | | | 786,4 | 20 | 25 | 30 |
| | | | 657,4 | 20 | 25 | 25 |
| LITLEEEMTK (SEQ ID N°38) | 4,01 | 603,8 | 328,2 | 20 | 30 | 11 |
| | | | 766,3 | 20 | 30 | 25 |
| | | | 980,5 | 20 | 30 | 32 |
| ENLLDFIK (SEQ ID N°39) | 4,11 | 496,3 | 407,3 | 20 | 25 | 16 |
| | | | 522,3 | 20 | 25 | 21 |
| | | | 635,4 | 20 | 25 | 26 |

| *Plastine I* | | | | | | |
|---|---|---|---|---|---|---|
| Séquence | pI | Q1 | Q3 | DP | CE | CXP |
| QFTPADVVSGNPK (SEQ ID N°40) | 6,96 | 729,8 | 983,4 | 20 | 27 | 30 |
| | | | 1183,6 | 20 | 27 | 35 |
| SLADGILLCK (SEQ ID N°41) | 6,08 | 545,3 | 889,4 | 20 | 25 | 28 |
| | | | 533,4 | 20 | 25 | 20 |
| | | | 703,4 | 20 | 25 | 25 |
| | | | 818,4 | 20 | 25 | 28 |

### Références bibliographiques

1 : J.D. Potter, 1999, J Natl Cancer Inst, 91, 916-32
2 : J. Faivre, 2001, Epidémiologie et dépistage du cancer colorectal, Ed. Springer
3 : E.E. Niederkofler et al., 2003, J Lipid Res, 44, 630-639
4 : G.L. Hortin, 2006, Clinical Chemistry, 52(7), 1223-1237
5 : H. Hachem et al., 1986, J. Chem. Clin. Biochem., 24, 161-166
6 : E. Remold-O'Donnell et al., 1992, Proc Natl Acad Sci USA, 89, 563-5639
7 : J. Cooley et al., 2001, Biochemistry, 15762-15770
8 : M. Algrain et al., 1993, J Cell Biol, 120, 129-139
9 : W.G. Jiang et S. Hiscox, 1996, Anticancer Res, 16, 861-865
10 : S. Hiscox et W.G. Jiang, 1999, J Cell Sci, 112, 3081-3090
11 : T. Xiao et al, 2005, Mol Cell Proteomics, 4, 1480-1486
12 : M. Anders et W. Dekant, 1994, Advances in Pharmacology, 431-448
13 : K. Lorentz et al., 1975, Clinica Chimica Acta, 263-269
14 : K. Lorentz et B. Flatter, 1975, Clinica Chimica Acta, 271-274
15 : R.M. Cook et al., 1993, J Bio Chem, 17010-17017
16 : Y.E. Miller et al., 1989, J Clin Invest, 2120-2124
17 : S. Balabanov et al., 2001, Eur J Biochem, 5977-5980
18 : E. Chan et al., 1985, J Biol Chem, 260, 2629-2632
19 : R. Das et al., 2001, Clin. Cancer Res., 7, 1706-1715
20 : J. Stulik et al., 2001, Electrophoresis, 22, 3019-3025
21 : T. Yamazaki et al., 1999, J Surg Oncol, 72, 83-87
22 : D.A. Sweetser et al., 1987, J Biol Chem, 266, 16060-16071
23 : M. Pelsers et al., 2003, Clin Biochem, 36, 529-535
24 : R. Xiao, et al., 2005, Molecular Cancer, 4, 1-17
25 : J.Y. Engwegen et al., 2006, World J Gastroenterol, 12(10), 1536-1544
26 : Z. Zhang et al., 2004, Cancer Research, 64, 5882-5890
27 : C.S. Lin, et al., 1993, J Biol Chem., 268, 2781-92
28 : V. Delanote et al., 2005, Acta Pharama Sinica, 769-779
29 : A.P. Arrigo et al., 1988, Nature, 331, 192-194
30 : T. Lavabre-Bertrand et al., 2001, Cancer, 92, 2493-2500
31 : S. Nakahara et al., 2005, Apoptosis, 10, 267-275
32 : I. Iurisci et al., 2000, Clin Can Res, 6, 1389-1393
33 : M.K. Schwartz, 2006, Clin Chim Acta, 1992, 77-82
34 : D.J. McCool et al., 1999, Biochem J, 593-600
35 : J.L. Iovanna et al., 1994, Gastroenterology, 106, 728-734
36 : Y. Motoo et al., 1999, Dig Dis Sci, 44, 1142-1147
37 : M. Herlyn et al., 1979, Proc Natl Acad Sci USA, 76, 1438-1442
38 : A. Armstrong et S. Eck, 2003, Cancer Biol Ther, 2, 320-325
39 : D. Herlyn et al., 1982, Proc Natl Acad Sci USA, 79, 4761-4765
40 : H Abe et al., 2002, J Immunol Methods, 270, 227-233
41 : V. Barak et al., 2004, Clin Biochem, 37, 529-540
42 : H. Kim et al., 2006, Ann Clin Lab Sci, 36, 294-298
43 : F. Roca et al., 2006, J Surg Oncol, 151-160
44 : C.H. Damsky et al., 1983, Cell, 455-466
45 : M. Katayama et al., 1994, Br J Cancer, 580-585
46 : C. Willmanns et al., 2004, Clin Exp Metastasis, 75-78
47 : P. Gold et S. Freedman, 1965, J Exp Med, 467-481
48 : M. Duffy, 2001, Clin Chem 624-630
49 : Y. Kim et al., 2003, Ann Clin Lab Sci, 32-38
50 : J:L. Magnani et al., 1983, Cancer Research, 43, 5489-5492
51 : J. Holmgren et al., 1984, Br Med J (Clin. Re. Ed.), 288, 1479-1482
52 : T.L. Klug et al., 1986, Int J Cancer, 38, 6661-669
53 : P. Kuusela et al., 1991, Br J Cancer, 63, 636-640
54 : M. Holland et al., 1993, Medicina (B. Aires), 53(2), 117-23
55: F. Model et al., juillet 2006, World Congress on Gastrointestinal Cancer, « Detection of Methylated DNA in Plasma from Colorectal Cancer Patients and Controls by Real-Time PCR Analysis of Septin 9 »
56 : M.P. Ebert et al., 2006, Gastroentrology, 131(5), 1418-1430
57 : C. Bianco et al., 2006, Clin Cancer Res, 12, 5158-5164
58 : R. Yasumatsu et al., 2006, Am J Physiol Lung Cell Mol Physiol 291, L619-L627
59 : J. Chevalier et al., 1997, J Histochem Cytochem, 45, 481-491
60 : S. Patterson, 2000, Physiological Genomics 2, 59-65
61 : L. Anderson et C.L. Hunter, 2006, Mol Cell Proteomics, 5, 573-588.
62 : L.J. Kricka et al., 1999, Clinical Chemistry, 45(4), 453-458
63 : S. Tyagi et F.R. Kramer, 1996, Nature Biotech, 14, 303-308
64 : T. F. Imperiale et al., 2004, N Engl J Med, 351(26), 2704-2714
65 : D.A. Ahlquist et al., 2000, Gastroenterology, 119, 1219-1227
66 : I. H. Wong, 2006, Methods Mol Biol, 336, 33-46
67 : M. P. Ebert et al., 2005, Neoplasia, 7(8), 771-778
68 : C. Lofton-Day et al., 2007, AACR Annual Meeting 2007, Los Angeles, U.S.A., Poster no LB-165, Clinical case-control study in plasma shows that the DNA methylation biomarker, Septin-9, detects 70% of stage I-III colorectal cancer patients
69 : P. Métézeau et al., La cytométrie en flux pour l'étude de la cellule normale ou pathologique (Tome I), Eds Medsi-MacGrawhill
70 : Mathieu J. et al. 2006. Fonctions cellulaires et métabolisme. In: (coordonnateurs : Ronot X.et al.). La cytométrie en flux. Tec & Doc, 255-298. ISBN 978-2-7430-0898-7
71 : V. Cheynet et al., 1993, Protein Expr Purif, 4(5), 367-372
72 : G. Köhler et C. Milstein, 1975, Nature, 256, 495-497
73 : G. Köhler et C. Milstein, 1976, Eur J Immunol, 6, 511-519

## Revendications

1. Procédé de diagnostic *in vitro* du cancer colorectal par détermination de la diminution de la concentration du marqueur Apolipoprotéine AII, par rapport aux valeurs de référence déterminées pour les patients sains, dans un échantillon biologique issu d'un patient suspecté d'être atteint du cancer colorectal, ledit échantillon étant distant de la tumeur.

2. Procédé selon la revendication 1, **caractérisé en ce qu'**il consiste également à déterminer la présence d'au moins un autre marqueur tumoral choisi parmi les marqueurs suivants : Leucocyte Elastase Inhibitor, Ezrine, Aminoacylase 1, Liver Fatty Acid-Binding Protein, Intestinal Fatty Acid-Binding Protein, Apolipoprotéine AI et Plastine-I.

3. Procédé selon l'une des revendications 1 ou 2, **caractérisé en ce qu'**il consiste également à déterminer la présence d'au moins un autre marqueur tumoral choisi parmi les marqueurs suivants : Beta 2 Microglobuline, Protéasome 20S, Galectine-3, L-Lactate Deshydrogénase Chaîne B, Calréticuline, Regenerating Islet-Derived Protein 3 Alpha, Tumor-Associated Calcium Signal Transducer 1, Keratine type II Cytoskeletal 8, Keratine type I Cytoskeletal 18, Keratine type I Cytoskeletal 19, Epithelial-Cadhérine, ACE, Villine, CA19-9, CA 242, CA 50, CA 72-2, Testostérone, TIMP-1, Cripto-1, Intélectine-1, Protéine Disulfide Isomérase, Cytokératine 20, Translationally-Controlled Tumor Protein, (Pro)défensine-A5, la détection d'ADN méthylé dans le sang, de préférence l'ADN méthylé du gène AXL4 ou l'ADN méthylé du gène Septin-9, la détection d'altérations spécifiques de fragments d'ADN dans les selles comme des mutations spécifiques de l'ADN dans les selles ou des altérations spécifiques du profil de méthylation de l'ADN dans les selles, la détection d'hémoglobine humaine dans les selles.

4. Utilisation du procédé selon l'une quelconque des revendications 1 à 3 dans le diagnostic précoce, le dépistage, le suivi thérapeutique, le pronostic et le diagnostic des rechutes dans le cadre du cancer colorectal.

## Patentansprüche

1. Verfahren zur *in vitro*-Diagnose von kolorektalem Krebs durch Bestimmung der Verringerung der Konzentration des Markers Apolipoprotein AII, bezogen auf die für gesunde Patienten bestimmten Referenzwerte, in einer biologischen Probe von einem Patienten, von dem vermutet wird, dass er an kolorektalem Krebs leidet, wobei die Probe eine entfernt vom Tumor entnommene Probe ist.

2. Verfahren nach Anspruch 1, **dadurch gekennzeichnet, dass** man bei dem Verfahren auch das Vorhandensein von mindestens einem anderen Tumormarker bestimmt, ausgewählt aus den folgenden Markern: Leukozytenelastase-Inhibitor, Ezrin, Aminoacylase 1, Leberfettsäure-Bindungsprotein, Darmfettsäure-Bindungsprotein, Apolipoprotein AI und Plastin-I.

3. Verfahren nach einem der Ansprüche 1 oder 2, **dadurch gekennzeichnet, dass** man dabei außerdem das Vorhandensein von mindestens einem anderen Tumormarker bestimmt, ausgewählt aus den folgenden Markern: Beta 2-Mikroglobulin, Proteasom 20S, Galectin-3, L-Lactat-Dehydrogenase Kette B, Calreticulin, Regenerating Islet-derived Protein 3 Alpha, tumorassoziierter Calciumsignal-Transducer 1, Keratin Typ II Cytoskeletal 8, Keratin Typ I Cytoskeletal 18, Keratin Typ I Cytoskeletal 19, epitheliales Cadherin, ACE, Villin, CA19-9, CA 242, CA 50, CA 72-2, Testosteron, TIMP-1, Cripto-1, Intelectin-1, Protein-Disulfid-Isomerase, Cytokeratin 20, translational kontrolliertes Tumorprotein, (Pro)Defensin-A5, der Nachweis von methylierter DNA im Blut, vorzugsweise methylierter DNA des Gens AXL4 oder methylierter DNA des Septin-9-Gens, der Nachweis von spezifischen Veränderungen von DNA-Fragmenten im Stuhl, wie spezifische Mutationen der DNA im Stuhl oder spezifische Veränderungen des Methylierungsprofils der DNA im Stuhl, der Nachweis von menschlichem Hämoglobin im Stuhl.

4. Verwendung des Verfahrens nach einem der Ansprüche 1 bis 3 zur frühen Diagnose, zum Screening, zur therapeutischen Nachverfolgung, zur Prognose und Diagnose von Rückfällen in Zusammenhang mit kolorektalem Krebs.

## Claims

1. A method for the *in vitro* diagnosis of colorectal cancer by determining decrease of the concentration of the Apolipoprotein AII marker in a biological sample taken from a patient suspected of having colorectal cancer, relative to the reference values determined for healthy patients, the sample being a sample that is remote from the tumor.

2. The method as claimed in claim 1, **characterized in that** it also consists in determining the presence of at least one other tumor marker chosen from the following markers: Leukocyte Elastase Inhibitor, Ezrin, Aminoacylase 1, Liver Fatty Acid-Binding Protein, Intestinal Fatty Acid-Binding Protein, Apolipoprotein AI and I-Plastin.

3. The method as claimed in claim 1 or 2, **characterized in that** it also consists in determining the presence of at least one other tumor marker chosen from the following markers: Beta2-Microglobulin, Proteasome 20S, Galectin-3, L-Lactate Dehydrogenase Chain B, Calreticulin, Regenerating Islet-Derived Protein 3 Alpha, Tumor-Associated Calcium Signal Transducer 1, Keratin type II Cytoskeletal 8, Keratin type I Cytoskeletal 18, Keratin type I Cytoskeletal 19, Epithelial-Cadherin, CEA, Villin, CA19-9, CA 242, CA 50, CA 72-2, Testosterone, TIMP-1, Cripto-1, Intelectin-1, Protein Disulfide Isomerase, Cytokeratin 20, Translationally-Controlled Tumor Protein, (Pro)defensin-A5, the detection of methylated DNA in the blood, preferably methylated DNA of the AXL4 gene or methylated DNA of the septin-9 gene, the detection of specific alterations in fecal DNA fragments, such as specific mutations of fecal DNA or specific alterations of the methylation profile of fecal DNA, and the detection of fecal human hemoglobin.

4. The use of the method as claimed in any one of claims 1 to 4, for early diagnosis, screening, therapeutic follow-up, prognosis and relapse diagnosis in relation to colorectal cancer.


### SEQUENCE LISTING

<110> bioMérieux
<120> Procédé de dosage de l'Apolipoprotéine AII pour le diagnostic in vitro du cancer colorectal
<130> APOA2ANDCO
<150> FR07 05210
   <151> 2007-07-19
<160> 41
<170> PatentIn version 3.3
<210> 1
   <211> 24
   <212> DNA
   <213> artificial sequence
<220>
   <223> Amorce
<400> 1
   atggagcagc tgagctcagc aaac 24
<210> 2
   <211> 24
   <212> DNA
   <213> Artificial sequence
<220>
   <223> Amorce
<400> 2
   ctaaggggaa gaaaatctcc ccaa 24
<210> 3
   <211> 33
   <212> DNA
   <213> Artificial sequence
<220>
   <223> Amorce
<400> 3
   cggagcgtct cccatgagtt tctccggcaa gta 33
<210> 4
   <211> 49
   <212> DNA
   <213> Artificial sequence
<220>
   <223> Amorce
<400> 4
   gaaatgcaga cttgtctaga tgcgcttgct gatgcgcttg aagacaatg 49
<210> 5
   <211> 22
   <212> DNA
   <213> Artificial sequence
<220>
   <223> Amorce
<400> 5
   atggcagaca atttttcgct cc 22
<210> 6
   <211> 26
   <212> DNA
   <213> Artificial Sequence
<220>
   <223> Amorce
<400> 6
   ttatatcatg gtatatgaag cactgg 26
<210> 7
   <211> 64
   <212> DNA
   <213> Artificial sequence
<220>
   <223> Amorce
<400> 7
   gcgaattctt taagaaggag atatacatat gacgagcaaa ggtccggaag aggagcaccc 60
   atcg 64
<210> 8
   <211> 28
   <212> DNA
   <213> Artificial sequence
<220>
   <223> Amorce
<400> 8
   gcaagcttca gctgtcactg ggcagggc 28
<210> 9
   <211> 424
   <212> DNA
   <213> Artificial sequence
<220>
   <223> I-FABP
<400> 9
   ggtaccgaat tccgcgtttg acagcacttg gaaggtagac cggagtgaaa actatgacaa 60
   gttcatggaa aaaatgggtg ttaatatagt gaaaaggaag cttgcagctc atgacaattt 120
   gaagctgaca attacacaag aaggaaataa attcacagtc aaagaatcaa gcgcttttcg 180
   aaacattgaa gttgtttttg aacttggtgt cacctttaat tacaacctag cagacggaac 240
   tgaactcagg gggacctgga gccttgaggg aaataaactt attggaaaat tcaaacggac 300
   agacaatgga aacgaactga atactgtccg agaaattata ggtgatgaac tagtccagac 360
   ttatgtgtat gaaggagtag aagccaaaag gatctttaaa aaggattcta gagtcgacga 420
   gctc 424
<210> 10
   <211> 31
   <212> DNA
   <213> Artificial sequence
<220>
   <223> Amorce
<400> 10
   atgggaattc aggagcagct gagctcagca a 31
<210> 11
   <211> 30
   <212> DNA
   <213> Artificial sequence
<220>
   <223> Amorce
<400> 11
   cgataagctt aaggggaaga aaatctcccc 30
<210> 12
   <211> 62
   <212> DNA
   <213> Artificial sequence
<220>
   <223> Amorce
<400> 12
   gctggccatg ggcagcagcc atcatcatca tcatcacatg agtttctccg gcaagtacca 60
   ac 62
<210> 13
   <211> 39
   <212> DNA
   <213> Artificial sequence
<220>
   <223> Amorce
<400> 13
   gcacggatcc tagatgcgct tgctgatgcg cttgaagac 39
<210> 14
   <211> 31
   <212> DNA
   <213> Artificial sequence
<220>
   <223> Amorce
<400> 14
   atgggaattc aggcagacaa tttttcgctc c 31
<210> 15
   <211> 34
   <212> DNA
   <213> Artificial sequence
<220>
   <223> Amorce
<400> 15
   cgataagctt atatcatggt atatgaagca ctgg 34
<210> 16
   <211> 9
   <212> PRT
   <213> artificial
<220>
   <223> ApoA peptide
<400> 16
   Ala Lys Pro Ala Leu Glu Asp Leu Arg
   1 5
<210> 17
   <211> 11
   <212> PRT
   <213> artificial
<220>
   <223> ApoA1 peptide
<400> 17
   Ala Thr Glu His Leu Ser Thr Leu Ser Glu Lys
   1 5 10
<210> 18
   <211> 9
   <212> PRT
   <213> artificial
<220>
   <223> ApoA1 peptide
<400> 18
   Leu Ser Pro Leu Gly Glu Glu Met Arg
   1 5
<210> 19
   <211> 11
   <212> PRT
   <213> artificial
<220>
   <223> ApoA1 peptide
<400> 19
   Gln Gly Leu Leu Pro Val Leu Glu Ser Phe Lys
   1 5 10
<210> 20
   <211> 11
   <212> PRT
   <213> artificial
<220>
   <223> ApoA1 peptide
<400> 20
   Thr His Leu Ala Pro Tyr Ser Asp Glu Leu Arg
   1 5 10
<210> 21
   <211> 10
   <212> PRT
   <213> artificial
<220>
   <223> ApoA1 peptide
<400> 21
   Val Gln Pro Tyr Leu Asp Asp Phe Gln Lys
   1 5 10
<210> 22
   <211> 13
   <212> PRT
   <213> artificial
<220>
   <223> ApoA1 peptide
<400>
   Asp Tyr Val Ser Gln Phe Glu Gly Ser Ala Leu Gly Lys
   1 5 10
<210> 23
   <211> 8
   <212> PRT
   <213> artificial
<220>
   <223> ApoA2 peptide
<210> 24
   <211> 14
   <212> PRT
   <213> artificial
<220>
   <223> Aminoacylase peptide
<400> 24
   Ala Val Gly Val Pro Ala Leu Gly Phe Ser Pro Met Asn Arg
   1 5 10
<210> 25
   <211> 9
   <212> PRT
   <213> artificial
<220>
   <223> Aminoacylase peptide
<400> 25
   Val Val Asn Ser Ile Leu Ala Phe Arg
   1 5
<210> 26
   <211> 11
   <212> PRT
   <213> artificial
<220>
   <223> Aminoacylase peptide
<400> 26
   Glu Gly Ser Val Thr Ser Val Asn Leu Thr Lys
   1 5 10
<210> 27
   <211> 13
   <212> PRT
   <213> artificial
<220>
   <223> Aminoacylase peptide
<400> 27
   Gly Pro Glu Glu Glu His Pro Ser Val Thr Leu Phe Arg
   1 5 10
<210> 28
   <211> 9
   <212> PRT
   <213> artificial
<220>
   <223> Ezrine peptide
<400> 28
   Ile Gly Phe Pro Trp Ser Glu Ile Arg
   1 5
<210> 29
   <211> 8
   <212> PRT
   <213> artificial
<220>
   <223> Ezrine peptide
<400> 29
   Glu Leu Ser Glu Gln Ile Gln Arg
   1 5
<210> 30
   <211> 8
   <212> PRT
   <213> artificial
<220>
   <223> Ezrine peptide
<400> 30
   Ser Gly Tyr Leu Ser Ser Glu Arg
   1 5
<210> 31
   <211> 10
   <212> PRT
   <213> artificial
<220>
   <223> Ezrine peptide
<400> 31
   Ala Pro Asp Phe Val Phe Tyr Ala Pro Arg
   1 5 10
<210> 32
   <211> 11
   <212> PRT
   <213> artificial
<220>
   <223> L-FABP peptide
<400> 32
   Ala Ile Gly Leu Pro Glu Glu Leu Ile Gln Lys
   1 5 10
<210> 33
   <211> 10
   <212> PRT
   <213> artificial
<220>
   <223> L-FABP peptide
<400> 33
   Gly Val Ser Glu Ile Val Gln Asn Gly Lys
   1 5 10
<210> 34
   <211> 10
   <212> PRT
   <213> artificial
<220>
   <223> L-FABP peptide
<400> 34
   Thr Val Val Gln Leu Glu Gly Asp Asn Lys
   1 5 10
<210> 35
   <211> 8
   <212> PRT
   <213> artificial
<220>
   <223> L-FABP peptide
<400> 35
   Phe Thr Ile Thr Ala Gly Ser Lys
   1 5
<210> 36
   <211> 10
   <212> PRT
   <213> artificial
<220>
   <223> PDI peptide
<400> 36
   Glu Ala Asp Asp Ile Val Asn Trp Leu Lys
   1 5 10
<210> 37
   <211> 9
   <212> PRT
   <213> artificial
<220>
   <223> PDI peptide
<400> 37
   Asp His Glu Asn Ile Val Ile Ala Lys
   1 5
<210> 38
   <211> 10
   <212> PRT
   <213> artificial
<220>
   <223> PDI peptide
<400> 38
   Leu Ile Thr Leu Glu Glu Glu Met Thr Lys
   1 5 10
<210> 39
   <211> 8
   <212> PRT
   <213> artificial
<220>
   <223> PDI peptide
<400> 39
   Glu Asn Leu Leu Asp Phe Ile Lys
   1 5
<210> 40
   <211> 13
   <212> PRT
   <213> artificial
<220>
   <223> Plastine-I peptide
<400> 40
   Gln Phe Thr Pro Ala Asp Val Val Ser Gly Asn Pro Lys
   1               5                   10
<210> 41
   <211> 10
   <212> PRT
   <213> artificial
<220>
   <223> Plastine-I peptide
<400> 41
   Ser Leu Ala Asp Gly Ile Leu Leu Cys Lys
   1               5                   10